(19) **Europäisches Patentamt / European Patent Office / Office européen des brevets**

(11) **EP 4 585 678 A1**

(12) # EUROPEAN PATENT APPLICATION

(43) Date of publication:
16.07.2025 Bulletin 2025/29

(21) Application number: 24214894.8

(22) Date of filing: 22.11.2024

(51) International Patent Classification (IPC):
*C12N 1/20* (2006.01)     *A61Q 19/00* (2006.01)
*C12R 1/01* (2006.01)

(52) Cooperative Patent Classification (CPC):
**C12N 1/205; A61K 8/99; A61K 35/745;
A61Q 19/00; A61Q 19/02; A61Q 19/08; C12N 1/20;
C12R 2001/01**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 10.01.2024 CN 202410036421

(71) Applicants:
• **Guangzhou Jiyan Cosmetics Technology Co.,
Ltd.**
**Guangzhou City, Guangdong Province 510000
(CN)**
• **Sun Yat-Sen University**
**Guangzhou, Guangdong 510275 (CN)**

(72) Inventors:
• **CHENG, Jing**
**Guangzhou City, 510000 (CN)**
• **ZHANG, Ge**
**Guangzhou City, 510000 (CN)**
• **ZHANG, Yi**
**Guangzhou City, 510000 (CN)**

• **LU, Wangwang**
**Guangzhou City, 510000 (CN)**
• **YANG, Yang**
**Guangzhou City, 510000 (CN)**
• **ZHOU, Xie**
**Guangzhou City, 510000 (CN)**
• **DOU, Shuheng**
**Guangzhou City, 510000 (CN)**
• **PAN, Wenchan**
**Guangzhou City, 510000 (CN)**
• **ZHANG, Tao**
**Guangzhou City, 510000 (CN)**
• **JOURDAN, Eric**
**08008 Barcelona (ES)**
• **GARNIER, Mathilde**
**TOULOUSE, 31300 (FR)**

(74) Representative: **Vidon Brevets & Stratégie
16B, rue de Jouanet
35700 Rennes (FR)**

Remarks:
The complete document including Reference
Table(s) and the Sequence Listing(s) can be
downloaded from the EPO website

(54) **BIFIDOBACTERIUM LONGUM SUBSP. INFANTIS, MICROBIAL AGENT AND USE THEREOF**

(57)    The present invention relates to the field of cosmetics, and particularly to *Bifidobacterium longum subsp. infantis,* a microbial agent and use thereof. The present invention provides *Bifidobacterium longum subsp. infantis,* with preservation No. CGMCC NO. 27851. A species related to in the present invention can achieve efficacies of repairing, anti-inflammation, whitening and the like in food and beauty fields, has a wider range of efficacies, and can play a better role in the field of beauty and skin care.

EP 4 585 678 A1

**Description**

**TECHNICAL FIELD**

The present invention relates to the field of cosmetics, and particularly to *Bifidobacterium longum subsp. infantis*, a microbial agent and use thereof.

**BACKGROUND ART**

Skin barrier is a self-protecting function of the skin. When the barrier is intact, the skin presents a stable and healthy state. When the barrier is damaged, it is prone to trigger a series of problems such as pimples, closed acne, allergies, redness and tingling pain. There are various main factors causing damage to the skin barrier, and repairing the skin barrier is a difficult and important point of research at present.

**SUMMARY**

In view of this, the present invention provides *Bifidobacterium longum subsp. infantis*, a microbial agent and use thereof. The species (strain) provided in the present invention can achieve efficacies of repairing, anti-inflammation, whitening and the like in the beauty field, and has more significant effects.

The present invention provides *Bifidobacterium longum subsp. infantis*, with preservation No. CGMCC NO. 27851.

The present invention further provides a microbial agent, including the above *Bifidobacterium longum subsp. infantis.*

The present invention further provides use of the above *Bifidobacterium longum subsp. infantis* and/or the above microbial agent in preparing a product for barrier repair.

In some embodiments of the present invention, in the above use, the barrier repair is the *Bifidobacterium longum subsp. infantis* and/or the microbial agent up-regulating expression of one or more genes in *FLG* gene, *LOR* gene, *IVL* gene and *AQP3* gene.

In some embodiments of the present invention, in the above use, the barrier repair is the *Bifidobacterium longum subsp. infantis* and/or the microbial agent elevating a cell migration capability.

In some embodiments of the present invention, in the above use, the barrier repair is the *Bifidobacterium longum subsp. infantis* and/or the microbial agent up-regulating expression of one or more genes in *Caspase14* gene, *DSG1* gene, *Claudin-4* gene, *ABCA12* gene, *ACACA* gene, *HMGCR* gene and *Claudin-1* gene.

The present invention further provides use of the above *Bifidobacterium longum subsp. infantis* and/or the above microbial agent in preparing a soothing product.

In some embodiments of the present invention, in the above use, the soothing is the *Bifidobacterium longum subsp. infantis* and/or the microbial agent reducing a content of PGE2.

In some embodiments of the present invention, in the above use, the soothing is the *Bifidobacterium longum subsp. infantis* and/or the microbial agent reducing a content of inflammatory factors of macrophages.

In some embodiments of the present invention, in the above use, the inflammatory factors include: IL6 and/or IL-1β.

The present invention further provides use of the above *Bifidobacterium longum subsp. infantis* and/or the above microbial agent in preparing a whitening product.

In some embodiments of the present invention, in the above use, the whitening is the *Bifidobacterium longum subsp. infantis* and/or the microbial agent inhibiting expression of tyrosinase.

In some embodiments of the present invention, in the above use, the whitening is the *Bifidobacterium longum subsp. infantis* and/or the microbial agent down-regulating expression of one or more genes of *TYR* gene, *Rab27a* gene, *Myo5a* gene, *MLPH* gene, *GPR143* gene, *SLC45A2* gene, *MITF* gene, *TRP-1* gene, *TRP-2* gene, *Pmel17* gene, *Kinesin* gene and *CDC42* gene.

The present invention further provides use of the above *Bifidobacterium longum subsp. infantis* and/or the above microbial agent in preparing an anti-aging product.

In some embodiments of the present invention, in the above use, the anti-aging is the *Bifidobacterium longum subsp. infantis* and/or the microbial agent reducing quantity and/or fluorescence intensity of CPD positive cells.

In some embodiments of the present invention, in the above use, the anti-aging is the *Bifidobacterium longum subsp. infantis* and/or the microbial agent up-regulating expression of one or more genes in *COL IV* gene, *COL VII* gene and *BGN* gene.

In some embodiments of the present invention, in the above use, the anti-aging is the *Bifidobacterium longum subsp. infantis* and/or the microbial agent down-regulating expression of one or more genes in *MMP* gene, *p16* gene, *TIMP-1* gene, *NF-κB* gene and *TNFR* gene. The anti-aging is the *Bifidobacterium longum subsp. infantis* and/or the microbial agent increasing a content of one or more of collagen fiber, hyaluronic acid, elastin and type III collagen.

The present invention further provides a product, including the above *Bifidobacterium longum subsp. infantis*, a fermentation broth of the above *Bifidobacterium longum subsp. infantis* and/or the above microbial agent and an acceptable auxiliary.

In some embodiments of the present invention, the above product includes one or more of a personal care product, a pharmaceutical product and a health product.

In some embodiments of the present invention, in the above product, the personal care product includes a cosmetic product.

In some embodiments of the present invention, the above product further includes one or more of a washing and nursing product, food, an oral nutritional product, a health product and an intestinal conditioner.

In some embodiments of the present invention, in the above product, a dosage form of the personal care product includes one or more of cream form, emulsion form, powder form and essence form.

In some embodiments of the present invention, in the above product, a dosage form of the cosmetic product includes one or more of cream form, emulsion form, powder, essence form, aqueous solution form, mask form, oil form, gel form, mud type, freeze-dried type, aerosol form, wax-based form, solid type, toiletries, lip-care form and scalp-care form.

In some embodiments of the present invention, in the above product, the personal care product includes a basic care product and/or a make-up product.

In some embodiments of the present invention, in the above product, the cosmetic product includes one or more of mask, emulsion, cream, serum, toner, face cleanser, lipstick, lip gloss, shampoo, hair care essence, body lotion, mascara and eyebrow pencil.

The present invention provides *Bifidobacterium longum subsp. infantis*, with preservation No. CGMCC NO. 27851.

The present invention isolates and screens a new strain of *Bifidobacterium longum subsp. infantis (B. longum subsp. Infantis YSGBifido001),* with preservation No. CGMCC NO. 27851.

The species provided by the present invention is isolated from breast-fed healthy infants. Experimental results indicate that the species provided by the present invention has efficacies of repairing, anti-inflammation, whitening and the like, and has more significant effects.

## BRIEF DESCRIPTION OF DRAWINGS

In order to more clearly illustrate technical solutions in the examples of the present invention or in the prior art, drawings that need to be used in the description of the examples or the prior art will be briefly introduced below.

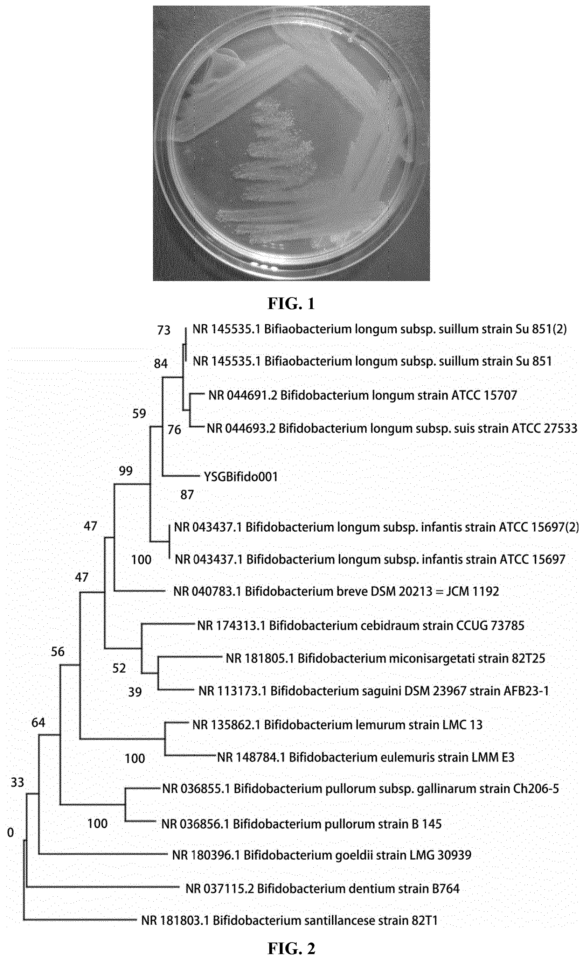

FIG. 1 is a morphological diagram of strain;
FIG. 2 shows an evolutionary tree of strain;

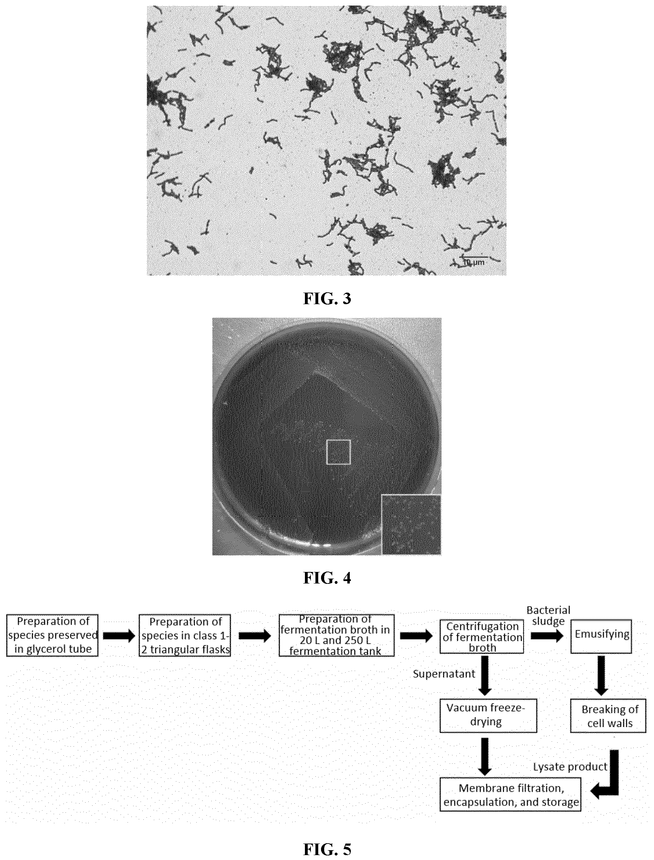

FIG. 3 shows a gram staining diagram;
FIG. 4 shows no hemolytic loop;
FIG. 5 shows a process of preparing a strain fermentation supernatant and a lysate product;
FIG. 6 shows results of strain fermentation products promoting an amount of expression of a barrier-related gene *FLG* in HaCaT cells;
FIG. 7 shows results of the strain fermentation products promoting an amount of expression of a barrier-related gene *LOR* in the HaCaT cells;
FIG. 8 shows results of the strain fermentation products promoting an amount of expression of a barrier-related gene *IVL* in the HaCaT cells;
FIG. 9 shows results of the strain fermentation products promoting an amount of expression of a barrier-related gene *AQP-3* in the HaCaT cells;
FIG. 10 shows physical photographs of the strain products promoting cell migration in a cell scratching experiment;
FIG. 11 shows a histogram of the strain fermentation products promoting relative mobility of cell migration in the cell scratching experiment;
FIG. 12 shows results of the strain fermentation products promoting an amount of expression of a barrier-related gene *Caspase14* in the HaCaT cells;
FIG. 13 shows results of the strain fermentation products promoting an amount of expression of a barrier-related *gene DSG1* in the HaCaT cells;
FIG. 14 shows results of the strain fermentation products promoting an amount of expression of a barrier-related gene *Claudin-4* in the HaCaT cells;
FIG. 15 shows results of the strain fermentation products promoting an amount of expression of a barrier-related gene *ABCA12* in the HaCaT cells;

FIG. 16 shows results of the strain fermentation products promoting an amount of expression of a barrier-related gene *ACACA* in the HaCaT cells;

FIG. 17 shows results of the strain fermentation products promoting an amount of expression of a barrier-related gene *HMGCR* in the HaCaT cells;

FIG. 18 shows results of the strain fermentation products promoting an amount of expression of a barrier-related gene *Claudin-1* in the HaCaT cells;

FIG. 19 shows results of the strain fermentation products improving tissue morphology of 3D epidermal skin model stimulated and damaged by SLS;

FIG. 20 shows results of the strain fermentation products inhibiting inflammatory factor PGE2 on keratinocytes;

FIG. 21 shows results of the strain fermentation products inhibiting inflammatory factor *IL-6* on macrophages;

FIG. 22 shows results of the strain fermentation products inhibiting inflammatory factor *IL-1β* on macrophages;

FIG. 23 shows physical photographs of the strain fermentation products promoting exfoliation on excised suckling pig skin;

FIG. 24 shows a histogram of total protein concentration of the strain fermentation products promoting exfoliation on the excised suckling pig skin;

FIG. 25 shows anti-oxidation results of the strain fermentation products removing ROS on keratinocytes;

FIG. 26 shows clearance rates of DPPH free radicals by the strain fermentation products;

FIG. 27 shows effect of the strain fermentation products promoting expression of an antioxidation-related gene *NQO1* on keratinocytes;

FIG. 28 shows effect of the strain fermentation products promoting expression of an antioxidation-related gene *CAT* on keratinocytes;

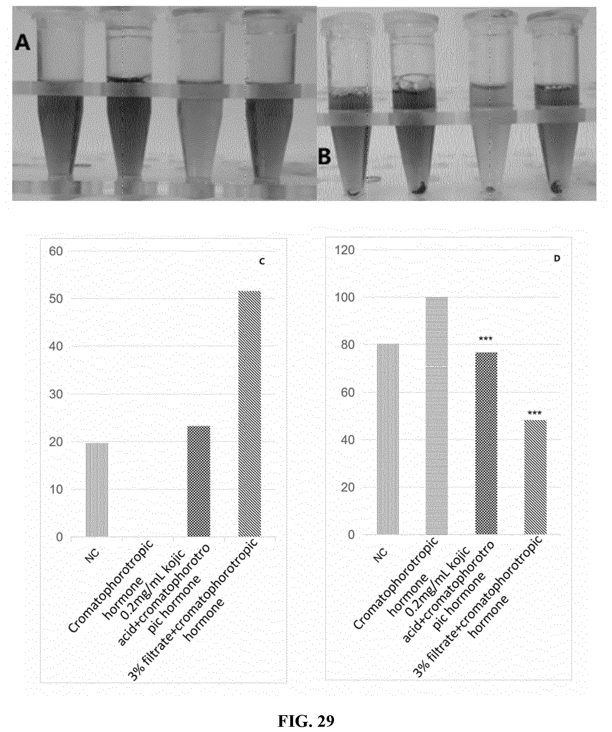

FIG. 29 shows results of the strain fermentation products inhibiting tyrosinase activity on mouse melanoma cells B 16F 10, where A shows a photograph of parallel tests of strain lysate products, being NC, cromatophorotropic hormone, 3% strain filtrate, and 0.2 mg/mL kojic acid in turn from left to right; B shows photographs of parallel tests of strain filtrates, being NC, cromatophorotropic hormone, 3% strain filtrate, and 0.2 mg/mL kojic acid in turn from left to right; C shows inhibition rates (%) of tyrosinase activity in a strain filtrate group; and D shows tyrosinase activity (%) in the strain filtrate group.

FIG. 30 shows results of the strain fermentation products inhibiting expression of a tyrosinase gene *TYR* on melanocytes;

FIG. 31 shows results of the strain fermentation products inhibiting expression of a melanosome transport-related gene *Rab27a* on melanocytes;

FIG. 32 shows results of the strain fermentation products inhibiting expression of a melanosome transport-related gene *Myo5a* on melanocytes;

FIG. 33 shows results of the strain fermentation products inhibiting expression of a melanosome transport-related gene *MLPH* on melanocytes;

FIG. 34 shows results of the strain fermentation products inhibiting expression of a melanin synthesis-related gene *GPR143* on melanocytes;

FIG. 35 shows results of the strain fermentation products inhibiting expression of a melanin synthesis-related gene *SLC45A2* on melanocytes;

FIG. 36 shows results of the strain fermentation products inhibiting expression of a melanin synthesis-related gene *MITF* on melanocytes;

FIG. 37 shows results of the strain fermentation products inhibiting expression of a melanin synthesis-related gene *TRP-1* on melanocytes;

FIG. 38 shows results of the strain fermentation products inhibiting expression of a melanin synthesis-related gene *TRP-2* on melanocytes;

FIG. 39 shows results of the strain fermentation products inhibiting expression of a melanosome transport-related gene *Pmel17* on melanocytes;

FIG. 40 shows results of the strain fermentation products inhibiting expression of a melanosome transport-related gene *Kinesin* on melanocytes;

FIG. 41 shows results of the strain fermentation products inhibiting expression of a melanosome transport-related gene *CDC42* on melanocytes;

FIG. 42 shows results of the strain fermentation products reducing UVB-induced CPD positive cells on a 3D epidermal model;

FIG. 43 shows results of the strain fermentation products reducing UVB-induced CPD mean fluorescence intensity on the 3D epidermal model;

FIG. 44 shows physical results of the strain fermentation products improving UVB-induced tissue morphology on the 3D epidermal model;

FIG. 45 shows results of the strain fermentation products reducing the quantity of UVB-induced damaged cells on the 3D epidermal model;

FIG. 46 shows results of the strain fermentation products promoting expression of collagen *COL I* gene on fibroblasts;

FIG. 47 shows results of the strain fermentation products promoting expression of collagen *COL III* gene on fibroblasts;

FIG. 48 shows results of the strain fermentation products promoting expression of collagen *COL IV* gene on fibroblasts;

FIG. 49 shows results of the strain fermentation products promoting expression of collagen *COL VII* gene on fibroblasts;

FIG. 50 shows results of the strain fermentation products promoting expression of proteoglycan-related gene *DCN* on fibroblasts;

FIG. 51 shows results of the strain fermentation products promoting expression of proteoglycan-related gene *VCAN* on fibroblasts;

FIG. 52 shows results of the strain fermentation products promoting expression of proteoglycan-related gene *BGN* on fibroblasts;

FIG. 53 shows results of the strain fermentation products promoting expression of elastin on fibroblasts;

FIG. 54 shows results of the strain fermentation products promoting expression of fibronectin on fibroblasts;

FIG. 55 shows results of the strain fermentation products promoting expression of hyaluronic acid synthase *HAS1* on fibroblasts;

FIG. 56 shows results of the strain fermentation products inhibiting matrix metalloproteinase MMP-1 on fibroblasts;

FIG. 57 shows results of the strain fermentation products inhibiting expression of inflammatory factor *NF-κB* gene on fibroblasts;

FIG. 58 shows results of the strain fermentation products inhibiting expression of aging-related gene *p21* on fibroblasts;

FIG. 59 shows results of the strain fermentation products inhibiting expression of aging-related gene *p21* on fibroblasts;

FIG. 60 shows results of the strain fermentation products inhibiting expression of aging-related gene *MMP-1* on fibroblasts;

FIG. 61 shows results of the strain fermentation products inhibiting expression of aging-related gene *MMP-3* on fibroblasts;

FIG. 62 shows results of the strain fermentation products inhibiting expression of aging-related gene *TIMP-1* on fibroblasts;

FIG. 63 shows results of the strain fermentation products inhibiting expression of aging-related gene *TNFR* on fibroblasts;

FIG. 64 shows photographs of the strain fermentation products promoting Masson staining of collagen fiber on excised skin;

FIG. 65 shows relative area mean of the strain fermentation products promoting collagen fiber generation on the excised skin;

FIG. 66 shows fluorescence photographs of the strain fermentation products promoting hyaluronic acid generation on the excised skin;

FIG. 67 shows a histogram of relative IOD mean value of the strain fermentation products promoting hyaluronic acid generation on the excised skin;

FIG. 68 shows Victoria Blue staining photographs of the strain fermentation products promoting elastin generation on the excised skin;

FIG. 69 shows a histogram of relative IOD mean of the strain fermentation products promoting elastin generation on the excised skin;

FIG. 70 shows IHC staining photographs of the strain fermentation products promoting type III collagen generation on the excised skin;

FIG. 71 shows a histogram of relative IOD mean of the strain fermentation products promoting type III collagen generation on the excised skin;

FIG. 72 shows IHC staining photographs of the strain fermentation products promoting type IV collagen generation on the excised skin;

FIG. 73 shows a histogram of relative IOD mean of the strain fermentation products promoting type IV collagen generation on the excised skin;

FIG. 74 shows IHC staining photographs of the strain fermentation products promoting type XVII collagen generation on the excised skin;

FIG. 75 shows a histogram of relative IOD mean of the strain fermentation products promoting type XVII collagen generation on the excised skin;

FIG. 76 shows results of the strain fermentation products promoting expression of autophagy-related gene *LC3-II* on fibroblasts;

FIG. 77 shows results of cell fluorescence pictures of the strain fermentation products promoting generation of autophagy-related protein Beclin 1 on fibroblasts; and

FIG. 78 shows a histogram of relative IOD mean of the strain fermentation products promoting generation of autophagy-related protein Beclin 1 on fibroblasts.

## DESCRIPTION OF BIOLOGICAL PRESERVATION

Biomaterial: YSGBifido001; Classification name: *Bifidobacterium longum subsp. infantis*, deposited on July 10, 2023 at the China General Microbiological Culture Collection Center; Address: No. 3, Yard No. 1, Beichen West Road, Chaoyang District, Beijing, Institute of Microbiology, Chinese Academy of Sciences; and Preservation No.: CGMCC No. 27851.

## DETAILED DESCRIPTION OF EMBODIMENTS

The present invention discloses *Bifidobacterium longum subsp. infantis*, a microbial agent and use thereof.

It should be understood that the expression "one or more of..." separately includes each object recited after the expression as well as various different combinations of two or more of the recited objects, unless otherwise understood from the context and usage. The expression "and/or" in conjunction with three or more recited objects should be understood to have the same meaning, unless otherwise understood from the context.

The terms "including", "having" or "containing, including use of grammatical equivalents thereof, should generally be understood as open-ended and non-limiting, for example, not excluding other unrecited elements or steps, unless specifically stated otherwise or otherwise understood from the context.

It should be understood that the order of steps or the order of performing certain actions is not important as long as the present invention still can be implemented. In addition, two or more steps or actions may be performed simultaneously.

The use of any and all examples or exemplary language such as "for example" or "including" herein is merely intended to better illustrate the present invention and does not constitute limitation on the scope of the present invention unless otherwise claimed. No language in the present description should be construed as indicating any non-claimed element as essential to the practice of the present invention.

In addition, numerical ranges and parameters used to define the present invention are all approximate numerical values, and relevant numerical values in specific examples have been presented herein as precisely as possible. However, any numerical values inherently inevitably contain standard deviations due to individual testing methods. Accordingly, unless otherwise expressly indicated, it should be understood that all ranges, amounts, numerical values and percentages used in the present invention are to be modified by "about". Herein, "about" generally means that an actual numerical value is a particular numerical value or within a range plus or minus 10%, 5%, 1% or 0.5%.

Source of species of the present invention:

Microbial source (bacterial source), DNA sequence being verified as an exclusive species, CGMCC NO.: 27851.

Class of the species of the present invention:

*Bifidobacterium longum subsp. infantis*, bacterium.

Introduction of the species of the present invention:

YSGBifido001 is isolated from 4-month old Guangzhou breast-fed healthy infants and identified as *Bifidobacterium longum subsp. infantis* through 16S rDNA sequence alignment. It grows well in MRS culture medium (solid/liquid), and can reach a logarithmic growth phase after being culture for 1 day under an anaerobic condition. A colonial morphology of strains growing on the MRS solid culture medium is milky white and round, with a regular edge. The strains grow on a blood plate without obvious hemolytic ring. After gram staining, bacteria are blue-purple under microscope, and rod-shaped, being thin in the middle and expanded at two ends, like a "barbell".

The present invention is further illustrated below in conjunction with examples.

In Example 1 to Example 4 and Verification Example 1 to Verification Example 9 of the present invention, all of raw materials and reagents used are commercially available.

Example 1 The present example performed isolation and purification on strains of ***Bifidobacterium longum subsp. infantis***

(1) Experimental method

1) Feces collection: Feces samples of Guangzhou breast-fed infants were collected. Into a cell culture flask, 20 mL of a bacterial cryopreservation solution was added, and feces just excreted were collected into the cell culture flask by using a sterilized cotton swab. The culture flask was sealed in an anaerobic bag, and stored and transported in an ice box.

2) Dilution and coating: After shaking, 100 μL of fecal suspension was pipetted into 900 μL of a PBS buffer solution and pipetted and evenly mixed by a pipette gun, and then 100 μL of suspension was pipetted again into 900 μL of the PBS buffer solution. In this way, gradient dilution was performed sequentially for 10 times. 20 μL of diluted solutions of each gradient were taken, respectively applied to MRS, BHI and CBA plates, and cultured at 37 °C for 48-72 h under an aerobic or anaerobic condition.

3) Single-colony enrichment culture: A plate with 5-100 colonies was selected, a single colony was randomly picked using a disposable inoculating loop into 20 μL of PBS, and pipetted and mixed evenly. 15 μL of bacterial solution was taken and coated onto the same kind of plate, and cultured at 37 °C for 48-72 h under an aerobic or anaerobic condition. Additional 5 μL of the bacterial solution was left for gram-staining identification.

4) Bacterial passage: Bacteria growing on the plate were scrapped using the disposable inoculating loop as much as possible, and re-suspended in 200 μL of the PBS buffer solution, and then 200 μL of the bacterial solution was re-coated onto the same kind of plate, and cultured for 48-72 h under the same condition.

5) Cryopreservation of bacteria: After the bacteria reached a sufficient amount through enrichment culture, the bacteria on the plate were collected by using the inoculating loop in a sterile cryopreservation solution formed by mixing fetal calf serum, BHI culture medium, and glycerin, and pipetted and mixed evenly, and the species was stored at -80 °C. At the same time, a suitable amount of bacteria were left to extract bacterial genomic DNA.

6) Gram staining identification: 5 μL of the bacterial solution prepared when picking the single colony was added dropwise to a center of a glass slide, intermittently baked using an alcohol lamp and fixed, covered for 1 min by a crystal violet staining solution dropwise added, washed with running water, then stained for 1 min with dropwise addition of a Lugol's iodine solution, and washed with running water. Then a surface of the glass slide was covered with decolorized alcohol for 20-30s, during which period the glass slide was gently shaken. After the alcohol was washed off with running water, re-staining was performed for 1 min with a safranin staining solution. Washing was performed with running water. After the glass slide was dried, bacterial morphology was observed using an optical microscope. If the colony was not pure and more than one kind of bacterial morphology existed, streak inoculation was performed on the sample, and a single colony was picked up again after culture.

7) Extraction of bacterial genomic DNA: A bacterial genomic DNA extraction kit was used to extract the bacterial genomic DNA according to instructions of Tiangen Biotec (Beijing) Co., Ltd., and a DNA concentration was measured using NanoDrop 2000.

8) 16S fragment amplification:

<1> Primer pair 27F/1492R was used, 27F sequence: 3'-AGAGTTTGATCMTGGCTCAG-5' (as set forth in SEQ ID NO: 1).

1492R sequence: 3'-GGTTACCTTGTTACGACTT-5' (as set forth in SEQ ID NO: 2).

Reaction system was as follows:

| Reagent | Volume |
| --- | --- |
| LA Taq enzyme | 15 μL |
| PCR Forward Primer ( 10 μM) | 1.2 μL |
| PCR Reverse Primer ( 10 μM) | 1.2 μL |
| Bacterial genomic DNA | 3 μL |
| ddH₂O | up to 30 μL |

<2> PCR amplification program was as follows: pre-denaturation at 95 °C for 60 s; denaturation at 95 °C for 30 s, renaturation at 56 °C for 30 s, extension at 72 °C for 90 s, cycle number being 31; re-extension at 72 °C for 7 min, and end temperature being set at 4 °C.

9) Agarose gel electrophoresis: 25 mL of a 1×TAE buffer solution containing 1% (w/v) agarose was formulated, heated and melted using a microwave oven, and added with 2.5 μL of a nucleic acid dye GoldView. They were evenly mixed and then poured into a gel-cast tank, into which a comb was inserted to wait for cooling thereof. After the agarose gel was cooled, the comb was removed, and the agarose gel was put into a horizontal electrophoresis tank. Into each well 5 $\mu$L of amplified DNA sample and 3 $\mu$L of DL2000 DNA marker were added. Power was turned on, constant-voltage electrophoresis was performed at 110 V for 20 min, and then it was observed under an ultraviolet lamp whether amplified bands were singular and had a size of about 1500 bp.

10) 16S rRNA sequencing and result alignment: DNA 16S fragment samples having been amplified and confirmed to be error-free by the agarose gel electrophoresis were sent to Shanghai Sangon Company for sequencing. After a sequencing result was obtained, sequence assembly was performed using ChromasPro software, and 16S rRNA sequence blast was performed on NCBI website (https://blast.ncbi.nlm.nih.gov/Blast.cgi), to primarily identify a type of bacteria.

(2). Experimental results

The isolated and identified strains were named as *Bifidobacterium longum subsp. infantis* YSGBifido001, preserved at the China General Microbiological Culture Collection Center on July 10, 2023, with preservation number CGMCC No. 27851, at the address No. 3, Yard No. 1, Beichen West Road, Chaoyang District, Beijing. Nucleotide sequence of 16S rDNA of *B. longum subsp. Infantis* YSGBifido001 is as set forth in SEQ ID NO: 3. 16S rDNA thereof has at least two base mutation sites compared with known strains of *Bifidobacterium longum* (National Center for Biotechnology database), and the most closely related strain is *B. longum subsp. infantis strain* ATCC 15697.

[0145] Sequence: 3'-

TGCAAGTCGAACGGGATCCATCAGGCTTTGCTTGGTGGTGAGAGTGGCGAACGGGT
GAGTAATGCGTGACCGACCTGCCCCATACACCGGAATAGCTCCTGGAAACGGGTGG
TAATGCCGGATGTTCCAGTTGATCGCATGGTCTTCTGGGAAAGCTTTCGCGGTATGG
GATGGGGTCGCGTCCTATCAGCTTGACGGCGGGGTAACGGCCCACCGTGGCTTCGA
CGGGTAGCCGGCCTGAGAGGGCGACCGGCCACATTGGGACTGAGATACGGCCCAGA
CTCCTACGGGAGGCAGCAGTGGGGAATATTGCACAATGGGCGCAAGCCTGATGCAG
CGACGCCGCGTGAGGGATGGAGGCCTTCGGGTTGTAAACCTCTTTTATCGGGGAGC
AAGCGTGAGTGAGTTTACCCGTTGAATAAGCACCGGCTAACTACGTGCCAGCAGCC
GCGGTAATACGTAGGGTGCAAGCGTTATCCGGAATTATTGGGCGTAAAGGGCTCGT
AGGCGGTTCGTCGCGTCCGGTGTGAAAGTCCATCGCTTAACGGTGGATCCGCGCCGG
GTACGGGCGGGCTTGAGTGCGGTAGGGGAGACTGGAATTCCCGGTGTAACGGTGGA
ATGTGTAGATATCGGGAAGAACACCAATGGCGAAGGCAGGTCTCTGGGCCGTTACT
GACGCTGAGGAGCGAAAGCGTGGGGAGCGAACAGGATTAGATACCCTGGTAGTCCA
CGCCGTAAACGGTGGATGCTGGATGTGGGGCCCGTTCCACGGGTTCCGTGTCGGAG
CTAACGCGTTAAGCATCCCGCCTGGGGAGTACGGCCGCAAGGCTAAAACTCAAAGA
AATTGACGGGGGTCCGCACAAGCGGCGGAGCATGCGGATTAATTCGATGCAACGCG
AAGAACCTTACCTGGGCTTGACATGTTCCCGACGATCCCAGAGATGGGGTTTCCCTT
CGGGGCGGGTTCACAGGTGGTGCATGGTCGTCGTCAGCTCGTGTCGTGAGATGTTGG
GTTAAGTCCCGCAACGAGCGCAACCCTCGCCCCGTGTTGCCAGCGGATTGTGCCGGG
AACTCACGGGGGACCGCCGGGGTTAACTCGGAGGAAGGTGGGGATGACGTCAGATC
ATCATGCCCCTTACGTCCAGGGCTTCACGCATGCTACAATGGCCGGTACAACGGGAT
GCGACGCGGCGACGCGGAGCGGATCCCTGAAAACCGGTCTCAGTTCGGATCGCAGT
CTGCAACTCGACTGCGTGAAGGCGGAGTCGCTAGTAATCGCGAATCAGCAACGTCG
CGGTGAATGCGTTCCCGGGCCTTGT*ACACA*CCGCCCGTCAAGTCATGAAAGTGGGCA
GCA-5' (as set forth in SEQ ID NO: 3).

**Example 2 Production process of strain filtrate of *Bifidobacterium longum subsp. infantis***

(1). Preparation of glycerol tube species (species in glycerol tube)

1). Activation of original lyophilized powder species: Lyophilized powder was transferred into a 100 mL HuanKai MRS triangular flask medium, and anaerobically stood and cultured at 37 °C for 20-24 h to render a bacterial solution for later use.
2). Isolation of single colony: MRS solid medium plate with the bacterial solution was subjected to scribing separation, and anaerobically stood and cultured at 37 °C for 48 h, and the single colony grew up for later use.
3). Activation of single colony: The single colony was transferred into a 100 mL liquid triangular flask medium (as shown in TABLE 1), and anaerobically stood and cultured at 37 °C for 20 h to render a bacterial solution. OD and pH were detected through microscopic examination to see whether standards OD* 10 > 0.260 and pH <4.50 were reached.

4). Breed conservation of original glycerol tube species: An inoculum size of 5% of the above bacteria (after 20-24 h of culture, OD * 10 > 0.260 was detected, which can determine culture to be qualified) was transferred into a triangular flask medium of a formula card 1 (as shown in TABLE 1) and cultured for 20 h to detect OD*10> 0.260 and pH <4.50. After standards were reached through microscopic examination, the species glycerol tube (0.70 mL of a breed conservation liquid and 0.70 mL of the bacterial solution) was subjected to breed conservation. Storage life of glycerol tube species at -80 °C is 3 months, and the species is preserved once after 3 months of passage. (the species preserved with the glycerol tube needs to be passaged regularly, once in 3 months, and whether the species is polluted or not must be checked in each passage. During identification, the species can be inoculated on an MRS plate and subjected to gram staining microscopic examination. Species in the logarithmic growth phase cell-age is used for biochemical identification and 16S rDNA sequencing where necessary, and working strains can be inoculated on a slant agar culture medium.)

5). Establishment of seed lot: A system of seed lots should be established for species for production according to relevant regulations of "Management and Quality Control of Bacterial and Virus Strains for Production and Testing of Biological Products". Three stages of seed lots should be respectively lyophilized and stored at an appropriate temperature; the number of passages should be limited for the seed lot passage, where after a primary seed lot and a master seed lot are started to passage, the number of passages should not exceed 10, and the number of passages from starting of the working seed lot to fermentation culture should not exceed 5.

(2). The strain *Bifidobacterium longum subsp. infantis* obtained in step (1) was inoculated into a culture medium (formula of the culture medium is as shown in TABLE 1) in an inoculum size (after 20-24 h of culture, OD * 10 > 0.260 was detected, which can determine the culture to be qualified) of 5%, and cultured at 37 °C for 20-24 h. Resultant was centrifuged and precipitated, and supernatant was taken, and freeze-dried and concentrated by five times, to render a concentrated fermentation broth.

TABLE 1

| Formula Card 1 | |
| --- | --- |
| Strain culture medium | Triangular flask (g /L) |
| Glucose | 12 |
| Wheat peptone | 10 |
| Yeast powder | 10 |
| Sodium acetate | 2 |
| Dipotassium phosphate | 2 |
| Triammonium citrate | 2 |
| Magnesium sulfate heptahydrate | 0.58 |
| Manganese sulfate | 0.05 |
| Tween 80 | 2 |

(3). Ingredients of a concentrated fermentation broth of strain obtained in step (2) are as shown in TABLE 2.

TABLE 2

| Ingredient | Content |
| --- | --- |
| Glycollic acid | 17% |
| Pyroglutamic acid | 8.5% |
| Citric acid | 6% |
| Hydroxylamine | 4.5% |
| Sorbitol | 3% |

**Example 3 Production process of strain lysate**

(1). The strain *Bifidobacterium longum subsp. infantis* obtained in step (1) of Example 2 was inoculated into a culture medium (formula of the culture medium is as shown in TABLE 1) in an inoculum size of 5%, and cultured at 37 °C for 20-24 h. After precipitate was obtained through centrifugation, a PBS buffer solution was added to emulsify until uniform, and cell walls were broken to render a lysate product.

(2). Ingredients of the strain lysate product obtained are as shown in TABLE 3.

### TABLE 3

| Ingredient | Content |
|---|---|
| Glycollic acid | 17% |
| Pyroglutamic acid | 4% |
| Hydroxylamine | 2% |
| Deoxyerythritol | 2% |
| D-talose | 1 % |

**Example 4 Production process of strain filtrate+lysate**

The filtrate obtained in Example 2 the lysate product obtained in Example 3 were mixed in 1:1 to render the strain filtrate+lysate.

**Verification Example 1 Assay of total acid, pH, and total protein**

The filtrate obtained in Example 2 and the lysate obtained in Example 3 were detected respectively.

(1) Total acid content assay
An acid-base titration method was used to assay.
(2) pH assay
A pH meter was used to assay.
(3) Total protein content assay
A detection kit was used to assay. BCA protein concentration assay kit (Biosharp company)

Experimental results are as shown in TABLE 4.

### TABLE 4

| | Total Acid ($\mu$mol/mL) | pH | Total Protein (mg/mL) |
|---|---|---|---|
| Filtrate | 0.3~0.5 | 3.5~4.5 | 14~16 |
| 5-fold concentrated filtrate | 0.7~0.9 | 3.5~4.5 | 27~29 |
| Lysate | 0.08~0.1 | 4.5~5.5 | 0.9~1.1 |

**Verification Example 2**

(1). Competitor: CLR bifida ferment lysate (Repair Complex CLR PF), 1%, 3%, 5%;

Method: qPCR;
Model: HaCat;
Indexes: *FLG, LOR, IVL, AQP3.*

(2) Experimental results
Conclusion: Fermentation product increases amplification folds of all of *FLG, LOR, IVL, AQP3* genes.

1) *FLG* PCR

*FLG* is a key ingredient in a CE assembly process. Besides being a structural composition of the skin barrier, *FLG* can also be hydrolyzed by *Caspase-14* to form a natural moisturizing factor, and thus also has a moisturizing effect.

*FLG* primer: F: 5'-AGGGAAGATCCAAGAGCCCA-3' (as set forth in SEQ ID NO: 4); and R: 5'-ACTCTGGATCCCCTACGCTT-3' (as set forth in SEQ ID NO: 5).

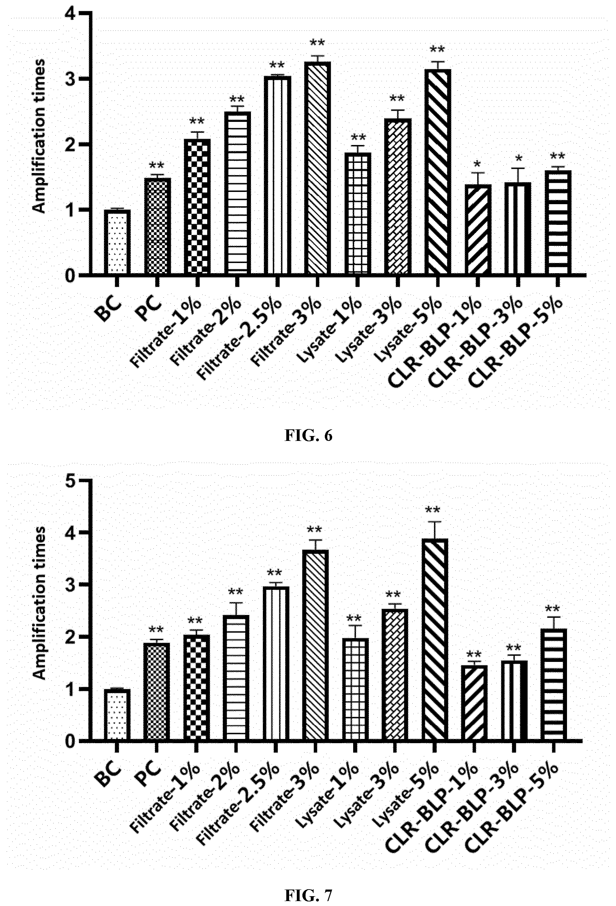

Experimental results are as shown in TABLE 5 and FIG. 6. The filtrate or the lysate product have a high amount of *FLG* gene expression at a low concentration; and through comparison with the same competitor CLR, it can found that the fermentation product has higher increase in the *FLG* gene expression. The filtrate or the lysate product can achieve a repairing efficacy by up-regulating the *FLG* gene.

TABLE 5 Experimental Results

| | | Test Results | | |
|---|---|---|---|---|
| | Sample | Concentration | Result | |
| *FLG* | Filtrate | 1.0% | 109%** | |
| | | 2.0% | 150%** | |
| | | 2.5% | 204%** | |
| | | 3.0% | 226%** | |
| | Lysate | 1.0% | 87%** | |
| | | 3.0% | 140%** | |
| | | 5.0% | 215%** | |
| | CLR-BLP | 1.0% | 39%* | |
| | | 3.0% | 41%* | |
| | | 5.0% | 60%** | |

In the above: * indicates significant difference, and ** indicates extremely significant difference.

2) *LOR* PCR

Loricrin, *LOR,* is a key component in an assembly process of protein envelope CE, accounts for about 80% of CE content, and has a reinforcing effect on the skin barrier. Reduced content thereof is a major factor of weakening of the skin barrier functions.

LOR primer: F: 5'-CTTGGAACATGGCCTAGCTC-3' (as set forth in SEQ ID NO: 6); and R: 5'-CATTCCCAGCACAACAGAGA-3' (as set forth in SEQ ID NO: 7).

Experimental results are as shown in TABLE 6 and FIG. 7. The filtrate or the lysate product have a high amount of *LOR* gene expression at a low concentration; and through comparison with the same competitor CLR, it can found that the fermentation product has higher increase in the *LOR* gene expression. The filtrate or the lysate product can achieve a repairing efficacy by up-regulating the *LOR* gene.

TABLE 6 Experimental Results

| | | Test Results | | |
|---|---|---|---|---|
| | Sample | Concentration | Result | |
| *LOR* | Filtrate | 1.0% | 104%** | |
| | | 2.0% | 142%** | |
| | | 2.5% | 197%** | |
| | | 3.0% | 267%** | |
| | Lysate | 1.0% | 98%** | |
| | | 3.0% | 154%** | |
| | | 5.0% | 289%** | |
| | CLR-BLP | 1.0% | 46%** | |
| | | 3.0% | 55%** | |
| | | 5.0% | 116%** | |

In the above: * indicates significant difference, and ** indicates extremely significant difference.

3) *IVL* PCR

*IVL* involucrin is an early differentiation index. This index, together with *FLG, LOR* and *TGM1*, is involved in cross-linking and formation of barrier function-related structural protein envelop, and is one of substance foundations of the skin barrier functions. Reduced content thereof affects the skin barrier functions.

*IVL* primer: F: 5'-AAAGAAGAGCAGGTGCTGGA-3' (as set forth in SEQ ID NO: 8); and
R: 5'-TGCTCACTGGTGTTCTGGAG-3' (as set forth in SEQ ID NO: 9).

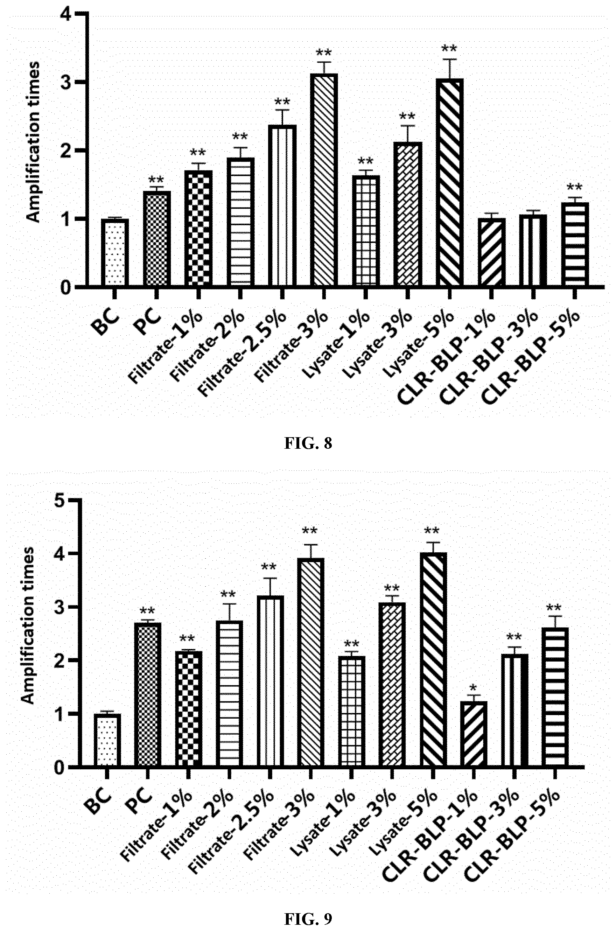

Experimental results are as shown in TABLE 7 and FIG. 8. The filtrate or the lysate product have a high amount of *IVL* gene expression at a low concentration; and through comparison with the same competitor CLR, it can found that the fermentation product has higher increase in the *IVL* gene expression. The filtrate or the lysate product can achieve a repairing efficacy by up-regulating the *IVL* gene.

TABLE 7 Test Results

| | | Test Results | |
|---|---|---|---|
| | Sample | Concentration | Result |
| *IVL* | Filtrate | 1.0% | 71%** |
| | | 2.0% | 90%" |
| | | 2.5% | 137%** |
| | | 3.0% | 213%** |
| | Lysate | 10% | 6,4%** |
| | | 3.0% | 112%** |
| | | 5.0% | 205%** |
| | CLR-BLP | 1.0% | 1% |
| | | 3.0% | 6% |
| | | 5.0% | 24%** |

In the above: * indicates significant difference, and ** indicates extremely significant difference.

4) *AQP-3*

Aquaporin, a protein (integral membrane protein) located on cell membrane, forms a "pore channel" on the cell membrane, and can control ingress and egress of water in cell. *AQP-3* has a function of transporting small molecular substances such as water, glycerin and urea across a membrane.

*AQP-3* primer: F: 5'-CAGCCTCCCTTATCGTGTGT-3' (as set forth in SEQ ID NO: 10); and
R: 5'-CAAGGGCTGTAAAAAGGCGG-3' (as set forth in SEQ ID NO: 11).

Experimental results are as shown in TABLE 8 and FIG. 9. The filtrate or the lysate product have a high amount of *AQP3* gene expression at a low concentration; and through comparison with the same competitor CLR, it can found that the fermentation product has higher increase in the *AQP3* gene expression. The filtrate or the lysate product can achieve a repairing efficacy by up-regulating the *AQP3* gene.

TABLE 8 Test Results

| | | Test Results | |
|---|---|---|---|
| | Sample | Concentration | Result |
| *AQP3* | Filtrate | 1.0% | 118%** |
| | | 2.0% | 175%** |
| | | 2.5% | 222%** |
| | | 3.0% | 292%** |
| | Lysate | 1.0% | 108%** |
| | | 3.0% | 209%** |
| | | 5.0% | 302%** |

(continued)

| Test Results | | |
|---|---|---|
| Sample | Concentration | Result |
| | 1.0% | 57%* |
| CLR-BLP | 3.0% | 112%** |
| | 5.0% | 162%** |

In the above: * indicates significant difference, and ** indicates extremely significant difference.

**Verification Example 3 Repair-keratinocytes-based test**

(1). Cell scratch

TABLE 9

| Group | Sample Name | Dosing Concentration | Detection Model | Detection Index | Detection Method |
|---|---|---|---|---|---|
| Blank control (BC) | / | / | | | |
| Positive control (PC) | EGF | 1ng/mL | | | |
| | Filtrate | 0.3125% | | | Scratch (micro-scopi photo-graphing and observation) |
| | Lysate | 0.5% | Keratinocyte | Cell mobility | |
| Sample group | Filtrate+lysate | 0.0781% | | | |
| | Madecassoside | 0.625mg/mL | | | |
| | B5 | 1.25mg/mL | | | |

In the above, in the filtrate+lysate, the filtrate accounts for 50%, and the lysate accounts for 50%.
Cell migration capability is an important capability of repairing after tissue damage. After the tissue damage, the body will initiate repair of a damaged part, including enhancement of longitudinal cell proliferation capability and lateral crawling and migration of cells in surrounding parts of the damage to the damaged part. Cells cover the damaged part by crawling and migration.

1). Testing method

Cell inoculation: After resuscitation of cells, when a plating rate reached about 60%, the cells were inoculated in 6-well plates, and incubated overnight in a $CO_2$ incubator (37 °C, 5% $CO_2$).
Dosing: According to test groups in TABLE 9, when the plating rate of the cells in the 6-well plates reached about 70%-80%, dosing was performed for the groups respectively, with each group in triplicate. The blank control group was added with 2 mL of a culture solution per well, the positive control group was added with 2 mL of an EGF-containing culture solution per well, and the sample group was added with 2 mL of a test sample-containing culture solution at corresponding concentration per well. After the dosing was completed, the 6-well plates continued to be cultured in the $CO_2$ incubator (37 °C, 5% $CO_2$) for 24 h.
Scratching: After posterior incubation was finished, scratching was performed.
Cleaning: After the scratching was finished, the cells were cleaned in PBS for three times, added into a cell culture solution, and cultured in the $CO_2$ incubator (37 °C, 5% $CO_2$) for 24 h.
Photographing: 0 h after the scratching, photographs were taken under a 4x microscope, and 24 h after the scratching, PBS cleaning was performed once, and photographs were taken under the 4x microscope.

Calculation of increase rate: increase rate = (sample group - blank control group)/ blank control group $\times$ 100%

Statistical analysis of results: GraphPad Prism was applied for plotting, and results were expressed as Mean $\pm$ sD. Comparison between the groups was statistically analyzed using t-test. Statistical analysis was two-tailed. $P$<0.05 is considered to have a significant difference, and $P<0.01$ is considered to have an extremely significant difference.

2). Test results

TABLE 10

| Sample Name | Relative Mobility Mean | Relative Mobility SD | P-value | Increase Rate |
|---|---|---|---|---|
| BC | 1.00 | 0.04 | / | / |
| PC | 1.41 | 0.04 | 0.000** | / |
| Filtrate-0.3125% | 1.09 | 0.14 | 0.170 | 9.00% |
| Lysate-0.5% | 1.20 | 0.05 | 0.000** | 20.00% |
| Filtrate+lysate-0.0781% | 1.24 | 0.12 | 0.001 ** | 24.00% |
| Madecassoside -0.625 mg/mL | 1.02 | 0.15 | 0.766 | 2.00% |
| B5-1.25 mg/mL | 1.04 | 0.14 | 0.485 | 4.00% |

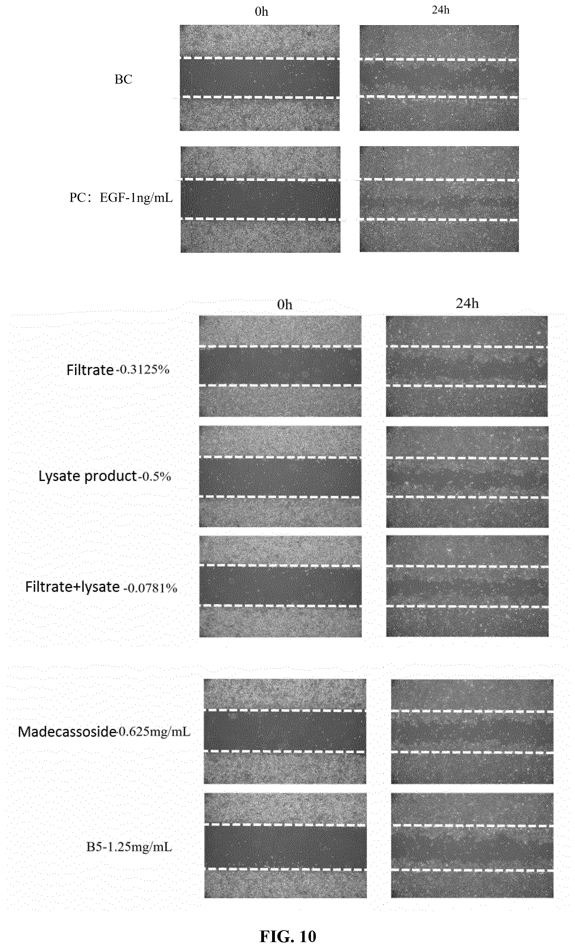

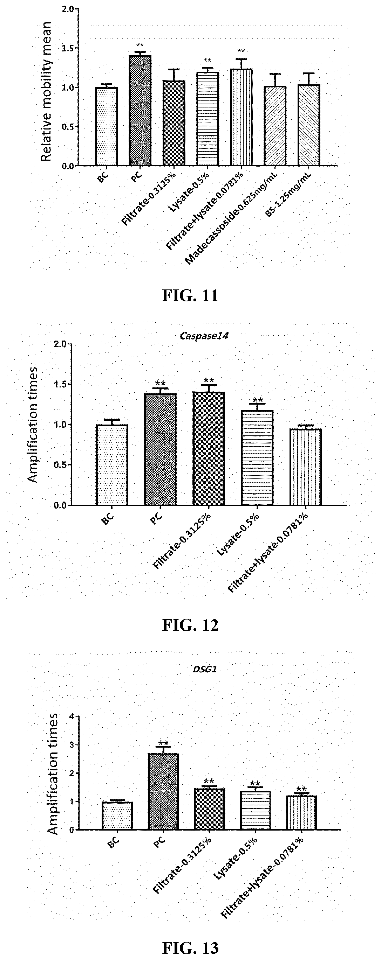

Experimental results are as shown in TABLE 10, FIG. 10 and FIG. 11. The lysate and the filtrate+lysate can inhibit cell migration, with significant difference compared with the BC group.

(2). Amount of gene expression

1). Testing method

Cell inoculation: After resuscitation of the cells, when the plating rate reached about 60%, the cells were inoculated in the 6-well plates, and incubated overnight in the $CO_2$ incubator (37 °C, 5% $CO_2$).

Dosing: According to test groups in TABLE 11 to TABLE 17, when the plating rate of the cells in the 6-well plates reached about 40%-60%, dosing was performed for the groups respectively, with each group in triplicate. The blank control group was added with 2 mL of the culture solution per well, the positive control group was added with 2 mL of a VE-containing culture solution per well, and the sample group was added with 2 mL of the test sample-containing culture solution at a corresponding concentration per well. After the dosing was completed, the 6-well plates were cultured in the $CO_2$ incubator (37 °C, 5% $CO_2$) for 24 h.

Cell collection: After 24 h of culture, an original solution was sucked out and discarded, PBS cleaning was performed twice, and each well was added with 1 mL of RNAiso Plus, to pipet and lyse the cells, after which samples were collected.

Detection of gene expression: RNA was extracted and subjected to reverse transcription to cDNA to undergo fluorescence quantitative PCR detection, and result was calculated by a $2^{-\Delta\Delta CT}$ method.

Calculation of up-regulation rate: up-regulation rate = (sample group - blank control group) / blank control group $\times$ 100%

Statistical analysis of results: GraphPad Prism was applied for plotting, and results were expressed as Mean $\pm$SD. Comparison between the groups was statistically analyzed using t-test. Statistical analysis was two-tailed. P<0.05 is considered to have a significant difference, and P<0.01 is considered to have an extremely significant difference.

In the above, in the filtrate+lysate, the filtrate accounts for 50%, and the lysate accounts for 50%.

2). Test results

① *Caspase 14* PCR

*Caspase14,* cysteine protease 14, is a member of cysteine protease family and has a main effect of lysing Filaggrin to form natural moisturizing factors PCA and UCA, thus playing an important role in the direction of moisturizing function.

*Caspase 14* primer: F: 5'-GCGAGCTAAGCCCAAGGTGTA-3' (as set forth in SEQ ID NO: 12); and R: 5'-GATGACCATCACAATCTCATCTCCA-3' (as set forth in SEQ ID NO: 13).

TABLE 11

| Group | Mean | SD | P-value | Increase Rate (vs BC) |
|---|---|---|---|---|
| BC | 1,00 | 0.06 | / | / |
| PC:0.05%VE | 1.39 | 0.06 | 0.002** | / |
| Filtrate-0.3125% | 1.41 | 0.08 | 0.002** | 41.00% |
| Lysate-0.5% | 1.18 | 0.08 | 0.038** | 18.00% |
| Filtrate+lysate-0.0781% | 0.95 | 0.04 | 0.349 | / |

Experimental results are as shown in TABLE 11 and FIG. 12. The filtrate and the lysate can improve the expression of *Caspase14*, with significant difference.

(2) *DSG1* PCR

*DSG1*, desmoglein 1, is a main constituent protein of desmosome. Desmosome junction is a common cell junction structure located in a deep part of intermediate junction. The desmosome has an effect of maintaining intercellular junction. Once the desmosome is damaged, and keratinocyte is caused to loosen.

*DSG1* primer: F: 5'-TCCTCATCATGGGATTCTTGGTC-3' (as set forth in SEQ ID NO: 14); and R: 5'-GAACATTCGGGAACAGGCTCA-3' (as set forth in SEQ ID NO: 15).

TABLE 12

| Group | Mean | SD | *P*-value | Increase Rate (vs BC) |
|---|---|---|---|---|
| BC | 1.00 | 0.05 | / | / |
| PC:0.05%VE | 2.71 | 0.22 | 0.000** | / |
| Filtrate-0.3125% | 1.46 | 0.08 | 0.001** | 46.00% |
| Lysate-0.5% | 1.38 | 0.13 | 0.009** | 38.00% |
| Filtrate+lysate-0.0781% | 1.21 | 0.09 | 0.025** | 21.00% |

Experimental results are as shown in TABLE 12 and FIG. 13. The filtrate, the lysate and the filtrate+lysate can all improve the expression of *DSG1*, with significant difference.

(3) *Claudin-4* PCR

Tight junctions (TJ) are cell-cell junctions which connect neighboring cells, control a paracellular pathway of molecules (barrier function) and separate an apical part from a basolateral part of a cell membrane (fence function). In human epidermis, various TJ proteins have been identified, including occludin, claudin1,4,7, JAM-1, etc. During epidermal regeneration, synthesis of the TJ protein precedes formation of SC. Thus, when SC barrier is disturbed, challenged, or missing, TJ may be a rescue system.

*Claudin-4* primer: F: 5'-TCTCCTCTGTTCCGGGTAGG-3' (as set forth in SEQ ID NO: 16); and R: 5'-CGTCCATCCACTCTGCACTT-3' (as set forth in SEQ ID NO: 17).

TABLE 13

| Group | Mean | SD | *P*-value | Increase Rate (vs BC) |
|---|---|---|---|---|
| BC | 1.00 | 0.07 | / | / |
| PC | 3.17 | 0.16 | 0.000** | / |
| Filtrate-0.3125% | 1.22 | 0.04 | 0.008** | 22.00% |
| Lysate-0.5% | 1.79 | 0.08 | 0.000** | 79.00% |
| Filtrate+lysate-0.0781% | 0.97 | 0.02 | 0.418 | / |

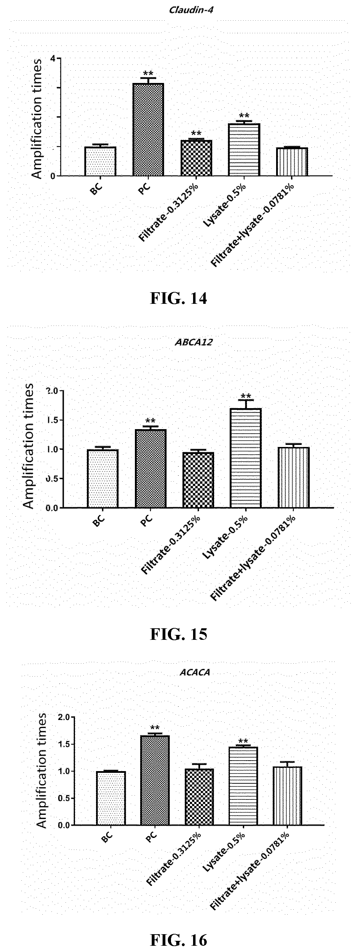

Experimental results are as shown in TABLE 13 and FIG. 14. The filtrate and the lysate can improve the expression of *Claudin-4,* with significant difference.

④ *ABCA12* PCR

*ABCA12*, adenosine triphosphate-binding cassette transporter A12 (*ABCA12*), is an ABC family lipid transporter located at a lamellar body limiting membrane, participates in formation of lamellar body, and transfers glucosylceramide to the lamellar body so that it is converted into ceramide under catalytic action of β-glucocerebrosidase, and subsequently ceramide is transported to surfaces of keratinocytes. *ABCA12* functions to facilitate transmembrane transport throughout the process and has an important effect in maintaining normal skin barrier functions. A decreased content of *ABCA12* affects transport of liposomes, and further affects the barrier functions.

*ABCA12* primer: F: 5'-GCTGGAAACCAACAAGACTGCTTTA-3' (as set forth in SEQ ID NO: 18); and R: 5'-TATAGTGGAACCTTGGCTGCTGGTA-3' (as set forth in SEQ ID NO: 19).

TABLE 14

| Group | Mean | SD | *P*-value | Increase Rate (vs BC) |
|---|---|---|---|---|
| BC | 1.00 | 0.04 | / | / |
| PC: 0.05%VE | 1.34 | 0.05 | 0.001** | / |
| Filtrate-0.3125% | 0.95 | 0.04 | 0.164 | / |
| Lysate-0.5% | 1.70 | 0.14 | 0.001** | 70.00% |
| Filtrate+lysate-0.0781% | 1.04 | 0.05 | 0.389 | 4.00% |

Experimental results are as shown in TABLE 14 and FIG. 15. The lysate can improve the expression of *ABCA12*, with significant difference.

⑤ *ACACA* PCR

*ACACA,* acetyl-CoA carboxylase alpha (*ACACA*), is a key rate-limiting enzyme for synthesis of fatty acids. Therefore, change in *ACACA* content is related to the synthesis of fatty acids, further affecting the skin barrier functions.

ACACA primer: F: 5'-AGTGAGGATGGCAGCTCTGGA-3' (as set forth in SEQ ID NO: 20); and R: 5'-ATGAGATGTGGGCAGCATGAAC-3' (as set forth in SEQ ID NO: 21).

TABLE 15

| Group | Mean | SD | *P*-value | Increase Rate (vs BC) |
|---|---|---|---|---|
| BC | 1.00 | 0.01 | / | / |
| PC: 0.05%VE | 1.66 | 0.04 | 0.000** | / |
| Filtrate-0.3125% | 1.05 | 0.08 | 0.349 | 5.00% |
| Lysate-0.5% | 1.45 | 0.03 | 0.000** | 45.00% |
| Filtrate+lysate-0.0781% | 1.09 | 0.08 | 0.135 | 9.00% |

Experimental results are as shown in TABLE 15 and FIG. 16. The lysate can improve the expression of *ACACA,* with significant difference.

⑥ *HMGCR* PCR

*HMGCR,* HMG-CoA reductase, is a rate-limiting enzyme for synthesis of cholesterols. Therefore, change in *HMGCR* content is related to the synthesis of cholesterols, further affecting the skin barrier functions.

*HMGCR* primer: F: 5'-GCCTGGCTCGAAACATCTGAA-3' (as set forth in SEQ ID NO: 22); and R: 5'-CTGACCTGGACTGGAAACGGATA-3' (as set forth in SEQ ID NO: 23).

TABLE 16

| Group | Mean | SD | *P*-value | Increase Rate (vs BC) |
|---|---|---|---|---|
| BC | 1.00 | 0.06 | / | / |
| PC | 2.91 | 0.28 | 0,000** | / |
| Filtrate-0.3125% | 2.71 | 0.24 | 0.000** | 171.00% |
| Lysate-0.5% | 0.97 | 0.05 | 0.529 | / |

(continued)

| Group | Mean | SD | *P*-value | Increase Rate (vs BC) |
|---|---|---|---|---|
| Filtrate+lysate-0.0781% | 0.95 | 0.14 | 0.582 | / |

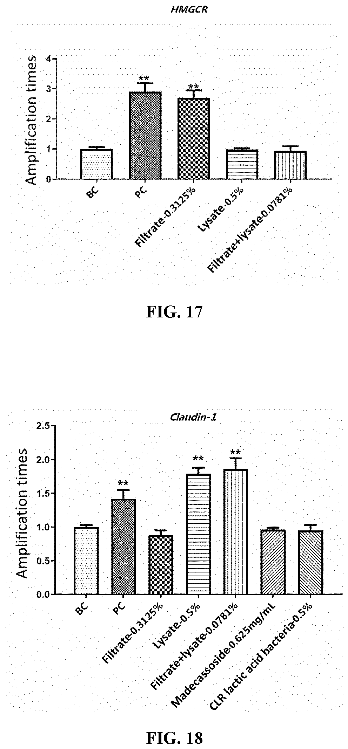

Experimental results are as shown in TABLE 16 and FIG. 17. The filtrate can improve the expression of *HMGCR,* with significant difference.

⑦ *Claudin-1* PCR

Tight junctions (TJ) are cell-cell junctions which connect neighboring cells, control a paracellular pathway of molecules (barrier function) and separate an apical part from a basolateral part of a cell membrane (fence function). In human epidermis, various TJ proteins have been identified, including occludin, claudin1,4,7, JAM-1, etc. During epidermal regeneration, synthesis of the TJ protein precedes formation of SC. Thus, when SC barrier is disturbed, challenged, or missing, TJ may be a rescue system. The effect of *Claudin-1* in the TJ protein is more critical, and it has been reported that *Claudin-1*-deficient mice die within 1 day after birth due to a large amount of water loss.
*Claudin-1* primer: F: 5'-GCATGAAGTGTATGAAGTGCTTGGA-3' (as set forth in SEQ ID NO: 24); and R: 5'-CCATACCATGCTGTGGCAACTAA-3' (as set forth in SEQ ID NO: 25).

TABLE 17

| Group | Mean | SD | *P*-value | Increase Rate (vs BC) |
|---|---|---|---|---|
| BC | 1.00 | 0.03 | / | / |
| PC: 0.05%VE | 1.42 | 0.13 | 0.006** | 42% |
| Filtrate-0.3125% | 0.88 | 0.07 | 0.057 | / |
| Lysate-0.5% | 1.79 | 0.09 | 0.000** | 79.00% |
| Filtrate+lysate -0.0781% | 1.86 | 0.16 | 0.001** | 86.00% |
| Madecassoside -0.625mg/mL | 0.96 | 0.03 | 0.181 | / |
| CLR lactic acid bacteria-0.5% | 0.95 | 0.08 | 0.417 | / |

Experimental results are as shown in TABLE 17 and FIG. 18. The lysate and the filtrate+lysate can improve the expression of *Claudin-1,* with significant difference.

(3) Test based on SLS-stimulated 3D epidermal skin model (EpiKutis®)

TABLE 18

| Group | Sample Name | Dosing Concentration | Induction Condition | Detection Model | Detection Index | Detection Method |
|---|---|---|---|---|---|---|
| Blank control (BC) | Culture medium | / | / | | | |
| Negative control (NC) | Culture medium | / | | | | |
| Positive control (PC) | WY14643 | 50μM | | EpiKuits® | Tissue morphology | H&E |
| Sample Group | Filtrate | 5% | SLS (0.1%) | | | |
| | Lysate | 5% | | | | |
| | Filtrate+lysate | 5% | | | | |
| | Madecassoside | 0.625mg/mL | | | | |

In the above, in the filtrate+lysate, the filtrate accounts for 50%, and the lysate accounts for 50%.

Tissue morphology: Tissue morphology is microscopic physiological structure analysis of tissues after HE staining. Through the tissue morphology of the model, changes in the skin barrier under different treatment conditions can be seen, for example, after SLS damage, the skin barrier becomes loose, thickness of a living cell layer decreases, and such damage can be ameliorated after treatment with active substances.

1). Testing method

Formulation of working solution:

1) Formulation of 0.1% SLS working solution: 0.006 g of SLS was weighed and dissolved in 6 mL of a PBS solution, filtered by 0.22 $\mu$m, and formulated into the 0.1% SLS working solution for later use.

2) Formulation of working solution of positive control group (50 $\mu$M WY14643): 10 $\mu$L of 30 mM WY14643 mother liquor was sucked and dissolved in 6 mL of the culture solution to be formulated into 50 $\mu$M WY14643.

Model dosing:

<1> According to test groups in TABLE 18, models were transferred into a 6-well plate (added with 0.9 mL of an EpiGrowth culture solution in advance), and numbers of test groups were marked on the 6-well plate.

<2> To surfaces of NC group, PC group and sample group, 25 $\mu$L of the 0.1% SLS solution was added and incubated for 30 min.

<3> After incubation was finished, the PC group was added with the working solution at a corresponding concentration below a liquid surface, and the sample working solution was evenly distributed on the surface of the model in the sample group, followed by incubation in the $CO_2$ incubator (37 °C, 5% $CO_2$) for 24 h.

<4> After the incubation was finished, test substances remaining on surfaces of the models were cleaned with a sterile PBS solution, and residual liquid inside and outside the models was wiped off with a sterile cotton swab.

Test of tissue morphology:

The models for detection of tissue morphology were fixed using 4% paraformaldehyde. After 24 h of fixation, H&E staining detection was performed, photographs were taken under a microscope and observed, and pictures were collected and analyzed.

Statistical analysis of results:

Statistical analysis of results: GraphPad Prism was applied for plotting, and results were expressed as Mean $\pm$SD. Comparison between the groups was statistically analyzed using t-test. Statistical analysis was two-tailed. $P<0.05$ is considered to have a significant difference, and $P<0.01$ is considered to have an extremely significant difference.

2). Test results

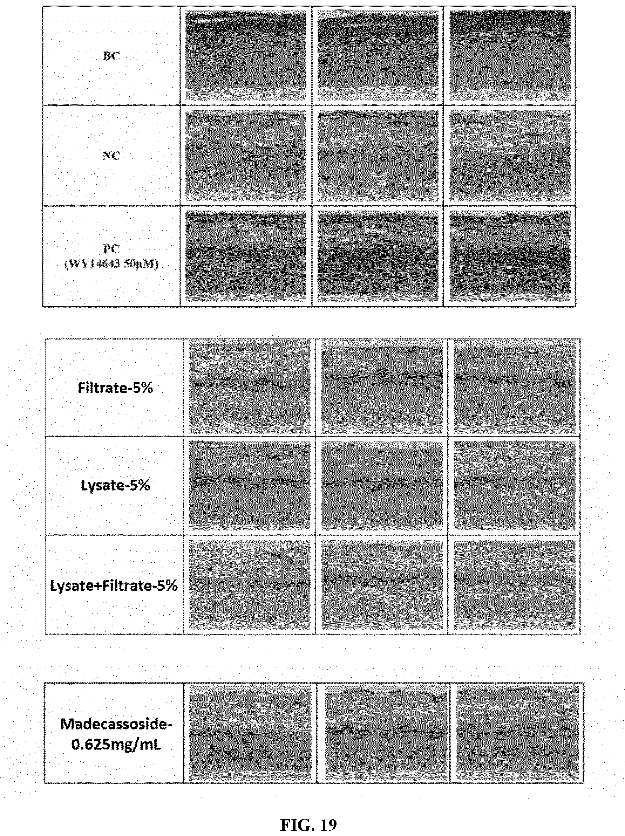

As shown in FIG. 19, the results of detection of tissue morphology show that compared with the BC group, a living cell layer of the epidermoid model of the NC group is damaged, vacuoles appear, and stratum corneum is loose, indicating that the SLS stimulation condition is effective.

Compared with the NC group, cells in the living cell layer are in compact arrangement in the PC group, vacuoles are reduced, and the phenomenon of living cell damage is obviously ameliorated, indicating that the present positive control detection is effective.

Compared with the NC group, the vacuoles in the samples, filtrate-5%, lysate-5%, combination-5% and madecassoside-0.625 mg/mL, are reduced, and the phenomenon of living cell damage is obviously ameliorated, indicating that the samples have a repairing effect on the tissue morphology of the models.

**Verification Example 4 Soothing**

(1) Keratinocyte-based inflammatory factor test

TABLE 19

| Group | Sample Name | Dosing Concentration | Stimulation Condition | Detection Model | Detection Item | Detection Method |
|---|---|---|---|---|---|---|
| Blank control (BC) | / | / | / | | | |
| Negative control (NC) | / | | | | | |
| Positive control (PC) | Dexamethasone | 0.01% | | Keratinocyte | IL-1$\alpha$, IL-6, PGE2 | ELISA |
| **Sample group** | Filtrate | 0.3125% | ( 300mJ/cm$^2$ ) | | | |
| | Lysate | 1% | | | | |
| | Filtrate+lysate | 0.0781% | | | | |
| | Madecassoside | 0.625mg/mL | | | | |

In the above, in the filtrate+lysate, the filtrate accounts for 50%, and the lysate accounts for 50%.

1) Testing method

Cell inoculation: After resuscitation of cells, when a plating rate reached about 60%, the cells were inoculated in 6-well plates, and incubated overnight in a $CO_2$ incubator (37 °C, 5% $CO_2$).

Dosing: According to test groups in TABLE 19, when the plating rate of the cells in the 6-well plates reached about 30%-50%, dosing was performed for the groups respectively, with each group in triplicate. The blank control group and the negative control group were added with 2 mL of a culture solution per well, the positive control group was added with 2 mL of a dexamethasone-containing culture solution per well, and the sample group was added with 2 mL of a test sample-containing culture solution at corresponding concentration per well. After the dosing was completed, the 6-well plates were cultured in the $CO_2$ incubator (37 °C, 5% $CO_2$) for 24 h.

UVB irradiation: after the cells are cleaned in PBS, according to the test groups, remaining groups other than the blank control group were all subjected to UVB irradiation, with an irradiation dosage of 300 mJ/cm$^2$.

Posterior incubation: After the cells were cleaned in the PBS, the 6-well plates were placed in the $CO_2$ incubator (37 °C, 5% $CO_2$) to undergo posterior incubation for 24 h.

Cell collection: Cell culture supernatant was collected in a centrifuge tube, and cryopreserved in a -80 °C freezer.

ELISA detection: detection and analysis were performed according to operation instructions of an ELISA detection kit.

Calculation of inhibition rate: inhibition rate = (negative control group - sample group)/ negative control group $\times$ 100%

Statistical analysis of results: GraphPad Prism was applied for plotting, and results were expressed as Mean $\pm$SD. Comparison between the groups was statistically analyzed using t-test. Statistical analysis was two-tailed. P<0.05 is considered to have a significant difference, and P<0.01 is considered to have an extremely significant difference.

2) Test Results

PGE2: PGE2 prostaglandin is an arachidonic acid metabolic product, and has a main effect of inducing an inflammatory response. PGE2 content is reduced after action of a test substance, and a certain soothing efficacy can be achieved.

TABLE 20

| Group | Mean Concentration (pg/mL) | SD | P-value | Inhibition Rate (vs NG) |
|---|---|---|---|---|
| BC | 165.31 | 13.37 | / | / |
| NC | 492.42 | 30.26 | 0.000 ** | / |
| PC: dexamethasone-0.01% | 159.23 | 14.25 | 0.000 ** | / |
| Filtrate -0.3125% | 229.77 | 10.14 | 0.000 ** | 53.34% |
| Lysate-1% | 192.41 | 2.50 | 0.000 ** | 60.93% |
| Filtrate+lysate-0.0781% | 185.08 | 2.39 | 0.000 ** | 62.41% |
| *Centella asiatica*-0.625mg/mL | 149.76 | 6.13 | 0.000 ** | 69.59% |

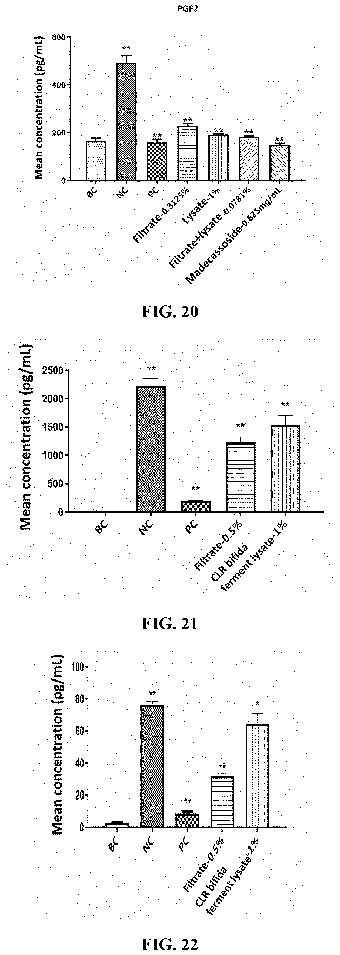

Experimental results are as shown in TABLE 20 and FIG. 20. The filtrate, the lysate and the filtrate+lysate can all inhibit the content of PGE2.

(2). Macrophage-based inflammatory factor test

TABLE 21

| Group | Sample Name | Dosing Concentration | Stimulation Condition | Detection D Model | etection Index | Detection Method |
|---|---|---|---|---|---|---|
| Blank control (BC) | / | / | / | | IL-6 | |
| Negative control (NC) | / | / | LPS (1$\mu$g/mL) | Macrophage | IL-1$\beta$ | ELISA |
| Positive control (PC) | Dexamethasone | 0.01% | | | | |

1) Testing method

Formulation of working solution:

<1> Formulation of working solution of positive control group (dexamethason): 2 $\mu$L of 10% mother liquor was sucked and dissolved in 2 mL of the culture solution to formulate 0.01% dexamethasone.
<2> Formulation of 1 $\mu$g/mL LPS-containing working solution: 2 mg/mL LPS stock solution was diluted to 1 $\mu$g/mL with the test-substance working solution remaining, negative control working solution and positive control working solution, respectively, after the dosing.

Operation steps:

<1> Cell inoculation: the cells were inoculated in 6-well plates at an inoculation density of $2.2 \times 10^5$ per well, 2 mL of a cell suspension was added to each well, the inoculated cell culture plates continued to be cultured in the incubator for 24 h (5% $CO_2$, 37 °C).
<2> Original cell culture solution in the wells was sucked out and discarded. The sample group was added with 1.8 mL of the test substance-containing culture solution at a corresponding concentration per well; the negative control group was added with 1.8 mL of a solvent control culture solution per well; the positive control group was added with 1.8 mL of a positive control culture solution per well; and the blank control group was added with 1.8 mL of a normal culture solution per well.
<3> After the dosing was completed, the 6-well plates were cultured in a cell incubator (5% $CO_2$, 37 °C) for 2 h.

<4> LPS stimulation: After 2 h of dosing, each group was added with 200 μL of the formulated LPS-containing working solution per well, and continued to be cultured in the cell incubator (5% $CO_2$, 37 °C) for 22 h.

<5> ELISA detection: cell culture supernatant was collected, and ELISA detection was performed according to instructions of the ELISA kit.

<6> Processing and analysis of data: GraphPad Prism was applied for plotting, and results were expressed as Mean ±SD. Comparison between the groups was statistically analyzed using t-test. Statistical analysis was two-tailed. $P < 0.05$ is considered to have a significant difference, and $P < 0.01$ is considered to have an extremely significant difference.

2) Test results

① IL-6

IL-6 is a pro-inflammatory factor and is a polypeptide containing a hydrophobic terminus, and thus can be secreted out of a cell after activation of inflammatory-related pathways. Reduced content of inflammatory factors after action of the test substance indicates having a soothing efficacy.

TABLE 22

| Group | Mean Concentration (pg/mL) | SD | P-value | Inhibition Rate |
|---|---|---|---|---|
| BC | 0.50 | 0.25 | / | / |
| NC | 2224.08 | 131.21 | 0.000 ** | / |
| PC: 0.01% dexamethasone | 193.04 | 6.23 | 0.000 ** | / |
| Filtrate-0.5% | 1223.63 | 101.32 | 0.000 ** | 44.98% |
| CLR bifida ferment lysate-1% | 1538.63 | 166.66 | 0.005 ** | 30.82% |

Experimental results are as shown in TABLE 22 and FIG. 21. The filtrate has a high inhibition rate on IL-6, even higher than that of CLR bifida ferment lysate.

② IL-1β

IL-1β, as a key pro-inflammatory cytokine, is involved in a variety of autoimmune inflammatory responses and a variety of cellular activities, including cell proliferation, differentiation and apoptosis. IL-1β is considered as a typical multifunctional cytokine and affects almost all types of cells, whether acting alone or in combination with other cytokines. IL-1β is crucial for cellular defense and tissue repair of almost all tissues, and is associated with pain, inflammation and autoimmunity. IL-1β is also involved in neuroprotection, tissue remodeling and repair.

TABLE 23

| Group | Mean Concentration (pg/mL) | SD | P-value | Inhibition Rate |
|---|---|---|---|---|
| BC | 2.83 | 0.48 | / | / |
| NC | 76.40 | 1.82 | 0.000 ** | / |
| PC: 0.01% dexamethasone | 8.51 | 1.41 | 0.000 ** | / |
| Filtrate-0.5% | 31.99 | 1.81 | 0.000 ** | 58.13% |
| CLR bifida ferment lysate-1% | 64.49 | 6.20 | 0.033 * | 15.60% |

Experimental results are as shown in TABLE 23 and FIG. 22. The filtrate has a high inhibition rate on IL-1β, even higher than that of CLR bifida ferment lysate.

**Verification Example 5 Exfoliation**

1) Testing method

Formulation of working solution:

<1> Formulation of cleaning solution (0.1% Triton X-100): 0.1 mL of Triton X-100 was weighed and dissolved in 100 mL of PBS to formulate 0.1% Triton X-100 solution.

<2> Formulation of sodium hydroxide solution (12 M): 4.8 g of sodium hydroxide solid was weighed, and added with deionized water to make up to 10 mL, and formulate 12 M sodium hydroxide solution.

Dosing:

<1> After being thawed and cleaned, pig skin was fixed between a supply chamber and a receiving chamber of Franz cell, with stratum corneum of the skin facing the supply chamber, and a dermal side facing the receiving chamber. Into the receiving chamber, 7.0 mL of a receiving liquid was added. After suckling pig skin was stretched and fixed well, into the receiving chamber, 1.5 mL of the receiving liquid (PBS) was added through a sample collector, and air was exhausted, so as to make the dermis of the skin be in close contact with the receiving liquid.

<2> Samples (as shown in TABLE 24) were added to the skin surface in the supply chamber, where 50 $\mu$L of the samples were added to the surface of the pig skin (a PBS buffer solution was added to the surface of the control group as blank control). The samples were evenly spread radially from a central part of the skin towards an edge, and an exposed area of the samples was 3.14 cm$^2$. Each sample had three parallel replicates. Constant-temperature water bath of (32$\pm$1) °C was maintained, and a water bath sandwich was ensured to be bubble free.

<3> After 24 h of incubation, areas where the samples were used were rubbed with a finger cot, and after two minutes of rubbing, 0.5 mL of a cleaning solution (0.1% Triton X-100) was added into the supply chamber to pipet and clean keratinocytes detached from the skin surface.

<4> The cleaning solution was transferred into a 1.5 mL centrifuge tube for later use.

Microscopic photographing:

The 1.5 mL centrifuge tube loaded with the cleaning solution was placed on a mini-mixer (MIX-2500), and shaken for 2 minutes at a maximum rotational speed. Then 10 $\mu$L of the cleaning solution was spotted on a cell counting plate. The cell counting plate was placed under an inverted microscope, and photographed at 10$\times$ magnification and observed.

Detection of total protein content:

<1> The collected cleaning solution was put into a high-speed centrifuge, and centrifuged for 10 min at a rotational speed of 15000 rpm. Keratinocytes in the cleaning solution were precipitated, supernatant was discarded, and precipitate was re-suspended with 0.5 mL of deionized water.

<2> Into the re-suspended liquid, 25 $\mu$L of a sodium hydroxide (12 M) solution was added, the keratinocytes were lysed for 30 min in a boiling water bath, and 25 $\mu$L of concentrated hydrochloric acid (12 M) was added to neutralize lysis solution.

<3> The total protein content was detected according to instructions of a BCA protein assay kit.

Result determination:

Eluted keratinocytes were lysed, to detect the protein content. The total protein content in the lysis solution was directly proportional to the quantity of detached keratinocytes. A higher total protein content indicates more keratinocytes eluted, indicating that the sample has an exfoliation efficacy.

Statistical analysis of results:

GraphPad Prism was applied for plotting, and results were expressed as Mean$\pm$SD. Comparison between the groups was statistically analyzed using t-test. Statistical analysis was two-tailed. $P<0.05$ is considered to have a significant difference, and $P<0.01$ is considered to have an extremely significant difference.

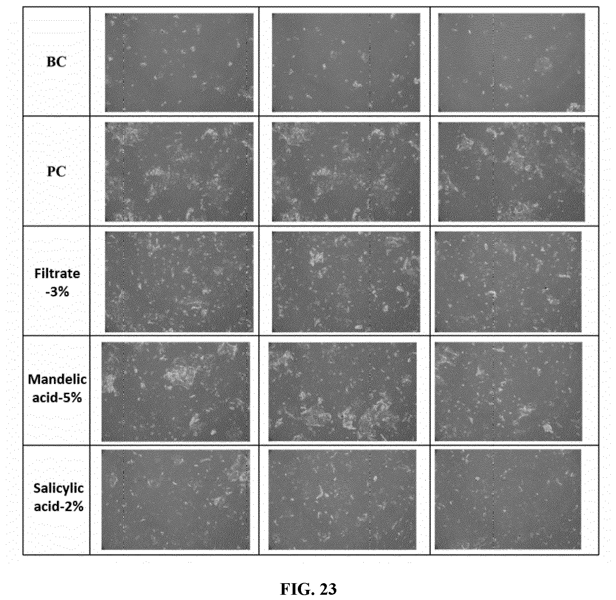

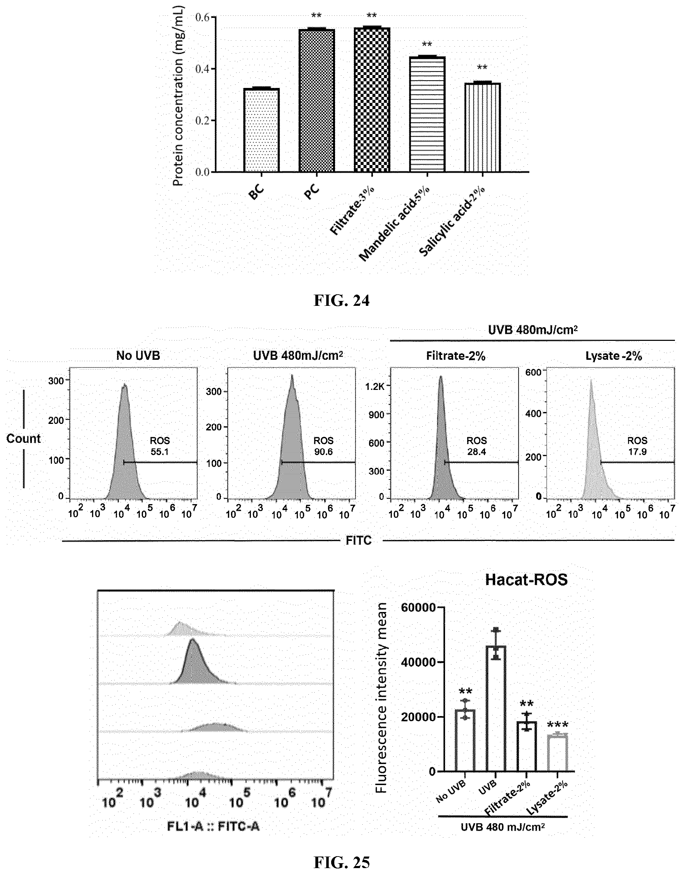

Experimental results are as shown in TABLE 24, FIG. 23 and FIG. 24. Detecting a mean of the total protein concentration using an excised skin model finds that 3% filtrate has a stronger effect of keratin exfoliation than 5% mandelic acid or 2% salicylic acid, even stronger than keratin exfoliation effect of positive control 10% salicylic acid.

TABLE 24

| Sample Name | Protein Concentration Mean (mg/mL) | SD | *P*-value |
|---|---|---|---|
| BC (blank control) | 0.326 | 0.002 | |
| PC (10% salicylic acid) | 0.555 | 0.002 | 0.000** |
| Filtrate-3% | 0.561 | 0.002 | 0.000** |
| Mandelic acid-5% | 0.448 | 0.002 | 0.000** |
| Salicylic acid-2% | 0.347 | 0.003 | 0.001** |

**Verification Example 6 Anti-oxidation**

(1). Test of ROS clearance (UVB induction)

<1> Human immortalized keratinocytes Hacat in a good growth state were inoculated in a small dish at $10^4$ cells per well.

<2> The cells were cultured in a 37 °C 5% $CO_2$ cell incubator for 24 h.

<3> After the cells in the small dish adhered to the wall, test samples of 2% fermentation concentrate obtained in Example 2 and 2% lysate product obtained in Example 3 were added for treatment for 24 h.

Supernatant was carefully discarded, and PBS cleaning was performed once. 400 μL of PBS was added, and UVB radiation was performed, with a radiation dosage of 480 mJ/$cm^2$. Supernatant was discarded after the radiation.

<5> Each dish was added with 2 mL of a DMEM medium containing 10% FBS, and cultured in the incubator for 24 h.

<6> Cells were digested with pancreatin and collected, and cleaned in PBS twice.

<7> DCFH-DA was diluted with a serum-free culture solution according to 1:1000 to a final concentration of 10 μmol/L.

<8> Cells were collected and then suspended in the diluted DCFH-DA, where a cell concentration was one million to 20 million per milliliter.

<9> Incubation was performed in the 37 °C cell culture incubator for 20 minutes. The cells were mixed evenly upside down every 3~5 min, allowing probe to be in full contact with the cells.

<10> The cells were cleaned with the serum-free cell culture solution for 3 times, so as to fully remove DCFH-DA that failed to enter the cells.

<11> Streaming computation was carried out to detect intensity of fluorescence before and after stimulation in real time or time point by time point by using 488 nm excitation-wave wavelength and 525 nm emission wavelength. Fluorescence spectrum of DCF is quite similar to that of FITC, and DCF can be set using parameters of FITC (FITC channel: excitation wave of 488 nm, and emission wave of 525 nm).

<12> Streaming results were processed using software Flow Jo.

Experimental results are as shown in FIG. 25. The fermentation filtrate and the lysate product can significantly inhibit ROS oxidative injury caused by UVB to the keratinocytes.

Sample name is HACAT lysate product, and the quantity of lymphocytes is 13859; sample name is HACAT filtrate, and the quantity of lymphocytes is 44358; sample name is HACAT UVB, and the quantity of lymphocytes is 13580; and sample name NO UVB, and the quantity of lymphocytes is 10352.

Meanwhile, ROS peak of UVB modeling group is significantly shifted right compared with UVB group not radiated, indicating that modeling is successful, and the fermentation filtrate and the lysate product both have an anti-ROS production effect.

(2). Experiment of clearance of DPPH free radicals

Formulation of solution:

95% ethanol: 95 mL of ethanol was put into a measuring cylinder, into which ultrapure water was added to make up to 100 mL and render 95% ethanol.

0.03% DPPH stock solution: 15 mg of DPPH powder was weighed into a 50 mL brown volumetric flask, into which about 40 mL of 95% ethanol was added, completely dissolved by means of ultrasound, made up to a scale mark, and shaken up to serve as the DPPH stock solution.

0.03‰ DPPH working solution: according to a required DPPH solution amount, 1/10 volume of the 0.03% DPPH stock solution was weighed and diluted to render the 0.03‰ DPPH working solution.

Formulation of sample and control competitor:

TABLE 25 Table of Formulation Method of Controls

| Competitor and Positive Control | Concentration | Formulation Method |
|---|---|---|
| CLR bifida ferment lysate product | 3% | Take 30 $\mu$L of CLR bifida ferment lysate product, add 970 $\mu$L of ulrtapure water, and mix well |
| EGCG | 0.005% | Take 50 $\mu$L of EGCG, add 950 $\mu$L of ultrapure water, mix well to formulate 5% solution, gradiently dilute to 0.005% using ultrapure water |

Chromogenic reaction:

Negative control tube: 200 $\mu$L of the 95% ethanol solution was added into 5 mL of the 0.03‰ DPPH solution, mixture was shaken up, and left to stand for 30 min in a light-tight manner to serve as negative control. Three parallel samples were made. Absorbance was measured, and mean was calculated as A.

Blank sample tube: 200 $\mu$L of the 95% ethanol solution was added into 5 mL of the 95% ethanol solution, mixture was shaken up and left to stand for 30 min in a light-tight manner to serve as negative control. One parallel sample was made. Absorbance was measured as A blank.

Sample control tube: 200 $\mu$L of the sample was added into 5 mL of 95% ethanol, mixture was shaken up and left to stand for 30 min in a light-tight manner to serve as sample control. One parallel sample was made. Absorbance was measured as A sample control.

Sample reaction tube: 200 $\mu$L of the sample solution was added into 5 mL of the 0.03‰ DPPH solution, mixture was shaken up and left to stand for 30 min in a light-tight manner. Three parallel samples were made. Absorbance was measured, and mean was calculated as A sample.

Measurement of absorbance:

A detection wavelength was set to be 517 nm, the 95% ethanol solution was taken as blank correction solution, and the above negative control solution, blank control solution, sample control solution and sample solution were taken to measure OD570 nm.

Data processing:

Clearance rate of DPPH free radicals (%) = (1 - (A sample - A sample control)/(A - A blank))* 100%.

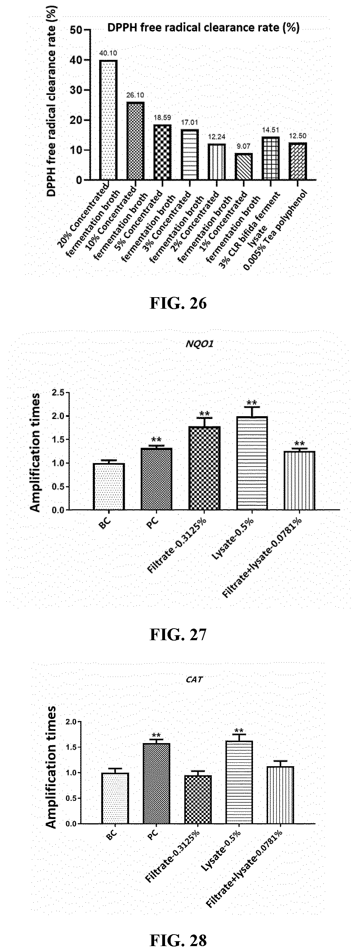

Experimental results are as shown in FIG. 26. The fermentation filtrate can significantly remove the DPPH free radicals.

(3). Keratinocyte-based genetic test

1) Testing method

Cell inoculation: After resuscitation of cells, when a plating rate reached about 60%, the cells were inoculated in the 6-well plates, and incubated overnight in the $CO_2$ incubator (37 °C, 5% $CO_2$).

Dosing: According to test groups, when the plating rate of the cells in the 6-well plates reached about 40%-60%, dosing was performed for the groups respectively, with each group in triplicate. The blank control group was added with 2 mL of the culture solution per well, the positive control group was added with 2 mL of a VE-containing culture solution per well, and the sample group was added with 2 mL of a test sample-containing culture solution at corresponding concentration per well. After the dosing was completed, the 6-well plates were cultured in the $CO_2$ incubator (37 °C, 5% $CO_2$) for 24 h.

Cell collection: After 24 h of culture, an original solution was sucked out and discarded, PBS cleaning was performed twice, and each well was added with 1 mL of RNAiso Plus, to pipet and lyse the cells, after which samples were

EP 4 585 678 A1

collected.

Detection of gene expression: RNA was extracted and subjected to reverse transcription to cDNA to undergo fluorescence quantitative PCR detection, and result was calculated by a 2$^{-\Delta\Delta}$CT method.

Calculation of up-regulation rate: up-regulation rate = (sample group - blank control group)/blank control group $\times$ 100%

Statistical analysis of results: GraphPad Prism was applied for plotting, and results were expressed as Mean $\pm$SD. Comparison between the groups was statistically analyzed using t-test. Statistical analysis was two-tailed. *P*<0.05 is considered to have a significant difference, and *P*<0.01 is considered to have an extremely significant difference.
In the above, in the filtrate+lysate, the filtrate accounts for 50%, and the lysate accounts for 50%.

2) Test results

① *NQO1* PCR

*NQO1* primer: F: 5'-TTTGGCTAGGTATCATTCAACTCTC-3' (as set forth in SEQ ID NO: 26); and R: 5'-CCCTTAGGGCAGGTAGATTCAG-3' (as set forth in SEQ ID NO: 27).
NAD(P)H: quinone acceptor oxidoreductase 1 (*NQO1*) is a widely-distributed FAD-dependent flavoprotein that can promote obligatory electron reductions of quinones, quinoneimines, nitroaromatics and azo. These reductions depress quinone levels and thereby minimize opportunities for generation of reactive oxygen intermediates by redox cycling. *NQO1* is a highly-inducible enzyme that is regulated by the Keap1/Nrf2/ARE pathway. Evidence for the importance of the antioxidant functions of *NQO1* in combating oxidative stress is provided by demonstrations that induction of *NQO1* levels or their depletion (knockout or knockdown) are associated with decreased and increased susceptibilities to oxidative stress, respectively.

TABLE 26

| Group | Mean | SD | *P*-value | Increase Rate (vs BC) |
|---|---|---|---|---|
| BC | 1.00 | 0.06 | / | / |
| PC: 0.05%VE | 1.32 | 0.05 | 0.002** | 32.00% |
| Filtrate-0.3125% | 1.78 | 0.18 | 0.002** | 78.00% |
| Lysate-0.5% | 1.99 | 0.20 | 0.001** | 99.00% |
| Filtrate+lysate-0.0781% | 1.26 | 0.05 | 0.004** | 26.00% |

Experimental results are as shown in TABLE 26 and FIG. 27. The filtrate, the lysate and the filtrate+lysate can improve the expression of anti-oxidation related gene *NQO1*, with significant difference.

② *CATPCR*

*CAT* Catalase primer: F: 5'-ATAGCCTTCGACCCAAGCAA-3' (as set forth in SEQ ID NO: 28); and R: 5'-GAGCACGGTAGGGACAGTTCA-3' (as set forth in SEQ ID NO: 29).
Keap1-Nrf2-ARE signaling pathway mediates transcription of phase II detoxification enzymes and antioxidant genes, and is considered as the most important pathway of cell antioxidant mechanism. The key to the *Nrf2* signaling pathway-mediated protective cells against oxidative stress is how to make *Nrf2* rapidly translocate from Nrf2-Keap1 complex into nucleus, to cause a series of subsequent responses. Under oxidative stress, including UV irradiation, Nrf2 is transferred from cytoplasm into the nucleus after phosphorylation activation to plays a regulating and controlling role. The Nrf2-Keap1 pathway may trigger simultaneous expression of numerous oxidative protective enzymes and scavengers, thereby having an impact on ROS level. The gene and protein expression level of *NRF2* inhibit expression of Keap1, and promote expression of its downstream antioxidant genes such as *NQO1*, *HO-1, CAT, SOD2,* and *SOD1*, thereby improving antioxidant capacity. Catalase CAT is a common and highly active enzyme in organisms, and directly catalyzes decomposition of hydrogen peroxide into non-toxic water and carbon dioxide.

TABLE 27

| Group | Mean | SD | *P*-value | Increase Rate (vs BC) |
|---|---|---|---|---|
| BC | 1.00 | 0.08 | / | / |

(continued)

| Group | Mean | SD | P-value | Increase Rate (vs BC) |
|---|---|---|---|---|
| PC | 1.58 | 0.07 | 0.001** | / |
| Filtrate-0.3125% | 0.95 | 0.08 | 0.446 | / |
| Lysate-0.5% | 1.63 | 0.12 | 0.002** | 63.00% |
| Filtrate+lysate-0.0781% | 1.13 | 0.10 | 0.163 | 13.00% |

Experimental results are as shown in TABLE 27 and FIG. 28. The lysate and the filtrate+lysate can improve the expression of *CAT*, with significant difference.

**Verification Example 7 Whitening**

(1). Whitening with fermentation broth: experiment of tyrosinase inhibition (B16 F10)

Positive control kojic acid was diluted with PBS to a series of concentration gradients of 1 mg/mL, 0.2 mg/mL, 0.04 mg/mL, and 0.008 mg/mL to verify a testing system.

Test substance was diluted with PBS into samples at multiple concentrations. Sample tubes (T), sample background (T0), enzyme reaction tubes (C) and solvent background (C0) were arranged in a 96-well plate. Sample wells (T) of each sample at each test concentration needed to be in triplicate, and meanwhile the enzyme reaction tubes (C) also needed to be in triplicate.

Into the sample tubes (T) and the sample background (T0), 50 $\mu$L of a sample solution at the same concentration was added respectively, and into the enzyme reaction tubes (C) and the solvent background (C0), 50 $\mu$L of PBS was added respectively.

Into the sample tubes (T) and the enzyme reaction tubes (C), 25 $\mu$L of a tyrosinase solution was added respectively, the sample background (T0) and the solvent background (C0) were added with 25 $\mu$L of PBS instead, and the sample and tyrosinase were fully mixed and incubated at 37 °C for 10 min.

Into each tube, 100 $\mu$L of an L-DOPA solution was added in sequence. Reaction was carried out for 5 min, and OD475 was measured.

$$\text{Calculation of tyrosinase inhibition rate: inhibition rate (\%)} = (1-(T-T0) / (C-C0)) \times 100\%$$

In the above, in the formula, T is an absorbance value of the sample well, that is, the absorbance value of the solution after reaction of the sample with tyrosinase; T0 is an absorbance value of the sample background; C is a mean of three absorbance values of the enzyme reaction wells, that is, absorbance values of reaction between tyrosinase and dopa without adding the sample; and CO is an absorbance value of the solvent background.

Experimental results are as shown in FIG. 29. The fermentation filtrate can significantly inhibit activity of tyrosinase.

(2). PCR detection of whitening gene

1) Testing method

Cell inoculation: After resuscitation of cells, when the plating rate reached about 60%, the cells were inoculated in the 6-well plates, and incubated overnight in the $CO_2$ incubator (37 °C, 5% $CO_2$).

Dosing: According to test groups in TABLES 28-39, when the plating rate of the cells in the 6-well plates reached about 50%-60%, dosing was performed for the groups respectively, with each group in triplicate. The blank control group was added with 2 mL of a culture solution per well, the positive control group was added with 2 mL of a kojic acid-containing culture solution per well, and the sample group was added with 2 mL of a test sample-containing culture solution at a corresponding concentration per well. After the dosing was completed, the 6-well plates were cultured in the $CO_2$ incubator (37 °C, 5% $CO_2$) for 24 h.

Cell collection: After 24 h of culture, an original solution was sucked out and discarded, PBS cleaning was performed twice, and each well was added with 1 mL of RNAiso Plus, to pipet and lyse the cells, after which samples were collected.

Detection of gene expression: RNA was extracted and subjected to reverse transcription to cDNA to undergo fluorescence quantitative PCR detection, and result was calculated by a $2^{-\Delta\Delta CT}$ method.

Results were calculated.

Calculation of down-regulation rate: down-regulation rate = (blank control group - sample group)/blank control group × 100%

Statistical analysis of results: GraphPad Prism was applied for plotting, and results were expressed as Mean ±SD. Comparison between the groups was statistically analyzed using t-test. Statistical analysis was two-tailed. $P<0.05$ is considered to have a significant difference, and $P<0.01$ is considered to have an extremely significant difference.
In the above, in the filtrate+lysate, the filtrate accounts for 50%, and the lysate accounts for 50%.

2) Test Results

① *TYR* PCR

TYR primer: F: 5'-TGCGGTGGGAACAAGAAA-3' (as set forth in SEQ ID NO: 30); and
R: 5'-GACAATCTGCCAAGAGGAGAAGA-3' (as set forth in SEQ ID NO: 31).
It is related to melanocyte differentiation, and is a key gene for regulating and controlling generation of melanin in the skin. For example, adding a whitening component into whitening cosmetics can reduce an amount of TYR expression, thereby reducing melanin synthesis and achieving a whitening effect.

TABLE 28

| Group | Mean | SD | *P*-value | Down-regulation Rate |
|---|---|---|---|---|
| BC | 1.00 | 0.06 | / | / |
| PC | 0.66 | 0.03 | 0.001 ** | / |
| Filtrate-0.1563% | 0.94 | 0.04 | 0.188 | 6,00% |
| Lysate-0.5% | 0.71 | 0.05 | 0.003 ** | 29.00% |
| Filtrate+lysate-0.0781% | 1.00 | 0.11 | 0.956 | / |

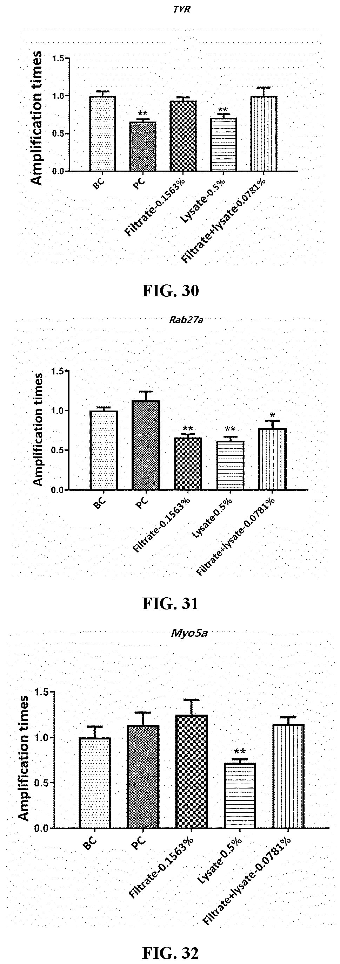

Experimental results are as shown in TABLE 28 and FIG. 30. The lysate can significantly down-regulate the expression of the melanin synthesis-related gene *TYR*.

② *Rab27a* PCR

*Rab27a* primer: F: 5'-GAATGGAACGGTGTGTGGAC-3' (as set forth in SEQ ID NO: 32); and
R: 5'-CCCCTTTCTCCTTTTCTTCACTT-3' (as set forth in SEQ ID NO: 33).
*MLPH,* small GTP binding protein (*Rab27a*) and myosin Va (*Myo5a*) form a ternary complex, which has a critical effect during transport of melanosomes, making the melanosomes aggregate at dendrite tips of melanocytes, and realizing transfer of the melanosomes from melanocytes to neighboring keratinocytes. Only when the melanosomes are transported from melanocytes to surrounding keratinocytes, can coloration of melanin on the skin be achieved.

TABLE 29

| Group | Mean | SD | *P*-value | Down-regulation Rate |
|---|---|---|---|---|
| BC | 1.00 | 0.04 | / | / |
| PC | 1.13 | 0.11 | 0.124 | / |
| Filtrate-0.1563% | 0.66 | 0.04 | 0.000 ** | 34.00% |
| Lysate-0.5% | 0.62 | 0.05 | 0.001 ** | 38.00% |
| Filtrate+lysate-0.0781 % | 0.78 | 0.09 | 0.018 * | 22.00% |

Experimental results are as shown in TABLE 29 and FIG. 31. The filtrate, the lysate and the filtrate+lysate all significantly down-regulate the expression of the melanosome transport-related gene *Rab27a*.

③ *Myo5a* PCR

*Myo5a* primer: F: 5'- GTGAGCGAGGAGCTTGATGT-3' (as set forth in SEQ ID NO: 34); and
R: 5'- TCATCCTTGGGTTGGATGGC-3' (as set forth in SEQ ID NO: 35).

*MLPH,* small GTP binding protein (*Rab27a*) and myosin Va (*Myo5a*) form a ternary complex, which has a critical effect during transport of melanosomes, making the melanosomes aggregate at dendrite tips of melanocytes, and realizing transfer of the melanosomes from melanocytes to neighboring keratinocytes. Only when the melanosomes are transported from melanocytes to surrounding keratinocytes, can coloration of melanin on the skin be achieved.

TABLE 30

| Group | Mean | SD | *P*-value | Down-regulation Rate |
|---|---|---|---|---|
| BC | 1.00 | 0.12 | / | / |
| PC | 1.14 | 0.13 | 0.263 | / |
| Filtrate-0.1563% | 1.25 | 0.16 | 0.111 | / |
| Lysate-0.5% | 0.72 | 0.04 | 0.021** | 28.00% |
| Filtrate+lysate-0.0781% | 1.15 | 0.07 | 0.158 | / |

Experimental results are as shown in TABLE 30 and FIG. 32. The lysate has a high down-regulation rate on the melanosome transport-related gene *Myo5a.*

④ *MLPH* PCR

*MLPH* primer: F: 5'-CAGCGACCAGACAGATGAGG-3' (as set forth in SEQ ID NO: 36); and
R: 5'-GACCTTGAGGCTGAGTGGAG-3' (as set forth in SEQ ID NO: 37).
*MLPH,* small GTP binding protein (*Rab27a*) and myosin Va (*Myo5a*) form a ternary complex, which has a critical effect during transport of melanosomes, making the melanosomes aggregate at dendrite tips of melanocytes, and realizing transfer of the melanosomes from melanocytes to neighboring keratinocytes. Only when the melanosomes are transported from melanocytes to surrounding keratinocytes, can coloration of melanin on the skin be achieved.

TABLE 31

| Group | Mean | SD | *P*-value | Down-regulation Rate |
|---|---|---|---|---|
| BC | 1.00 | 0.09 | / | / |
| PC | 1.00 | 0.09 | 0.959 | / |
| Filtrate-0.1563% | 1.10 | 0.06 | 0.188 | / |
| Lysate-0.5% | 0.54 | 0.05 | 0.001** | 46.00% |
| Filtrate+lysate-0.0781% | 0.92 | 0.10 | 0.375 | 8.00% |

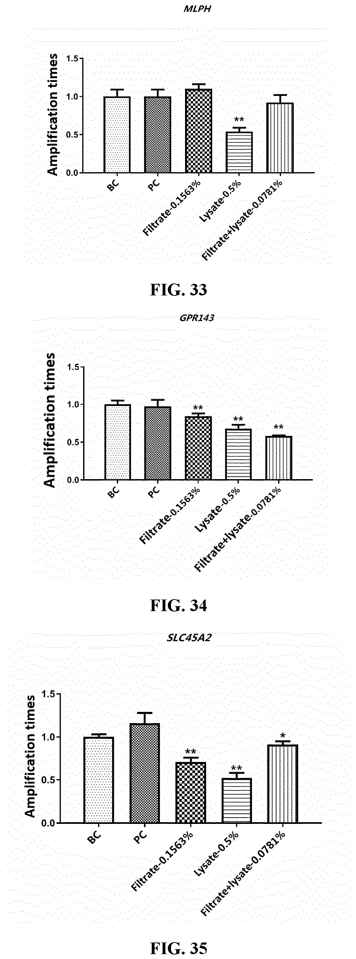

Experimental results are as shown in TABLE 31 and FIG. 33. The lysate has a high down-regulation rate on the expression of the melanosome transport-related gene *MLPH.*

(5) *GPR143* PCR

*GPR143* primer: F: 5'-CTGAAGGTTCTGATGCCAGC-3' (as set forth in SEQ ID NO: 38); and
R: 5'-TAGGTCTCCATGGGTTGGGA-3' (as set forth in SEQ ID NO: 39).
G protein-coupled receptor 143 gene has a key regulating effect in biosynthesis of melanosomes, and loss of function thereof will cause decreased quantity of early melanosomes and formation of giant melanosomes, and also reduce a melanin content.

TABLE 32

| Group | Mean | SD | *P*-value | Down-regulation Rate |
|---|---|---|---|---|
| BC | 1.00 | 0.05 | / | / |
| PC | 0.97 | 0.09 | 0.639 | / |
| Filtrate-0.1563% | 0.84 | 0.04 | 0.011** | 16.00% |

(continued)

| Group | Mean | SD | P-value | Down-regulation Rate |
|---|---|---|---|---|
| Lysate-0.5% | 0.68 | 0.05 | 0.001** | 32.00% |
| Filtrate+lysate-0.0781% | 0.58 | 0.01 | 0.000** | 42.00% |

Experimental results are as shown in TABLE 32 and FIG. 34. The filtrate, the lysate and the filtrate+lysate can significantly down-regulate the expression of the melanin synthesis-related gene *GPR143*.

⑥ *SLC45A2* PCR

*SLC45A2* primer: F: 5'-CCTCAATGGGGCTACTGTTGT-3' (as set forth in SEQ ID NO: 40); and R: 5'-GCCCATCAATGAAGTCGGCA-3' (as set forth in SEQ ID NO: 41).

*SLC45A2,* a type II transmembrane glycoprotein from SLC3 family, is a heavy-chain component of an amino acid transporter and an essential composition for it to transport amino acids, and has a function of regulating and controlling transport of amino acids.

TABLE 33

| Group | Mean | SD | P-value | Down-regulation Rate |
|---|---|---|---|---|
| BC | 1.00 | 0.03 | / | / |
| PC | 1.16 | 0.12 | 0.083 | / |
| Filtrate-0.1563% | 0.71 | 0.05 | 0.001** | 29.00% |
| Lysate-0.5% | 0.52 | 0.06 | 0.000** | 48.00% |
| Filtrate+lysate-0.0781% | 0.91 | 0.04 | 0.026* | 9.00% |

Experimental results are as shown in TABLE 33 and FIG. 35. The filtrate and the lysate can significantly down-regulate the expression of the melanin synthesis-related gene *SLC45A2*.

⑦ *MITF* PCR

*MITF* primer: F: 5'-AATTGTCCATCTGCCTCTGAGTAG-3' (as set forth in SEQ ID NO: 42); and R: 5'-GTATGACCAGGTTGCTTGTATGC-3' (as set forth in SEQ ID NO: 43).

*MITF* is a transcription factor involved in melanin systhesis.

TABLE 34

| Group | Mean | SD | P-value | Down-regulation Rate |
|---|---|---|---|---|
| BC | 1.00 | 0.09 | / | / |
| PC | 0.63 | 0.06 | 0.004** | / |
| Filtrate-0.1563% | 0.97 | 0.05 | 0.614 | 3.00% |
| Lysate-0.5% | 0.78 | 0.09 | 0.036** | 22.00% |
| Filtrate+lysate-0.0781% | 1.10 | 0.08 | 0.219 | / |

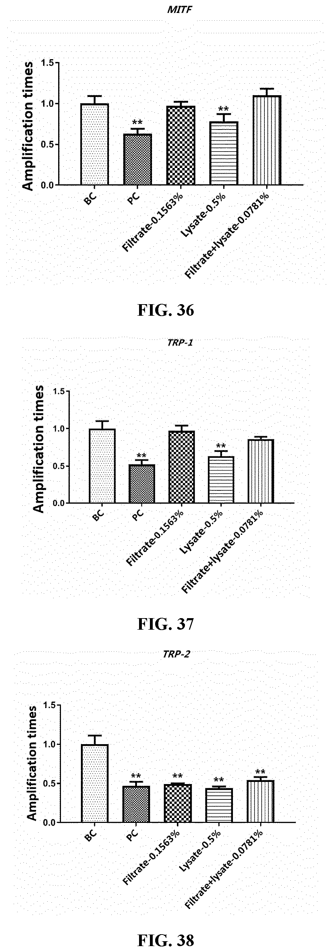

Experimental results are as shown in TABLE 34 and FIG. 36. The lysate can significantly down-regulate the expression of the melanin synthesis-related gene *MITF*.

⑧ *TRP-1* PCR

*TRP-1* primer: F: 5'-GTGCCACTGTTGAGGCTTTG-3' (as set forth in SEQ ID NO: 44); and R: 5'-ATGGGGATACTGAGGGCTGT-3' (as set forth in SEQ ID NO: 45).

*TRP-1* is involved in a process of synthesizing melanin from tyrosine.

TABLE 35

| Group | Mean | SD | *P*-value | Down-regulation Rate |
|---|---|---|---|---|
| BC | 1.00 | 0.10 | / | / |
| PC | 0.52 | 0.06 | 0.002** | / |
| Filtrate-0.1563% | 0.97 | 0.07 | 0.638 | 3.00% |
| Lysate-0.5% | 0.63 | 0.07 | 0.006** | 37.00% |
| Filtrate+lysate-0.0781% | 0.86 | 0.03 | 0.066 | 14.00% |

Experimental results are as shown in TABLE 35 and FIG. 37. The lysate and the filtrate+lysate can significantly down-regulate the expression of the melanin synthesis-related gene *TRP-1.*

⑨ *TRP-2* PCR

*TRP-2* primer: F: 5'-AGGATATACACCCCTAATGGAGACA-3' (as set forth in SEQ ID NO: 46); and R: 5'-AAGCAAGCAAAGCGGAAACTAC-3' (as set forth in SEQ ID NO: 47).
*TRP-2* is involved in a process of synthesizing melanin from tyrosine.

TABLE 36

| Group | Mean | SD | *P*-value | Down-regulation Rate |
|---|---|---|---|---|
| BC | 1.00 | 0.11 | / | / |
| PC | 0.47 | 0.05 | 0.001** | / |
| Filtrate-0.1563% | 0.49 | 0.01 | 0.001** | 51.00% |
| Lysate-0.5% | 0.44 | 0.02 | 0.001** | 56.00% |
| Filtrate+lysate-0.0781% | 0.54 | 0.04 | 0.002** | 46.00% |

Experimental results are as shown in TABLE 36 and FIG. 38. The filtrate, the lysate and the filtrate+lysate can significantly down-regulate the expression of *TRP-2.*

⑩ *Pmel17* PCR

*Pmel17* primer: F: 5'-AACAGCAGTGCAGATGACGA-3' (as set forth in SEQ ID NO: 48); and R: 5'-CGGTTGACCCTTCAGGTGTT-3' (as set forth in SEQ ID NO: 49).
*Pmel17* constitutes a structural component of melanosome and plays a mediating role in a process of synthesizing melanin from DHICA.

TABLE 37

| Group | Mean | SD | *P*-value | Down-regulation Rate |
|---|---|---|---|---|
| BC | 1.00 | 0.08 | / | / |
| PC | 0.92 | 0.04 | 0.220 | / |
| Filtrate-0.1563% | 0.67 | 0.04 | 0.003** | 33.00% |
| Lysate-0.5% | 0.57 | 0.05 | 0.001** | 43.00% |
| Filtrate+lysate-0.0781% | 0.79 | 0.02 | 0.012* | 21.00% |

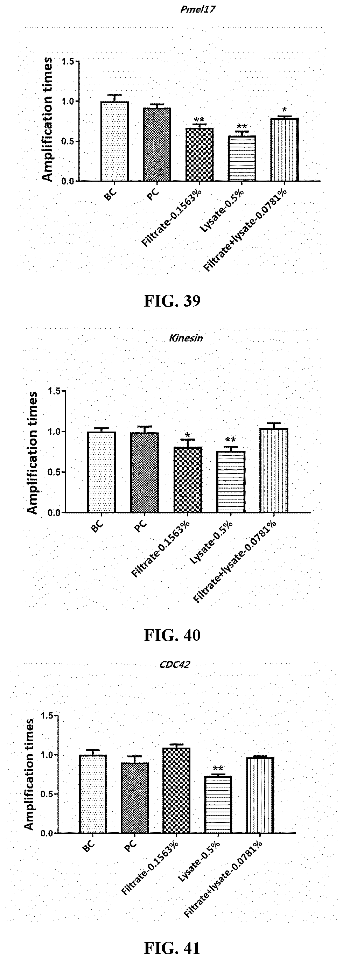

Experimental results are as shown in TABLE 37 and FIG. 39. The filtrate, the lysate and the filtrate+lysate can significantly down-regulate the expression of the melanosome transport-related gene *Pmel17.*

①① *Kinesin* PCR

*Kinesin* primer: F: 5'-GGGGACTCCGTAAAACAGGG-3' (as set forth in SEQ ID NO: 50); and R: 5'-TCCTCGTGCACGCTTAAGTT-3' (as set forth in SEQ ID NO: 51).
*Kinesin* constitutes a transport complex with *Rab1* and *SKIP,* and is involved in microtubule-mediated melanosome transport.

TABLE 38

| Group | Mean | SD | *P*-value | Down-regulation Rate |
|---|---|---|---|---|
| BC | 1.00 | 0.04 | / | / |
| PC | 0.99 | 0.07 | 0.845 | / |
| Filtrate-0.1563% | 0.81 | 0.09 | 0.032* | 19.00% |
| Lysate-0.5% | 0.76 | 0.05 | 0.003** | 24.00% |
| Filtrate+lysate-0.0781% | 1.04 | 0.06 | 0.358 | / |

Experimental results are as shown in TABLE 38 and FIG. 40. The filtrate and the lysate can significantly down-regulate the expression of the melanosome transport-related gene *Kinesin.*

①② *CDC42* PCR

*CDC42* primer: F: 5'-ATTACGACCGCTGAGTTATCCAC-3' (as set forth in SEQ ID NO: 52);
R: 5'-TCAGGCACCCACTTTTCTTTC-3' (as set forth in SEQ ID NO: 53).

Protein encoded by *CDC42* gene is a small GTPase of rhoc subfamily, and regulates and controls signaling pathways of a plurality of cellular functions, including cell form, migration, endocytosis and cell cycle progression.

TABLE 39

| Group | Mean | SD | *P*-value | Down-regulation Rate |
|---|---|---|---|---|
| BC | 1.00 | 0.06 | / | / |
| PC | 0.90 | 0.08 | 0.128 | / |
| Filtrate-0.1563% | 1.09 | 0.04 | 0.095 | / |
| Lysate-0.5% | 0.73 | 0.02 | 0.001** | 27.00% |
| Filtrate+ Lysate -0.0781% | 0.97 | 0.01 | 0.372 | 3.00% |

Experimental results are as shown in TABLE 39 and FIG. 41. The lysate can significantly down-regulate the expression of the melanosome transport-related gene *CDC42.*

**Verification Example 8 Anti-aging efficacy**

(1). DNA damage repair & epidermal atrophy based on 3D epidermal skin model

When a body is exposed to exogenous UV radiation, DNA damage may occur, and this damage may induce body aging. If such DNA damage is not repaired in time, symptoms such as aging of the body may occur.

TABLE 40

| | Group | Sample Name | Dosing Concentration | Induction Condition | Detection Model | Detection Index | Detection Method |
|---|---|---|---|---|---|---|---|
| | BC (blank control) | / | / | / | EpiKuits® | 1. Tissue morphology | 1. H&E |
| | NC (negative control) | / | / | | | | |
| | PC (positive control) | WY14 643 | 50μM | | | | |
| | Sample Group | Filtrate | 5% | UVB ( 600mJ/cm$^2$ ) | | | |
| | | Lysate | 5% | | | | |
| | | Filtrate+ lysate | 5% | | | 2. CPD | 2. IHC |
| | | Retinol | 0.1% | | | | |
| | | Bifida | 5% | | | | |

In the above, in the filtrate+lysate, the filtrate accounts for 50%, and the lysate accounts for 50%.

1) Testing method

Model dosing:

<1> According to test groups, models were transferred into 6-well plates (added with 0.9 mL of an EpiGrowth culture solution of corresponding groups in advance), and numbers of the test groups were marked on the 6-well plates. <2> UVB irradiation (a radiation dosage was 600 mJ/cm$^2$) was performed on the groups requiring UVB irradiation according to the test groups (as shown in TABLE 40). After the irradiation, a sample working solution was evenly distributed on surfaces of the models, and continued to be incubated in a $CO_2$ incubator (37 °C, 5% $CO_2$) for 24 h. <3> After incubation was finished, test substances remaining on surfaces of the models were cleaned with sterile PBS, and residual liquid inside and outside the models was wiped off with a sterile cotton swab.

Test of tissue morphology:

The models for detection of tissue morphology were fixed using 4% paraformaldehyde. After 24 h of fixation, H&E staining detection was performed, photographs were taken under a microscope and observed, and pictures were collected and analyzed.

Immunohistochemical detection:

The models for detection were fixed using 4% paraformaldehyde. After 24 h of fixation, immunohistochemical detection was performed, photographs were taken under a microscope and observed, and pictures were collected and analyzed.
Calculation of inhibition rate/down-regulation rate:

Inhibition rate/down-regulation rate (%) = (negative control group - sample)/negative control group $\times$ 100%

Statistical analysis of results:

GraphPad Prism was applied for plotting, and results were expressed as Mean±SD. Comparison between the groups was statistically analyzed using t-test. Statistical analysis was two-tailed. *P*<0.05 is considered to have a significant difference, and P<0.01 is considered to have an extremely significant difference.

Test Results:

① CPD: When a body is exposed to exogenous UV radiation, DNA damage may occur, and this damage may induce body aging. If such DNA damage is not repaired in time, symptoms such as aging of the body may occur.

TABLE 41 CPD Positive Cell Rate

| Groups | Positive cell Rate (%) | SD | P Value |
|---|---|---|---|
| BC | 0.00% | 0.00% | / |
| NC | 72.14% | 3.88% | 0.000 ** |
| PC | 60.55% | 3.11% | 0.016 * |
| Filtrate-5% | 60.75% | 4.37% | 0.028 * |
| Lysate-5% | 61.82% | 2.14% | 0.016 * |
| Filtrate+lysate-5% | 62.62% | 3.30% | 0.032 * |
| Retinol-0.1% | 58.31% | 5.41% | 0.023 * |
| Bifida ferment lysate-5% | 77.81% | 3.66% | 0.140 |

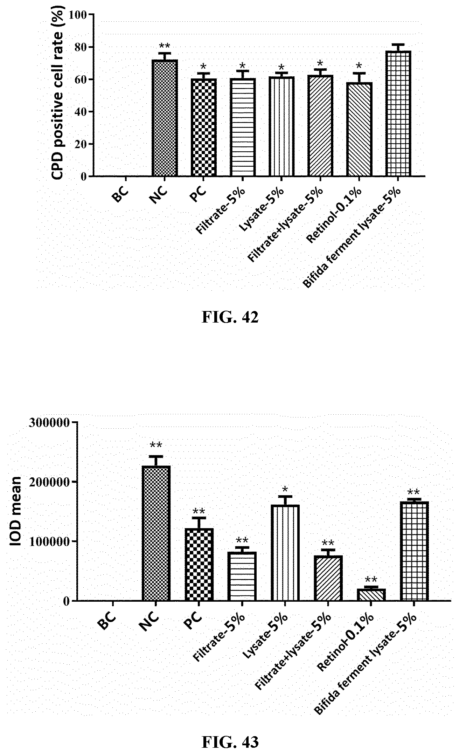

As can be seen from TABLE 41 and FIG. 42, CPD positive cells (smaller values are better): retinol 0.1% (58.31%*) < filtrate 5% (60.75%*) < lysate 5% (61.82%*) < filtrate+lysate 5% (62.62%*) < bifida ferment lysate 5% (77.81%)

TABLE 42 CPD Fluorescence Intensity Statistics

| Groups | IOD Mean | SD | P Value | Down-regulation Rate |
|---|---|---|---|---|
| BC | 0.00 | 0.00 | / | / |
| NC | 227349.69 | 14920.18 | 0.000 ** | / |
| PC | 122432.93 | 17005.09 | 0.002 ** | |
| Filtrate-5% | 82308.50 | 7331.10 | 0.000 ** | 63.80% |
| Lysate-5% | 161808.72 | 13328.76 | 0.011 * | 28.83% |
| Filtrate+lysate-5% | 76022.83 | 9702.35 | 0.000 ** | 66.56% |
| Retinol-0.1% | 20503.43 | 2967.70 | 0.000** | 90.98% |
| Bifida ferment lysate-5% | 166751.69 | 3878.76 | 0.006** | 26.65% |

As can be seen from TABLE 42 and FIG. 43, the down-regulation rate of CPD fluorescence intensity is: retinol 0.1% (90.98%**) > filtrate+lysate 5% (66.56%**) > filtrate 5% (63.80%*) > lysate 5% (28.83%*) > bifida ferment lysate 5% (26.65%**).

(2) Epidermal atrophy: Tissue morphology is physiological structure analysis of appearance of tissues after HE staining. The ability of the test substance to resist UVB can be evaluated by analyzing thickness changes of epidermis and growth of four layers of cells on the surface after use of the test substance.

TABLE 43

| Groups | Sunburn cells | SD | P Value | Inhibition Rate |
|---|---|---|---|---|
| BC | 0 | 0 | / | / |
| NC | 10 | 4 | 0.011 * | / |
| PC (WY14643 50μM) | 3 | 1 | 0.046 * | / |
| Filtrate-5% | 0 | 0 | 0.011 * | 100.00% |
| Lysate-5% | 0 | 1 | 0.013 * | 96.59% |
| Filtrate+lysate-5% | 1 | 1 | 0,017 * | 93.07% |
| Retinol-0.1% | 0 | 0 | 0.011 * | 100.00% |
| Bifida ferment lysate-5% | 5 | 2 | 0.116 | 51.71% |

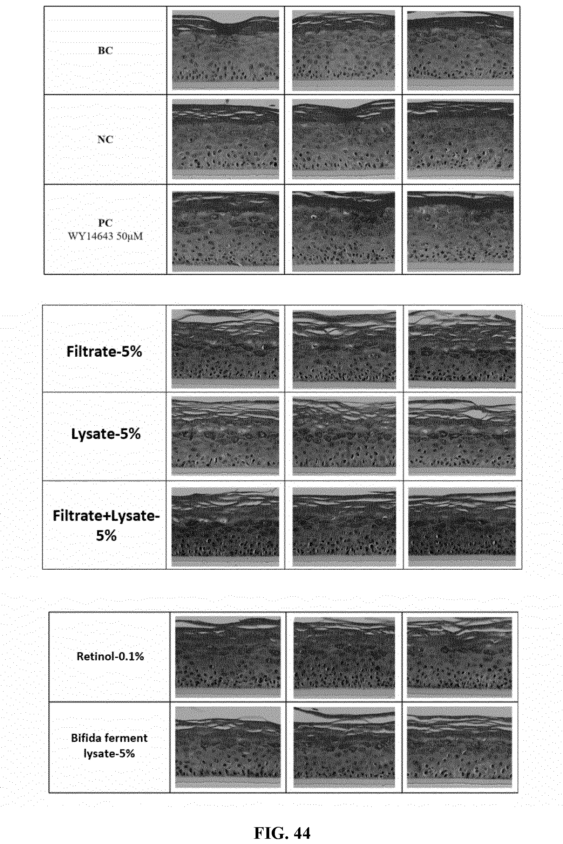

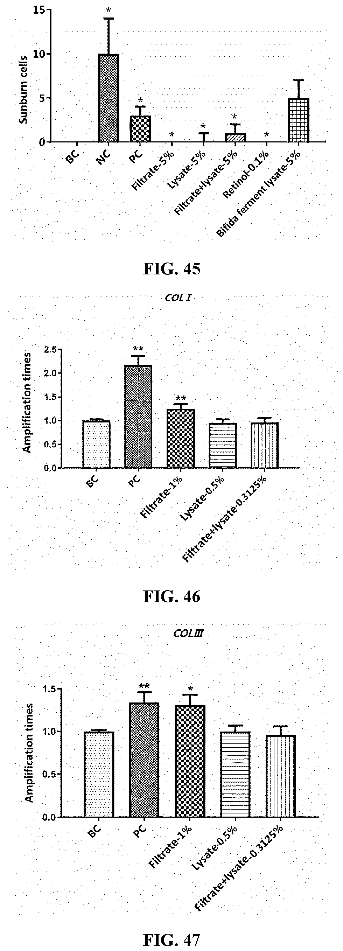

As shown in TABLE 43 and FIG. 44 and FIG. 45, the inhibition rates of sunburn cells are: filtrate 5% (100%*) = retinol 0.1% (100%*) > lysate 5% (96.59%*) > filtrate+lysate 5% (93.07%*) > bifida ferment lysate 5% (51.71%).

(2) Test of fibroblastic-based anti-aging effect

TABLE 44

| Group | Sample Name | Dosing Concentration | Detection Model | Detection Item | Detection Method |
|---|---|---|---|---|---|
| Blank control (BC) | / | / | Fibroblast | *COLI, COLIII, COLIV, COLVII, DCN, VCAN, BGN, elastin, fibronectin, HASI* | qRT-PCR |
| Positive control (PC) | TGFβ1 | 100ng/mL | | | |
| Sample group | Filtrate | 1% | | | |
| | Lysate | 0.5% | | | |
| | Filtrate+lysate | 0.3125% | | | |

In the above, in the filtrate+lysate, the filtrate accounts for 50%, and the lysate accounts for 50%.

1) Testing method

Cell inoculation: After resuscitation of cells, when a plating rate reached about 60%, the cells were inoculated in 6-well plates, and incubated overnight in the $CO_2$ incubator (37 °C, 5% $CO_2$).

Dosing: When the plating rate of the cells in the 6-well plates reached about 40%-60%, dosing was performed for the groups respectively, with each group in triplicate. The blank control group was added with 2 mL of a culture solution per well, the positive control group was added with 2 mL of a TGF-β1-containing culture solution per well, and the sample group was added with 2 mL of a test sample-containing culture solution at a corresponding concentration per well. After the dosing was completed, the 6-well plates were cultured in the $CO_2$ incubator (37 °C, 5% $CO_2$) for 24 h.

Cell collection: After incubation was finished, an original solution was sucked out and discarded, PBS cleaning was performed twice, and each well was added with 1 mL of RNAiso Plus, to pipet and lyse the cells, after which samples were collected.

Detection of gene expression: RNA was extracted and subjected to reverse transcription to cDNA to undergo fluorescence quantitative PCR detection, and result was calculated by a $2^{-\Delta\Delta CT}$ method.

Calculation of up-regulation rate: up-regulation rate (%) = (sample group - blank control group)/blank control group $\times$ 100%

Statistical analysis of results: GraphPad Prism Program software was applied for plotting, and results were expressed as Mean±SD. Comparison between the groups was statistically analyzed using t-test. Statistical analysis was two-tailed. *P*<0.05 is considered to have a significant difference, and *P*<0.01 is considered to have an extremely significant difference.

2) Test Results

① *COL I*

*COL I* primer: F:5'-GTGGCAGTGATGGAAGTGTG-3' (as set forth in SEQ ID NO: 54)
R: 5'-AGGACCAGCGTTACCAACAG-3' (as set forth in SEQ ID NO: 55)
*COL I* is collagen I. Collagen accounts for about 80% of the dermis of the skin, so as to make the skin plump. Promoting increase of *COL I* content can achieve a certain effect of resisting wrinkle generation.

TABLE 45

| Group | Mean | SD | *P*-value | Up-regulation Rate |
|---|---|---|---|---|
| BC | 1.00 | 0.03 | / | / |

(continued)

| Group | Mean | SD | P-value | Up-regulation Rate |
|---|---|---|---|---|
| PC | 2.17 | 0.19 | 0.000** | / |
| Filtrate-1% | 1.25 | 0.10 | 0.017** | 25.00% |
| Lysate-0.5% | 0.95 | 0.08 | 0.372 | / |
| Filtrate+lysate-0.3125% | 0.96 | 0.10 | 0.574 | / |

Experimental results are as shown in TABLE 45 and FIG. 46. The filtrate can significantly up-regulate the expression of the type I collagen *COL I* gene.

(2) *COL III*

*COL III* primer: F: 5'-ACCAGGAGCTAACGGTCTCA-3' (as set forth in SEQ ID NO: 56); and R: 5'-TCTGATCCAGGGTTTCCATC-3' (as set forth in SEQ ID NO: 57).
*COL III* is collagen III, which is assembled together with collagen I into collagen fibers. The collagen fibers play an important role in toughness of the skin.

TABLE 46

| Group | Mean | SD | P-value | Up-regulation Rate |
|---|---|---|---|---|
| BC | 1.00 | 0.02 | / | / |
| PC | 1.34 | 0.12 | 0.008** | / |
| Filtrate-1% | 1.31 | 0.12 | 0.010* | 31.00% |
| Lysate-0.5% | 1.00 | 0.07 | 0.929 | / |
| Filtrate+lysate-0.3125% | 0.96 | 0.10 | 0.562 | / |

Experimental results are as shown in TABLE 46 and FIG. 47. The filtrate can significantly up-regulate the expression of the type III collagen *COL III* gene.

(3) *COL IV*

*COL IV* primer: F: 5'-AGGTGTCATTGGGTTTCCTG-3' (as set forth in SEQ ID NO: 58); and R: 5'-GGTCCTCTTGTCCCTTTTGTT-3' (as set forth in SEQ ID NO: 59).
Junction between epidermis and dermis of the skin is also called as DEJ (Dermal Epidermal Junction). This layer of structure is equivalent to a layer of junction structure between the epidermis and the dermis. Reduced DEJ expression is a typical feature of photo-aged skin. DEJ also affects appearance of the skin such as elasticity and tightness. *COL IV* is collagen IV, mainly located at the dermal epidermal junction and plays an important role in an aging process of skin.

TABLE 47

| Group | Mean | SD | P-value | Up-regulation Rate |
|---|---|---|---|---|
| BC | 1.00 | 0.05 | / | / |
| PC | 2.46 | 0.28 | 0.001 ** | / |
| Filtrate-1% | 1.30 | 0.08 | 0.006 ** | 30.00% |
| Lysate-0.5% | 0.95 | 0.05 | 0.260 | / |
| Filtrate+lysate-0.3125% | 1.13 | 0.08 | 0.086 | 13.00% |

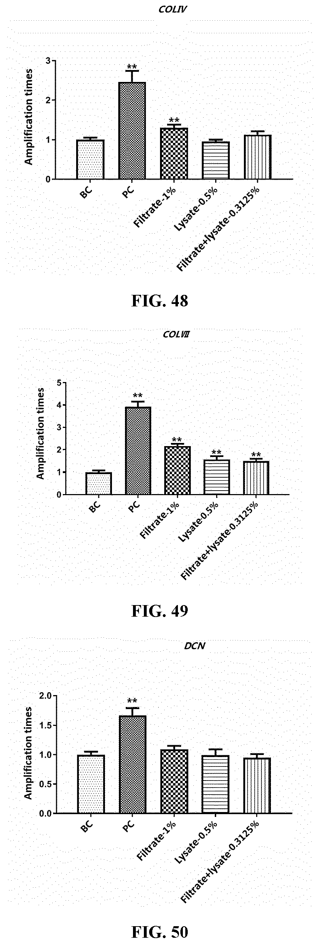

Experimental results are as shown in TABLE 47 and FIG. 48. The filtrate can significantly up-regulate the expression of the type IV collagen *COL IV* gene.

④ *COL VII*

*COL VII* primer: F: 5'-ACTGTGATTGCCCTCTACGC-3' (as set forth in SEQ ID NO: 60); and R: 5'-GGCTGTGGTATTCTGGATGG-3' (as set forth in SEQ ID NO: 61).
Junction between the epidermis and the dermis of the skin is also called as DEJ (Dermal Epidermal Junction).

This layer of structure is equivalent to a layer of junction structure between the epidermis and the dermis. Reduced DEJ expression is a typical feature of photo-aged skin. DEJ also affects appearance of the skin such as elasticity and tightness. *COL VII* is collagen VII, similar to *COL IV,* is also located at the dermal epidermal junction, and plays an important role in the aging process of skin.

TABLE 48

| Group | Mean | SD | *P*-value | Up-regulation Rate |
|---|---|---|---|---|
| BC | 1.00 | 0.07 | / | / |
| PC | 3.92 | 0.23 | 0.000 ** | / |
| Filtrate-1% | 2.16 | 0.10 | 0.000 ** | 116.00% |
| Lysate-0.5% | 1.56 | 0.15 | 0.004 ** | 56.00% |
| Filtrate+lysate-0.3125% | 1.49 | 0.10 | 0.002 ** | 49.00% |

Experimental results are as shown in TABLE 48 and FIG. 49. The filtrate, the lysate and the filtrate+lysate can significantly up-regulate the expression of the type VII collagen *COL VII* gene.

⑤ *DCN*

Decorin (*DCN*) primer: F: 5'-GTGTGCTTGACAGTGTTCTAGTG-3' (as set forth in SEQ ID NO: 62); and R: 5'-AGTTCTGCTTGACATTCCTCCA-3' (as set forth in SEQ ID NO: 63).

Decorin is a component with higher abundance of GAG in skin ECM, and is a leucine-rich proteoglycan with a small molecular weight. Protein core may carry characteristic dermatan/chondroitin sulfate GAG chains. Decorin can bind to a specific position on a surface of type I collagen fibril. Such binding is stabilized by electrostatic interaction of the GAG chains, is indispensable for assembling collagen fibers, and inhibits cleavage of collagen fibers by matrix metalloprotease-1.

TABLE 49

| Group | Mean | SD | *P*-value |
|---|---|---|---|
| BC | 1.00 | 0.05 | / |
| PC | 1.67 | 0.12 | 0.001** |
| Filtrate-1% | 1.09 | 0.06 | 0.117 |
| Lysate-0.5% | 0.99 | 0.10 | 0.826 |
| Filtrate+lysate-0.3125% | 0.95 | 0.06 | 0.306 |

Experimental results are as shown in TABLE 49 and FIG. 50. The lysate and the lysate do not affect the expression of the proteoglycan-related gene DCN.

⑥ *VCAN*

Versican (*VCAN*) primer: F: 5'-GTGTGGGCATTTCTTCCTGTTAG-3' (as set forth in SEQ ID NO: 64); and R: 5'-TCAAAGTCATCTTCAGCAGTCACT-3' (as set forth in SEQ ID NO: 65).

Versican is a main proteoglycan in the dermis, belongs to a macromolecular polymerization type, usually forms a macromolecular polymer with hyaluronic acid, and is an important GAG type in dermal tissues.

TABLE 50

| Group | Mean | SD | *P*-value | Up-regulation Rate |
|---|---|---|---|---|
| BC | 1.00 | 0.02 | / | / |
| PC | 1.68 | 0.10 | 0.000** | / |
| Filtrate-1% | 1.18 | 0.04 | 0.002** | 18.00% |
| Lysate-0.5% | 0.99 | 0.10 | 0.884 | / |
| Filtrate+lysate-0.3125% | 0.94 | 0.11 | 0.434 | / |

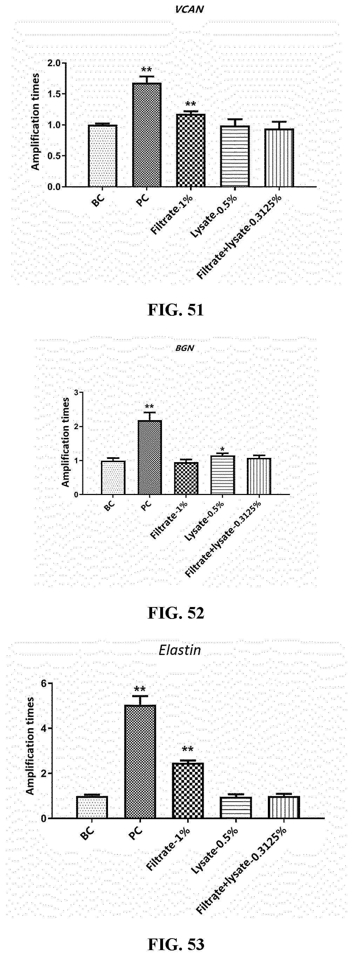

Experimental results are as shown in TABLE 50 and FIG. 51. The filtrate can obviously up-regulate the expression of the proteoglycan-related gene *VCAN.*

⑦ *BGN*

Biglycan (*BGN*) primer: F: 5'-ACCATCAACCGCCAGAGTC-3' (as set forth in SEQ ID NO: 66); and
R: 5'-GACAGCCACCGACCTCAG-3' (as set forth in SEQ ID NO: 67).
Biglycan, having an effect similar to that of Decorin, is also leucine-rich proteoglycan with a small molecular weight, and can bind to a surface of type I collagen fibril. Biglycan is different from Decorin in that this proteoglycan can be secreted in both epidermal cells and fibroblasts.

TABLE 51

| Group | Mean | SD | *P*-value | Up-regulation Rate |
|---|---|---|---|---|
| BC | 1.00 | 0.07 | / | / |
| PC | 2.18 | 0.23 | 0.001** | / |
| Filtrate-1% | 0.95 | 0.08 | 0.443 | / |
| Lysate-0.5% | 1.151 | 0.06 | 0.044* | 15.00% |
| Filtrate+lysate-0.3125% | 1.08 | 0.07 | 0.246 | 8.00% |

Experimental results are as shown in TABLE 51 and FIG. 52. The lysate can significantly up-regulate the expression of the proteoglycan-related gene *BGN.*

⑧ Elastin

Elastin primer: F: 5'-TCCAGGTGTAGGTGGAGCTT-3' (as set forth in SEQ ID NO: 68); and
R: 5'-GTGTAGGGCAGTCCATAGCC-3' (as set forth in SEQ ID NO: 69).
Elastin is a main protein constituting elastic fiber, and a reduced content causes the elastic fiber to become sparse and skin elasticity to decrease.

TABLE 52

| Group | Mean | SD | *P*-value | Up-regulation Rate |
|---|---|---|---|---|
| BC | 1.00 | 0.05 | / | / |
| PC | 5.05 | 0.38 | 0.000** | / |
| Filtrate-1% | 2.47 | 0.10 | 0.000** | 147.00% |
| Lysate-0.5% | 0.96 | 0.11 | 0.583 | / |
| Filtrate+lysate-0.3125% | 1.00 | 0.09 | 0.974 | / |

Experimental results are as shown in TABLE 52 and FIG. 53. The filtrate can significantly up-regulate the expression of elastin.

⑨ Fibronectin

Fibronectin primer: F: 5'-AGCCAGTCGAGGATACCTGT-3' (as set forth in SEQ ID NO: 70); and
R: 5'-GCACCAAAGCCTGAAACCAG-3' (as set forth in SEQ ID NO: 71).
Fibronectin (FN), as a high-molecular glycoprotein essential to the human body, widely exists in tissues and tissue fluids, is involved in cell migration, adhesion, proliferation, hemostasis, tissue repair and embryonic development in living activities of the body, has an effect of growth factor, can promote cell proliferation, induces epidermal cells to pass through granulation tissues, and promotes reconstruction of subcuticular basement membrane and normal keratinization process.

TABLE 53

| Group | Mean | SD | *P*-value | Up-regulation Rate |
|---|---|---|---|---|
| BC | 1.00 | 0.04 | / | / |

(continued)

| Group | Mean | SD | *P*-value | Up-regulation Rate |
|---|---|---|---|---|
| PC | 1.97 | 0.14 | 0.000** | / |
| Filtrate-1% | 1.70 | 0.19 | 0.003** | 70.00% |
| Lysate-0.5% | 1.27 | 0.04 | 0.001** | 27.00% |
| Filtrate+lysate-0.3125% | 0.99 | 0.05 | 0.718 | / |

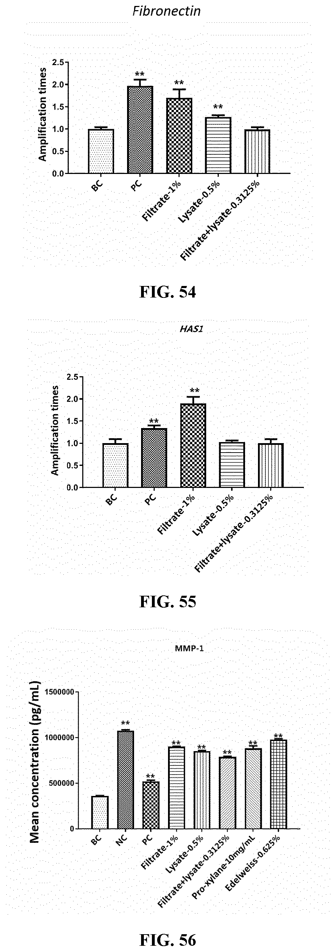

Experimental results are as shown in TABLE 53 and FIG. 54. The filtrate and the lysate can significantly up-regulate the expression of fibronectin.

⑩ *HAS1*

*HAS1* primer: F: 5'-ACTCGGACACAAGGTTGGAC-3' (as set forth in SEQ ID NO: 72); and R: 5'-TTAGGAAGCTGACCCAGGAG-3' (as set forth in SEQ ID NO: 73).

*HAS1,* a hyaluronic acid synthase, is a key enzyme for mediating hyaluronic acid synthesis. Promoting expression of *HAS1* can promote synthesis of more hyaluronic acids, and thus tightening efficacy of the test substance can be analyzed by detecting *HAS1* content.

TABLE 54

| Group | Mean | SD | *P*-value | Up-regulation Rate |
|---|---|---|---|---|
| BC | 1.00 | 0.09 | / | / |
| PC | 1.34 | 0.06 | 0.006** | / |
| Filtrate-1% | 1.90 | 0.15 | 0.001** | 90.00% |
| Lysate-0.5% | 1.03 | 0.03 | 0.638 | / |
| Filtrate+lysate-0.3125% | 1.00 | 0.09 | 0.995 | / |

Experimental results are as shown in TABLE 54 and FIG. 55. The filtrate can significantly up-regulate the expression of the hyaluronic acid synthase *HAS1.*

(3) Test based on UVA-irradiated fibroblasts

1) Testing method-special dosing (UVA continuous irradiation)

TABLE 55

| Group | Sample Name | Dosing Concentration | Stimulation Condition | Detection Model | Detection Item | Detection Method |
|---|---|---|---|---|---|---|
| Blank control (BC) | / | / | a | | | |
| Negative control (NC) | / | / | | | | |
| Positive control (PC) | TGFβ1 | 100ng/mL | | Fibroblast | MMP-1 | ELISA |
| Sample group | Filtrate | 1% | UVA radiation (30J/cm$^2$) | | | |
| | Lysate | 0.5% | | | | |
| | Filtrate+lysate | 0.3125% | | | | |
| | Pro-xylane | 10mg/mL | | | | |
| | Edelweiss | 0.625% | | | | |

TABLE 56

| Group | Sample Name | Dosing Concentration | Stimulation Condition | Detection Model | Detection Item | Detection Method |
|---|---|---|---|---|---|---|
| Blank control (BC) | | / | / | | | |
| Negative control (NC) | / | | / | | | |
| Positive control (PC) | TGFβ1 | 100ng/mL | UVA radiation (30J/cm$^2$) | Fibroblast | *p21, p16, NF-κB* | qRT-PCR |
| Sample group | Filtrate | 1% | | | | |
| | Lysate | 0.5% | | | | |
| | Filtrate+lysate | 0.3125% | | | | |
| | Pro-xylane | 10mg/mL | | | | |

Cell inoculation: After resuscitation of cells, when a plating rate reached about 60%, the cells were inoculated in 6-well plates, and incubated overnight in the $CO_2$ incubator (37 °C, 5% $CO_2$).

Dosing: When the plating rate of the cells in the 6-well plates reached about 50%-60%, UVA irradiation of 30 J/cm$^2$ was performed on groups with UVA irradiation, and after the irradiation was finished, dosing was performed for the groups respectively, with each group in triplicate. The blank control group and the negative control group were added with 2 mL of the culture solution per well, the positive control group was added with 2 mL of the TGF-β1-containing culture solution per well, and the sample group was added with 2 mL of the test sample-containing culture solution at a corresponding concentration per well. After the dosing was completed, the 6-well plates were cultured in the $CO_2$ incubator (37 °C, 5% $CO_2$) 23/52 for 24 h. The above operations were repeated for 3 times.

ELISA test: After the incubation was finished, cell culture supernatant was collected in a centrifuge tube, and after the collection was finished, a sample for ELISA detection was cryopreserved in a -80 °C freezer, and detection and analysis were performed according to operation instructions of an ELISA kit.

Detection of gene expression: After the incubation was finished, PBS cleaning was performed twice with 1 mL/well, each well was added with 1 mL of RNAiso Plus, to pipet and lyse the cells, after which samples were collected. RNA was extracted and subjected to reverse transcription to cDNA to undergo fluorescence quantitative PCR detection, and result was calculated by the $2^{-\Delta\Delta CT}$ method.

Calculation of up-regulation rate and down-regulation rate:

Up-regulation rate = (sample group - negative control group)/negative control group $\times$ 100%

Down-regulation rate = (negative control group - sample group)/negative control group $\times$ 100%

Statistical analysis of results: GraphPad Prism Program software was applied for plotting, and results were expressed as Mean$\pm$SD. Comparison between the groups was statistically analyzed using t-test. Statistical analysis was two-tailed. $P<0.05$ is considered to have a significant difference, and $P<0.01$ is considered to have an extremely significant difference.

In the above: * indicates significant difference, and ** indicates extremely significant difference.

Test results:

① *MMP1* ELISA

*MMP1,* a matrix metalloproteinase, has a main effect of degrading collagen. Collagen is a main structural protein of the dermis of the skin. When the collagen is degraded, a mechanical supporting force of the skin is lost, causing occurrence of wrinkles. After the test substance acts on the cell model, the anti-wrinkle efficacy of the test substance is evaluated by detecting changes in the expression of *MMP1*.

TABLE 57

| Group | Mean Concentration (pg/mL) | SD | P-value | Down-regulation Rate (vs NC) |
|---|---|---|---|---|
| BC | 359379.29 | 4164.50 | / | / |
| NC | 1075513.35 | 10117.05 | 0.000 ** | / |
| PC | 519820.49 | 12631.22 | 0.000 ** | / |
| Filtrate-1% | 900870.78 | 3500.88 | 0.000 ** | 16.24% |
| Lysate-0.5% | 850091.47 | 9031.59 | 0.000 ** | 20.96% |
| Filtrate+lysate-0.3125% | 788417.27 | 5893.60 | 0.000 ** | 26.69% |
| Pro-xylane-10 mg/mL | 882584.62 | 26433.99 | 0.000 ** | 17.94% |
| Edelweiss-0.625% | 976899.76 | 9136.17 | 0.000 ** | 9.17% |

Experimental results are as shown in TABLE 57 and FIG. 56. The filtrate, the lysate and the filtrate+lysate can significantly down-regulate generation of the *MMP1* protein.

(2) *NF-κB* PCR

The effect of ultraviolet rays on the skin is caused by ROS generated. Excess free radicals activate an *NF-κB* signaling pathway and an MAPK signaling pathway, further activate *AP-1* and *NF-κB*, further promote a level of TNF-α and expression of MMPs, induce ECM degradation, and accelerate the skin aging.

*NF-κB* primer: F: 5'-GGTGCGGCTCATGTTTACAG-3' (as set forth in SEQ ID NO: 74); and R: 5'-GATGGCGTCTGATACCACGG-3' (as set forth in SEQ ID NO: 75).

TABLE 58

| Group | Mean | SD | P-value | Inhibition Rate (vs NC) |
|---|---|---|---|---|
| BC | 1.00 | 0.12 | / | / |
| NC | 1.52 | 0.07 | 0.003 ** | / |
| PC | 0.96 | 0.02 | 0.000 ** | / |
| Filtrate-0.1% | 0.60 | 0.02 | 0.000 ** | 60.53% |
| Lysate-0.5% | 0.88 | 0.01 | 0.000 ** | 42.11%, |
| Filtrate+lysate-0.3125% | 0.73 | 0.05 | 0.000 ** | 51.97% |
| Pro-xylane-10 mg/mL | 0.53 | 0.05 | 0.000 ** | 65.13% |

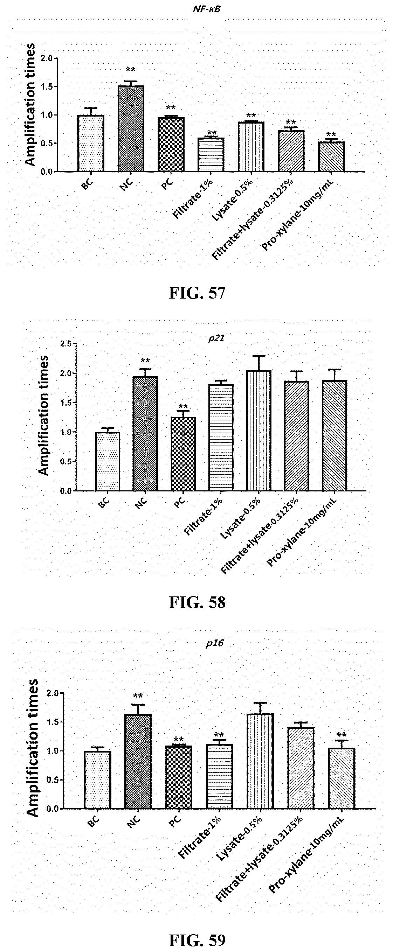

Experimental results are as shown in TABLE 58 and FIG. 57. The filtrate, the lysate and the filtrate+lysate can significantly inhibit the expression of the inflammatory signaling pathway-related gene *NF-κB*.

③ *p21* PCR

*p21* gene is a marker gene of cell aging.

*p21* primer: F: 5'-TCTCTGTGTTAGGGGTATATGATGG-3' (as set forth in SEQ ID NO: 76); and R: 5'-GAAGGTCGCTGGACGATTTG-3' (as set forth in SEQ ID NO: 77).

TABLE 59

| Group | Mean | SD | P-value |
|---|---|---|---|
| BC | 1.00 | 0.07 | / |
| NC | 1.95 | 0.12 | 0.000 ** |
| PC | 1.26 | 0.10 | 0.002 ** |
| Filtrate-1 % | 1.81 | 0.06 | 0.133 |
| Lysate-0.5% | 2.05 | 0.24 | 0.588 |
| Filtrate+lysate-0.3125% | 1.87 | 0.16 | 0.494 |
| Pro-xylane-10 mg/mL | 1.88 | 0.18 | 0.558 |

Experimental results are as shown in TABLE 59 and FIG. 58. The filtrate and the filtrate+lysate can significantly down-regulate the expression of the aging-related gene *p21*.

④*p16* PCR

*p16* gene is a marker gene of cell aging.

*p16* primer: F: 5'-CTCTGAGAAACCTCGGGAAAC-3' (as set forth in SEQ ID NO: 78); and R: 5'-ATGAAAACTACGAAAGCGGG-3' (as set forth in SEQ ID NO: 79).

TABLE 60

| Group | Mean | SD | *P*-value | Down-regulation Rate (vs NC) |
|---|---|---|---|---|
| BC | 1.00 | 0.06 | / | / |
| NC | 1.64 | 0.16 | 0.003 ** | / |
| PC | 1.09 | 0.02 | 0.004 ** | / |
| Filtrate-1% | 1.12 | 0.07 | 0.007 ** | 31.71% |
| Lysate-0.5% | 1.65 | 0.18 | 0.959 | / |
| Filtrate+lysate-0.3125% | 1.41 | 0.08 | 0.094 | 14.02% |
| Pro-xylane-10 mg/mL | 1.06 | 0.12 | 0.008 ** | 35.37% |

Experimental results are as shown in TABLE 60 and FIG. 59. The filtrate can significantly down-regulate the expression of the aging-related gene *p16*.

2) Testing method-conventional dosing

TABLE 61

| Group | Sample Name | Dosing Concentration | Stimulation Condition | Detection Model | Detection Item | Detection Method |
|---|---|---|---|---|---|---|
| Blank control (BC) | / | / | / | | | |
| Negative control (NC) | / | / | | | | |
| Positive control (PC) | TGFβ1 | 100ng/mL | UVA radiation (30J/cm$^2$) | Fibroblast | *MMP1, MMP3, TIMP-1, TNFR* | qRT-PCR |
| Sample group | Filtrate | 1% | | | | |
| | Lysate | 0.5% | | | | |
| | Filtrate+lysate | 0.3125% | | | | |

Cell inoculation: After resuscitation of cells, when the plating rate reached about 60%, the cells were inoculated in 6-well plates, and incubated overnight in the $CO_2$ incubator (5% $CO_2$).

Dosing: According to test groups in TABLE 61, when the plating rate of the cells in the 6-well plates reached about 40%-60%, dosing was performed for the groups respectively, with each group in triplicate. The blank control group and the negative control group were added with 2 mL of the culture solution per well, the positive control group was added with 2 mL of the TGF-β1-containing culture solution per well, and the sample group was added with 2 mL of the test sample-containing culture solution at a corresponding concentration per well. After the dosing was completed, the 6-well plates were cultured in the $CO_2$ incubator (37 °C, 5% $CO_2$) for 24 h.

UVA irradiation: according to the test groups, except the blank control group, other groups were all subjected to UVA irradiation, with an irradiation dosage of 30 J/cm$^2$. After the irradiation was finished, culture was continued in the $CO_2$ incubator (37 °C, 5% $CO_2$) for 24 h.

ELISA test: After the incubation was finished, cell culture supernatant was collected in a centrifuge tube, and after the collection was finished, a sample for ELISA detection was cryopreserved in a -80 °C freezer, and detection and analysis were performed according to operation instructions of an ELISA kit.

Detection if gene expression: After the incubation was finished, PBS cleaning was performed twice, and each well was added with 1 mL of RNAiso Plus, to pipet and lyse the cells, after which samples were collected. RNA was extracted and subjected to reverse transcription to cDNA to undergo fluorescence quantitative PCR detection, and result was calculated by the $2^{-\Delta\Delta CT}$ method.

Calculation of up-regulation rate and down-regulation rate:

Up-regulation rate = (sample group - negative control group)/negative control group $\times$ 100%

Down-regulation rate = (negative control group - sample group)/negative control group $\times$ 100%

Statistical analysis of results: GraphPad Prism Program software was applied for plotting, and results were expressed as Mean±SD. Comparison between the groups was statistically analyzed using t-test. Statistical analysis was two-tailed. $P<0.05$ is considered to have a significant difference, and $P<0.01$ is considered to have an extremely significant difference.

In the above: * indicates significant difference, and ** indicates extremely significant difference.

Test Results:

① *MMP1* PCR

*MMP,* a matrix metalloproteinase, has a main effect of degrading collagen. Collagen is a main structural protein of the dermis of the skin. When the collagen is degraded, a mechanical supporting force of the skin is lost, causing occurrence of wrinkles. After the test substance acts on the cell model, the anti-wrinkle efficacy of the test substance is evaluated by detecting changes in the expression of *MMP1*/*MMP3*.

*MMP1* primer: F: 5'-ACAACTGCCAAATGGGCTTGA-3' (as set forth in SEQ ID NO: 80); and R: 5'-CTGTCCCTGAACAGCCCAGTACTTA-3' (as set forth in SEQ ID NO: 81).

TABLE 62

| Group | Mean | SD | *P*-value | Down-regulation Rate (vs NC) |
|---|---|---|---|---|
| BC | 1.00 | 0.09 | / | / |
| NC | 1.47 | 0.04 | 0.001 ** | / |
| PC | 1.11 | 0.10 | 0.004 ** | / |
| Filtrate-1% | 1.32 | 0.10 | 0.080 | 10.20% |
| Lysate-0.5% | 1.07 | 0.03 | 0.000 ** | 27.21% |
| Filtrate+lysate-0.3125% | 1.39 | 0.11 | 0.312 | 5.44% |

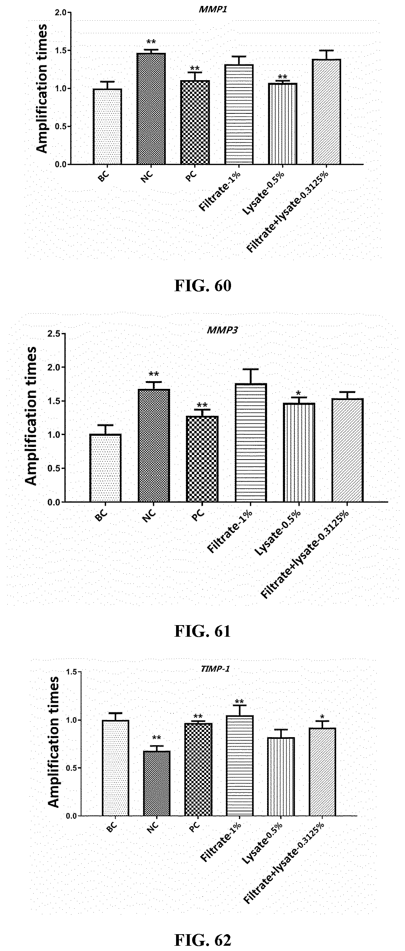

Experimental results are as shown in TABLE 62 and FIG. 60. The filtrate, the lysate and the filtrate+lysate can significantly down-regulate the expression of the matrix metalloproteinase *MMP1* gene.

(2) *MMP3* PCR

*MMP,* a matrix metalloproteinase, has a main effect of degrading collagen. Collagen is a main structural protein of the dermis of the skin. When the collagen is degraded, a mechanical supporting force of the skin is lost, causing occurrence of wrinkles. After the test substance acts on the cell model, the anti-wrinkle efficacy of the test substance is evaluated by detecting changes in the expression of *MMP1*/*MMP3*.

*MMP3* primer: F: 5'-CAGGCTTTCCCAAGCAAATA-3' (as set forth in SEQ ID NO: 82); and R: 5'-TGGGTCAAACTCCAACTGTG-3' (as set forth in SEQ ID NO: 83).

TABLE 63

| Group | Mean | SD | *P*-value | Down-regulation Rate (*vs* NC) |
|---|---|---|---|---|
| BC | 1.01 | 0.13 | / | / |
| NC | 1.68 | 0.10 | 0.002 ** | / |
| PC | 1.28 | 0.09 | 0.006 ** | / |
| Filtrate-1% | 1.76 | 0.21 | 0.579 | / |

(continued)

| Group | Mean | SD | P-value | Down-regulation Rate (vs NC) |
|---|---|---|---|---|
| Lysate-0.5% | 1.47 | 0.08 | 0.042 * | 12.50% |
| Filtrate+lysate-0.3125% | 1.54 | 0.09 | 0.125 | 8.33% |

Experimental results are as shown in TABLE 63 and FIG. 61. The lysate and the filtrate+lysate can significantly down-regulate the expression of the matrix metalloproteinase *MMP3* gene.

(3) *TIMP-1* PCR

A main biological effect of tissue inhibitor of metalloproteinase 1 lies in inhibiting matrix metalloproteinase MMP family, thereby reducing degradation of ECM in the skin by the MMP family, and relieving formation of wrinkles caused by occurrence of ECM loss.

*TIMP-1* primer: F: 5'-CTGTTGTTGCTGTGGCTGAT-3' (as set forth in SEQ ID NO: 84); and R: 5'-TCTGGTTGACTTCTGGTGTCC-3' (as set forth in SEQ ID NO: 85).

TABLE 64

| Group | Mean | SD | P-value | Increase Rate (vs NC) |
|---|---|---|---|---|
| BC | 1.00 | 0.07 | / | / |
| NC | 0.68 | 0.05 | 0.002 ** | / |
| PC | 0.97 | 0.02 | 0.001 ** | / |
| Filtrate-1% | 1.05 | 0.10 | 0.005 ** | 54.41% |
| Lysate-0.5% | 0.82 | 0.08 | 0.059 | 20.59% |
| Filtrate+lysate-0.3125% | 0.92 | 0.07 | 0.010 * | 35.29% |

Experimental results are as shown in TABLE 64 and FIG. 62. The filtrate, the lysate and the filtrate+lysate can significantly increase THE expression of the tissue inhibitor of metalloproteinase *TIMP-1.*

④ *TNFR* PCR

It is receptor protein of TNFa factor, and TNFa, after binding to TNFR, mediates inflammatory response of downstream oxidative stress damage.

TNFR primer: F: 5'-GCAGGGACCAGGAAAAGGAAT-3' (as set forth in SEQ ID NO: 86); and R: 5'-CTGAGACATAGGTGCCTGGCG-3' (as set forth in SEQ ID NO: 87).

TABLE 65

| Group | Mean | SD | P-value | Increase Rate (vs NC) |
|---|---|---|---|---|
| BC | 1.00 | 0.05 | / | / |
| NC | 1.46 | 0.02 | 0.000 ** | / |
| PC | 0.98 | 0.10 | 0.001 ** | / |
| Filtrate-1% | 1.47 | 0.08 | 0.891 | / |
| Lysate-0.5% | 0.88 | 0.06 | 0.000 ** | 39.73% |
| Filtrate+lysate-0.3125% | 1.00 | 0.01 | 0.000 ** | 31.51% |

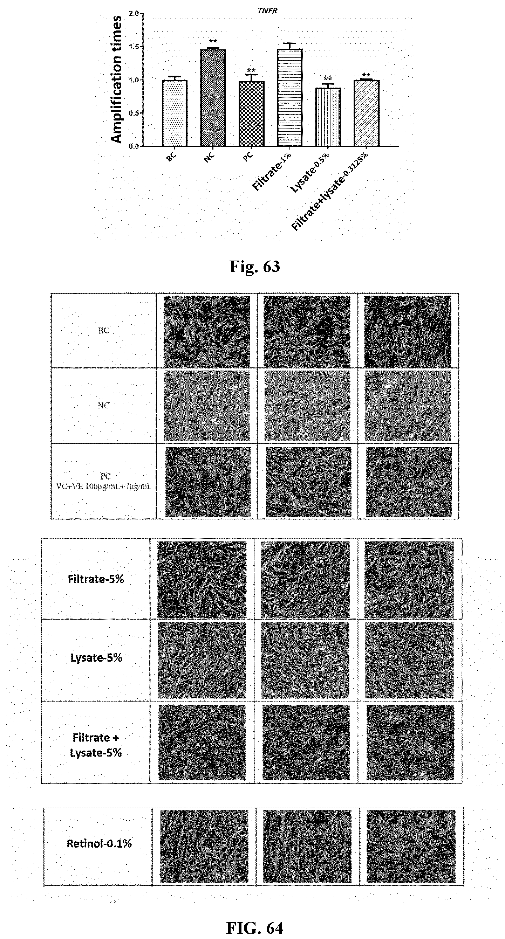

Experimental results are as shown in TABLE 65 and FIG. 63. The lysate and the filtrate+lysate can significantly down-regulate the expression of the oxidative stress-related gene *TNFR.*

(4) Test of excised skin tissues based on combined irradiation of UVA and UVB

TABLE 66

| | Group | Sample Name | Dosing Concentration | Induction Condition | Detection Model | Detection Index | Detection Method |
|---|---|---|---|---|---|---|---|
| | Blank control (BC) | | / | / | | | |
| | Negative control (NC) | / | / | | | 1. HA | 1. IF |
| | Positive control (PC) | VC+VE | 100μg/mL +7μg/mL | 30J/cm$^2$UVA+50mJ cm$^2$UVB | Exvivo | 2. Elastin | 2. Victoria staining |
| | Sample Group | Filtrate | 5% | | | 3. Collagen fiber | 3. Masson staining |
| | | Lysate | 5% | | | | |
| | | Filtrate+lysate | 5% | | | | |
| | | Retinol | 0.1% | | | | |

In the above, in the filtrate+lysate, the filtrate accounts for 50%, and the lysate accounts for 50%.

TABLE 67

| | Group | Sample Name | Dosing Concentration | Induction Condition | Detection Model | Detection Index | Detection Method |
|---|---|---|---|---|---|---|---|
| | Blank control (BC) | | / | / | | Collagen III | |
| | Negative control (NC) | / | / | | | | |
| | Positive control (PC) | VC+VE | 1 00μg/mL+7μ g/mL | 30J/cm$^2$UVA+50m Jcm$^2$UVB | Exvivo | Collagen IV | IHC |
| | Sample group | Filtrate | 5% | | | | |
| | | Lysate | 5% | | | Collagen XVII | |
| | | Filtrate+Lysate | 5% | | | | |
| | | Retinol | 0.1% | | | | |

In the above, in the filtrate+lysate, the filtrate accounts for 50%, and the lysate accounts for 50%.

1) Testing method

Tissue treatment: Freshly obtained skin tissues were immersed into 75% alcohol to be cleaned for 30 s, and then cleaned in a sterile PBS buffer solution for three times. After the cleaning was finished, the skin was cut into tissue blocks of $24\pm2$ mm$^2$, with the epidermis facing upwards and the dermis facing downwards, and put into culture moulds; and then the culture moulds were transferred into 6-well plates, with each well being added with 3.7 mL of the culture solution, and cultured in the $CO_2$ incubator (37 °C, 5% $CO_2$), with the solution being changed every day.

Dosing: After the excised skin tissues were cultured for 2 days, irradiation and dosing were started with reference to test groups and corresponding treatment conditions in the table. Irradiation dosages were UVA (30 J/cm$^2$) and UVB (50 mJ/cm$^2$). The irradiation was continuously performed for 4 days. After each time of irradiation was completed, the culture solution was replaced with a fresh culture solution, and a dosing treatment was performed. The positive control (VC + VE) was dosed below a liquid surface, and the test sample was does on a surface, with each group being in triplicate. After 4 days of consecutive irradiation, the excised skin tissues continued to be cultured for 3 days, during which period no irradiation was performed and only samples were dosed

Detection of collagen fiber: the skin tissues having undergone the dosing were fixed using 4% paraformaldehyde. The tissues were entrapped, sliced and then underwent Masson staining. Slicing result was recovered, photographed using a microscope, and analyzed using Image-Pro Plus image processing software.

Immunohistochemical detection: Detection was performed according to specific operation steps of immunohistochemistry.

Immunofluorescence detection: Detection was performed according to specific operation steps of immunofluorescence.

Calculation of increase rate:

$$\text{Increase rate} = (\text{sample group} - \text{negative control group}) / \text{negative control group} \times 100\%$$

Statistical analysis of results: GraphPad Prism was applied for plotting, and results were expressed as Mean $\pm$SD. Comparison between the groups was statistically analyzed using t-test. Statistical analysis was two-tailed. $P<0.05$ is considered to have a significant difference, and $P<0.01$ is considered to have an extremely significant difference.

In the above: * indicates significant difference, and ** indicates extremely significant difference.

2) Test Results

① Collagen fiber

Collagen fiber is a fiber bundle formed by assembling collagen fibrils, wherein the collagen fibrils are mainly composed of COL3 and COLI, which are resilient but have poor elasticity. Collagen fiber is crosslinked with the elastic fiber to maintain the elasticity and strength of the skin.

TABLE 68

| Experimental Group | Relative Area Mean | SD | P-value | Increase Rate |
|---|---|---|---|---|
| BC | 1.00 | 0.07 | / | / |
| NC | 0.64 | 0.04 | 0.002 ** | / |
| PC | 1.04 | 0.06 | 0.001 ** | / |
| Filtrate-5% | 1.08 | 0.02 | 0.000 ** | 68.75% |
| Lysate-5% | 0.85 | 0.11 | 0.032 * | 32.81% |
| Filtrate+lysate-5% | 1.24 | 0.08 | 0.000 ** | 93.75% |
| Retinol-0.1% | 0.96 | 0.02 | 0.000 ** | 50.00% |

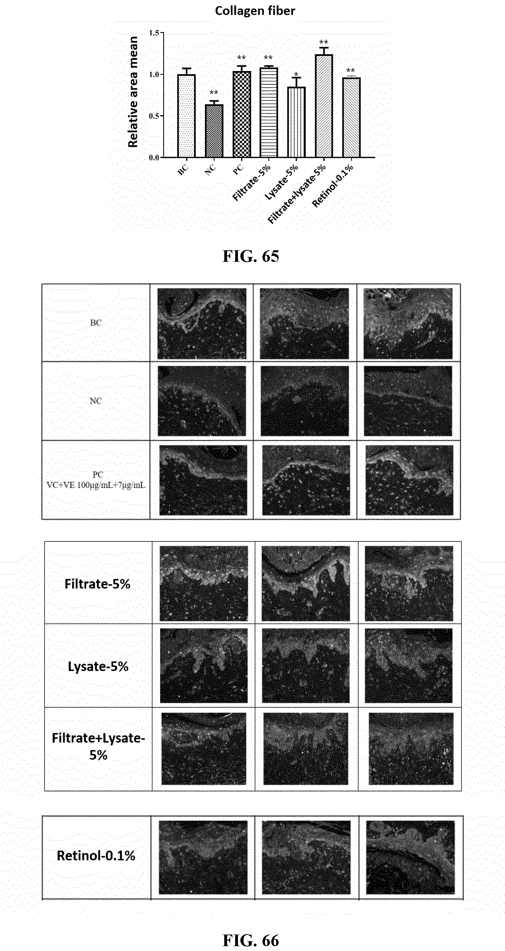

Experimental results are as shown in TABLE 68, FIG. 64 and FIG. 65. The filtrate, the lysate and the filtrate+lysate can all increase the expression of the collagen fiber.

(2) Hyaluronic acid

HA is hyaluronic acid. HA has the highest content in the skin, moisturizes, maintains an extracellular space and interaction with other substances and provides stability and elasticity of an extracellular matrix, thereby resisting wrinkles and enhancing skin elasticity, to make the skin plump.

TABLE 69

| Experimental Group | Relative IOD Mean | SD | P-value | Increase Rate |
|---|---|---|---|---|
| BC | 1.00 | 0.07 | / | / |
| NC | 0.44 | 0.01 | 0.000 ** | / |
| PC | 0.93 | 0.12 | 0.002 ** | / |
| Filtrate-5% | 0.91 | 0.10 | 0.001 ** | 106.82% |

(continued)

| Experimental Group | Relative IOD Mean | SD | P-value | Increase Rate |
|---|---|---|---|---|
| Lysate-5% | 0.55 | 0.05 | 0.024 * | 25.00% |
| Filtrate+lysate-5% | 0.62 | 0.11 | 0.045 * | 40.91% |
| Retinol-0.1 % | 0.69 | 0.13 | 0.029 * | 56.82% |

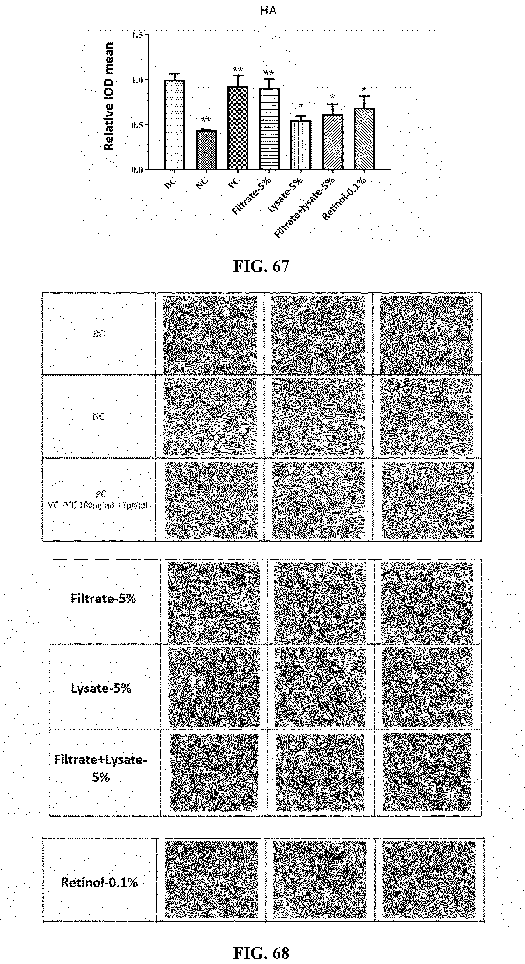

Experimental results are as shown in TABLE 69, FIG. 66 and FIG. 67. The filtrate, the lysate and the filtrate+lysate can increase the expression of the hyaluronic acid.

(3) Elastin

Elastin is a main protein constituting the elastic fiber, and a reduced content causes the elastic fiber to become sparse and skin elasticity to decrease.

TABLE 70

| Experimental Group | Relative IOD Mean | SD | P-value | Increase Rate |
|---|---|---|---|---|
| BC | 1.00 | 0.03 | / | |
| NC | 0.33 | 0.02 | 0.000 ** | / |
| PC | 0.58 | 0.04 | 0.000 ** | / |
| Filtrate-5% | 1.10 | 0.02 | 0.000 ** | 233.33% |
| Lysate-5% | 1.24 | 0.14 | 0.000 ** | 275.76% |
| Filtrate+lysate-5% | 1.48 | 0.21 | 0.001 ** | 348.48% |
| Retinol-0.1 % | 1.19 | 0.07 | 0.000 ** | 260.61% |

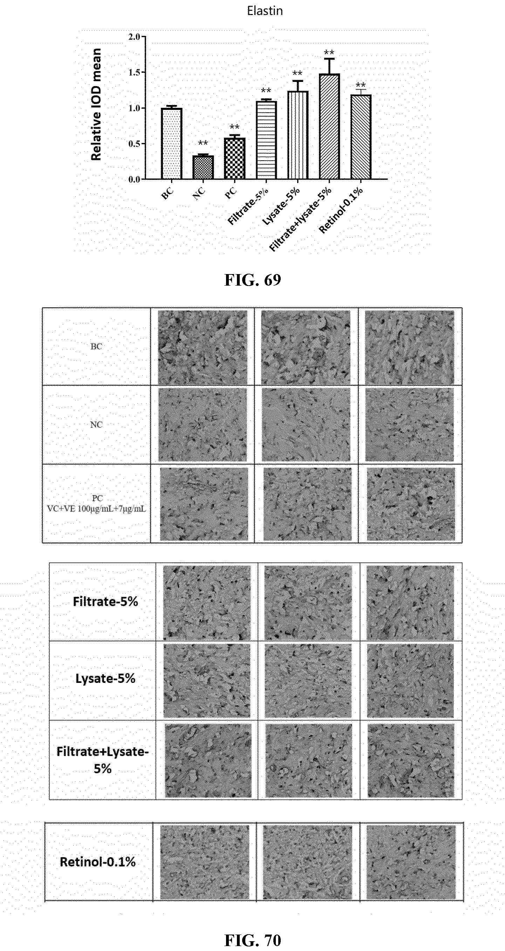

Experimental results are as shown in TABLE 70, FIG. 68 and FIG. 69. The filtrate, the lysate and the filtrate+lysate can significantly increase the expression of elastin.

④ Type III collagen

The type III collagen is one of the main components of collagen of dermal ECM, accounts for 5-20% of a total content of collagens in the human body, is often mixed with the type I collagen fiber, and is rich in elastic tissues. Promoting increase of *COL III* content can achieve a certain effect of resisting wrinkles and aging. After the test substance acts on the skin model, the anti-wrinkle efficacy of the test substance is evaluated by detecting changes of *COL III.*

TABLE 71

| Experimental Group | Relative IOD Mean | SD | P-value | Increase Rate |
|---|---|---|---|---|
| BC | 1.00 | 0.07 | / | / |
| NC | 0.59 | 0.04 | 0.001 ** | / |
| PC | 0.78 | 0.05 | 0.006 ** | / |
| Filtrate-5% | 0.81 | 0.02 | 0.001 ** | 37.29% |
| Lysate-5% | 0.71 | 0.09 | 0.096 | 20.34% |
| Filtrate+lysate-5% | 0.83 | 0.07 | 0.006 ** | 40.68% |
| Retinol-0.1% | 0.76 | 0.03 | 0.003 ** | 28.81% |

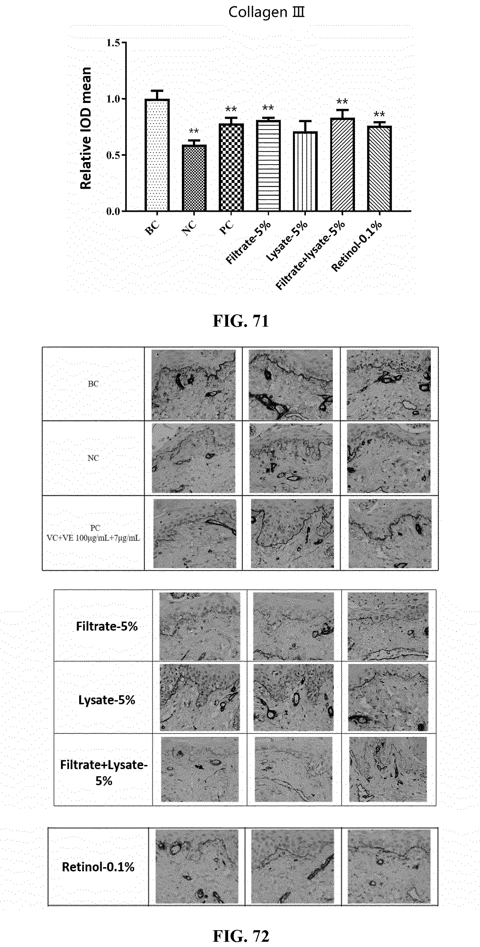

Experimental results are as shown in TABLE 71, FIG. 70 and FIG. 71. The filtrate, the lysate and the filtrate+lysate can all significantly increase the expression of *COL III.*

⑤ Type IV collagen

Expression of *COL XVII+COL IV* is distributed at the dermal-epidermal junction. The dermal-epidermal junction (DEJ) is located between the epidermis and the dermis, consists of important proteins such as Laminin 5, *COL IV, COL VII,* and *COLXVII* type collagens, and has effects of tightening the epidermis and exchanging substances between the dermis

47

and the epidermis. With influences of adverse environmental factors such as growth of age and ultraviolet radiation, dermal-epidermal adhesion is reduced, nutrient delivery therein is decreased, and cell proliferation ability is lowered down, thus causing acceleration of the skin aging, and occurrence of problems such as skin slackening.

TABLE 72

| Experimental Group | Relative IOD/area Mean | SD | P-value | Increase Rate |
|---|---|---|---|---|
| BC | 1.00 | 0.14 | / | / |
| NC | 0.60 | 0.03 | 0.008 ** | / |
| PC | 0.95 | 0.01 | 0.000 ** | / |
| Filtrate-5% | 0.53 | 0.04 | 0.064 | / |
| Lysate-5% | 0.87 | 0.08 | 0.002 ** | 45.00% |
| Filtrate+lysate-5% | 0.57 | 0.02 | 0.332 | / |
| Retinol-0.1 % | 0.76 | 0.03 | 0.003 ** | 26.67% |

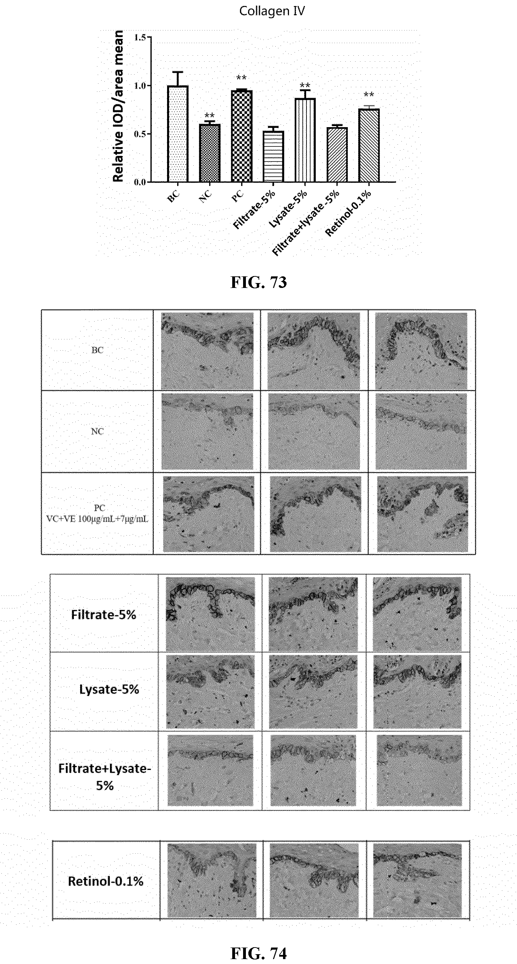

Experimental results are as shown in TABLE 72, FIG. 72 and FIG. 73. The lysate can significantly increase the expression of the type IV collagen.

⑥ Type XVII collagen

Expression of *COL XVII+COL IV* is distributed at the dermal-epidermal junction. The dermal-epidermal junction (DEJ) is located between the epidermis and the dermis, consists of important proteins such as Laminin 5, *COL IV, COL VII,* and *COLXVII* type collagens, and has effects of tightening the epidermis and exchanging substances between the dermis and the epidermis. With influences of adverse environmental factors such as growth of age and ultraviolet radiation, dermal-epidermal adhesion is reduced, nutrient delivery therein is decreased, and cell proliferation ability is lowered down, causing acceleration of the skin aging, and occurrence of problems such as skin slackening.

TABLE 73

| Experimental Group | Relative IOD Mean | SD | P-value | Increase Rate |
|---|---|---|---|---|
| tie | 1.00 | 0.16 | / | / |
| NC | 0.25 | 0.05 | 0.001 ** | / |
| PC | 0.79 | 0.15 | 0.004 ** | / |
| Filtrate-5% | 1.10 | 0.12 | 0.000 ** | 340.00% |
| Lysate-5% | 0.87 | 0.12 | 0.001 ** | 248.00% |
| Filtrate+lysate-5% | 0.32 | 0.02 | 0.100 | 28.00% |
| Retinol-0.1% | 0.69 | 0.11 | 0.003 ** | 176.00% |

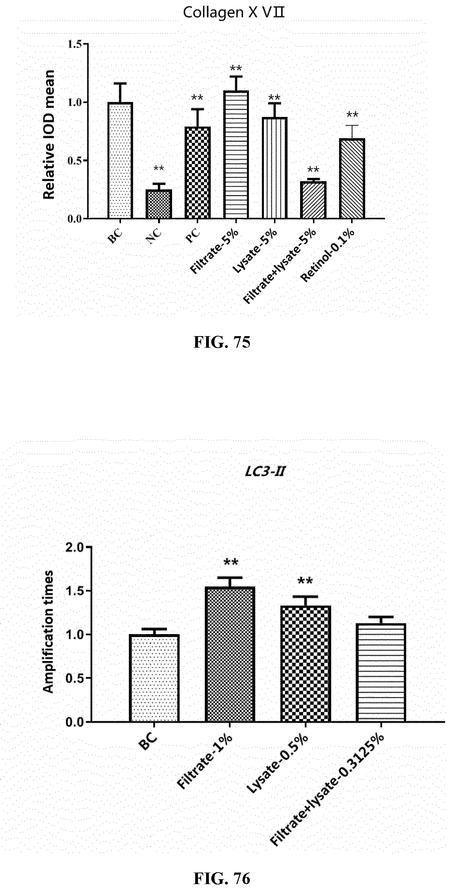

In the above: * indicates significant difference, and ** indicates extremely significant difference.
Experimental results are as shown in TABLE 73, FIG. 74 and FIG. 75. The filtrate, the lysate and the filtrate+lysate can significantly increase the expression of the type XVII collagen.

**Verification Example 9 Cell autophagy-fibroblast-based test**

(1). Amount of *LC3 II* gene expression

*LC3-II* primer: F: 5'-GAACTGAGCTGCCTCTACCG-3' (as set forth in SEQ ID NO: 88); and
R: 5'-GGGACAACCCTAACACGACC-3' (as set forth in SEQ ID NO: 89)
It is an important component in autophagy formation. LC3 binding system aims at performing phospholipidation (PE), and thus LC3 connection system is also called as LC3-lipidation system. LC3 protein has two shear forms of LC3-I and LC3-II in cells. LC3-I is located in cytoplasm and LC3-II is located in autophagic membrane. LC3-II is quite essential for formation of autophagosome, and studies have shown that removing LC3-II will form a series of non-closed autophagosome structures. As the quantity of LC3-II and the quantity of autophagosome are in one-to-one correspondence, LC3-II is generally recognized as a marker of mammalian autophagosomes. In an autophagic process, LC3-II on autophagic membrane is degraded by lysosome, and in a case of adding a lysosomal inhibitor, autophagic flux can be detected by comparing changes in the amount of LC3-II protein in the autophagic process by immunofluorescence, to reflect autophagic activity is reflected.

1) Testing method

Cell inoculation: After resuscitation of cells, when a plating rate reached about 60%, the cells were inoculated in 6-well plates, and incubated overnight in the $CO_2$ incubator (37 °C, 5% $CO_2$).

Dosing: According to test groups in TABLE 75, when the plating rate of the cells in the 6-well plates reached about 40%-60%, dosing was performed for the groups respectively, with each group in triplicate. The blank control group was added with 2 mL of a culture solution per well, and the sample group was added with 2 mL of a test sample-containing culture solution at a corresponding concentration per well. After the dosing was completed, the 6-well plates were incubated in the $CO_2$ incubator (37 °C, 5% $CO_2$) for 24 h.

Cell collection: After 24 h of culture, an original solution was sucked out and discarded, PBS cleaning was performed twice, and each well was added with 1 mL of RNAiso Plus, to pipet and lyse the cells, after which samples were collected.

Detection of gene expression: RNA was extracted and subjected to reverse transcription to cDNA to undergo fluorescence quantitative PCR detection, and result was calculated by a $2^{-\Delta\Delta CT}$ method.

Calculation of up-regulation rate: up-regulation rate = (sample group - negative control group) / negative control group $\times$ 100%

Statistical analysis of results: GraphPad Prism was applied for plotting, and results were expressed as Mean $\pm$ SD. Comparison between the groups was statistically analyzed using t-test. Statistical analysis was two-tailed. $P<0.05$ is considered to have a significant difference, and $P<0.01$ is considered to have an extremely significant difference.

2) Test results

TABLE 74

| Group | Mean | SD | *P*-value | Increase Rate |
|---|---|---|---|---|
| BC | 1.00 | 0.06 | / | / |
| Filtrate-1% | 1.55 | 0.10 | 0.001 ** | 55.00% |
| Lysate-0.5% | 1.33 | 0.10 | 0.008 ** | 33.00% |
| Filtrate+lysate-0.3125% | 1.13 | 0.07 | 0.061 | 13.00% |

In the above: * indicates significant difference, and ** indicates extremely significant difference.

Experimental results are as shown in TABLE 74 and FIG. 76. The filtrate, the lysate and the filtrate+lysate can significantly improve the expression of the autophagy-related gene *LC3 II.*

(2). *Beclin 1*

Autophagy is one of the important ways for intracellular material and energy metabolism, and is involved in multiple pathophysiological processes. *Beclin 1* is a homologue of yeast ATG6/Vps30 gene in mammals, plays an important role in a process of forming autophagic vacuoles and is considered as one of specific markers of autophagy. By detecting an expression level of *Beclin 1* in cells by immunohistochemistry, the autophagic activity of the cells can be dynamically monitored.

1) Testing method-immunofluorescence

Cell inoculation: After resuscitation of cells, when the plating rate reached about 60%, the cells were inoculated in 24-well plates, and incubated overnight in the $CO_2$ incubator (37 °C, 5% $CO_2$).

Dosing: According to test groups in TABLE 75, when the plating rate of the cells in the 24-well plates reached about 40%-60%, dosing was performed for the groups respectively, with each group in triplicate. The blank control group was added with 1 mL of the culture solution per well, and the sample group was added with 1 mL of the test sample-containing culture solution at corresponding concentration per well. After the dosing was completed, the 24-well plates were incubated in the $CO_2$ incubator (37 °C, 5% $CO_2$) for 24 h.

Immunofluorescence detection: After the incubation was finished, the cells were fixed with 4% paraformaldehyde. After 30 min of fixation, immunofluorescence detection was performed. Staining results were observed under a fluorescence microscope and photographed. Pictures were collected and analyzed using Image-Pro Plus software.

Calculation of increase rate: increase rate = (sample group - blank control group) / blank control group × 100%

Statistical analysis of results: GraphPad Prism was applied for plotting, and results were expressed as Mean ±SD. Comparison between the groups was statistically analyzed using t-test. Statistical analysis was two-tailed. $P < 0.05$ is considered to have a significant difference, and $P < 0.01$ is considered to have an extremely significant difference.

2) Test results

TABLE 75

| Experimental Group | Relative IOD/Cell Quantity Mean | SD | P-value | Increase Rate |
|---|---|---|---|---|
| BC | 1.00 | 0.07 | / | / |
| Filtrate-1% | 0.90 | 0.09 | 0.225 | / |
| Lysate-0.5% | 1.85 | 0.25 | 0.005 ** | 85.00% |
| Filtrate+lysate-0.3125% | 0.96 | 0.12 | 0.686 | / |

In the above: * indicates significant difference, and ** indicates extremely significant difference.

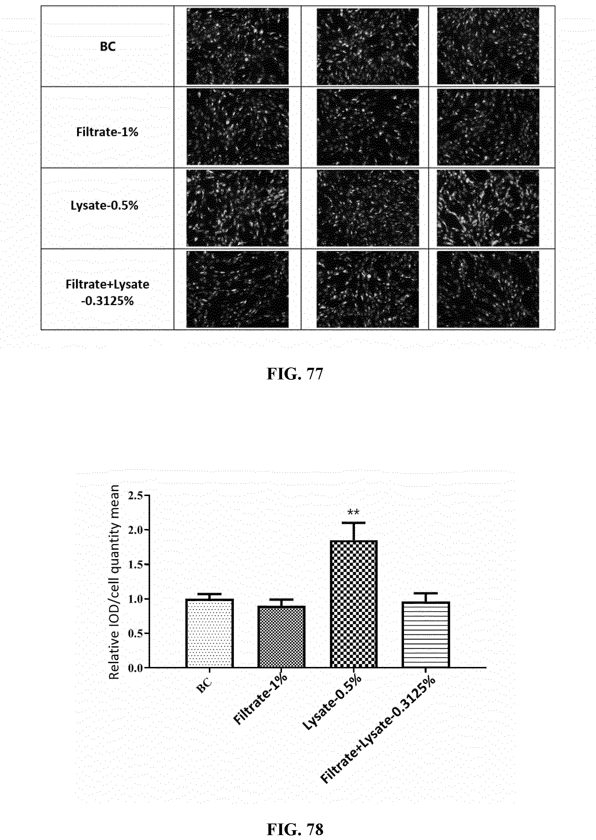

Experimental results are as shown in TABLE 75, FIG. 77 and FIG. 78. The lysate can significantly improve the expression of the autophagy-related protein *Beclin 1.*

The above-mentioned are merely for preferred embodiments of the present invention, and it should be indicated that those ordinarily skilled in the art further could make several improvements and modifications, without departing from the principle of the present invention, and all of these improvements and modifications also should be considered as the scope of protection of the present invention.

**Claims**

1. *Bifidobacterium longum subsp. infantis,* **characterized in that** a preservation No. thereof is: CGMCC NO. 27851.

2. A microbial agent, **characterized by** comprising the *Bifidobacterium longum subsp. infantis* according to claim 1.

3. Use of the *Bifidobacterium longum subsp. infantis* according to claim 1 and/or the microbial agent according to claim 2 in preparing a product for barrier repair,

   preferably, the barrier repair is the *Bifidobacterium longum subsp. infantis* and/or the microbial agent up-regulating expression of one or more genes in *FLG* gene, *LOR* gene, *IVL* gene and *AQP3* gene;
   the barrier repair is the *Bifidobacterium longum subsp. infantis* and/or the microbial agent elevating a cell migration capability; and
   the barrier repair is the *Bifidobacterium longum subsp. infantis* and/or the microbial agent up-regulating expression of one or more genes in *Caspase14* gene, *DSG1* gene, *Claudin-4* gene, *ABCA12* gene, *ACACA* gene, *HMGCR* gene and *Claudin-1* gene.

4. Use of the *Bifidobacterium longum subsp. infantis* according to claim 1 and/or the microbial agent according to claim 2 in preparing a soothing product.

5. The use according to claim 4, wherein the soothing is the *Bifidobacterium longum subsp. infantis* and/or the microbial agent reducing a content of PGE2.

6. The use according to claim 4 or 5, wherein the soothing is the *Bifidobacterium longum subsp. infantis* and/or the microbial agent reducing a content of inflammatory factors of macrophages,
   preferably, the inflammatory factors comprise: IL6 and/or IL-1β.

7. Use of the *Bifidobacterium longum subsp. infantis* according to claim 1 and/or the microbial agent according to claim 2 in preparing a whitening product,

preferably, the whitening is the *Bifidobacterium longum subsp. infantis* and/or the microbial agent inhibiting expression of tyrosinase; and

the whitening is the *Bifidobacterium longum subsp. infantis* and/or the microbial agent down-regulating expression of one or more genes of *TYR* gene, *Rab27a* gene, *Myo5a* gene, *MLPH* gene, *GPR143* gene, *SLC45A2* gene, *MITF* gene, *TRP-1* gene, *TRP-2* gene, *Pmel17* gene, *Kinesin* gene and *CDC42* gene.

8.  Use of the *Bifidobacterium longum subsp. infantis* according to claim 1 and/or the microbial agent according to claim 2 in preparing an anti-aging product.

9.  The use according to claim 8, wherein the anti-aging is the *Bifidobacterium longum subsp. infantis* and/or the microbial agent reducing quantity and/or fluorescence intensity of CPD positive cells.

10. The use according to claim 8 or 9, wherein the anti-aging is the *Bifidobacterium longum subsp. infantis* and/or the microbial agent up-regulating expression of one or more genes in *COL TV* gene, *COL VII* gene and *BGN* gene.

11. The use according to claim 8, wherein the anti-aging is the *Bifidobacterium longum subsp. infantis* and/or the microbial agent down-regulating expression of one or more genes in *MMP* gene, *p16* gene, *TIMP-1* gene, *NF-κB* gene and *TNFR* gene.

12. The use according to claim 8, wherein the anti-aging is the *Bifidobacterium longum subsp. infantis* and/or the microbial agent increasing a content of one or more of collagen fiber, hyaluronic acid, elastin and type III collagen.

13. A product, **characterized by** comprising the *Bifidobacterium longum subsp. infantis* according to claim 1, a fermentation broth of the *Bifidobacterium longum subsp. infantis* according to claim 1 and/or the microbial agent according to claim 2 and an acceptable auxiliary.

14. The product according to claim 13, comprising one or more of a personal care product, a pharmaceutical product and a health product.

15. The product according to claim 14, wherein a dosage form of the personal care product comprises one or more of cream form, emulsion form, powder form and essence form,

preferably, the personal care product comprises a basic care product and/or a make-up product.

**FIG. 1**

73 ┤ NR 145535.1 Bifiaobacterium longum subsp. suillum strain Su 851(2)

84 ┤ NR 145535.1 Bifiaobacterium longum subsp. suillum strain Su 851

NR 044691.2 Bifidobacterium longum strain ATCC 15707

59

76 └ NR 044693.2 Bifidobacterium longum subsp. suis strain ATCC 27533

99 ── YSGBifido001

87

47

NR 043437.1 Bifidobacterium longum subsp. infantis strain ATCC 15697(2)

100 ┤ NR 043437.1 Bifidobacterium longum subsp. infantis strain ATCC 15697

47

NR 040783.1 Bifidobacterium breve DSM 20213 = JCM 1192

NR 174313.1 Bifidobacterium cebidraum strain CCUG 73785

56

52 ┤ NR 181805.1 Bifidobacterium miconisargetati strain 82T25

39 └ NR 113173.1 Bifidobacterium saguini DSM 23967 strain AFB23-1

NR 135862.1 Bifidobacterium lemurum strain LMC 13

100 └ NR 148784.1 Bifidobacterium eulemuris strain LMM E3

64

NR 036855.1 Bifidobacterium pullorum subsp. gallinarum strain Ch206-5

33

100 └ NR 036856.1 Bifidobacterium pullorum strain B 145

0

NR 180396.1 Bifidobacterium goeldii strain LMG 30939

NR 037115.2 Bifidobacterium dentium strain B764

NR 181803.1 Bifidobacterium santillancese strain 82T1

**FIG. 2**

**FIG. 3**

**FIG. 4**

**FIG. 5**

FIG. 6

FIG. 7

FIG. 8

FIG. 9

**FIG. 10**

**FIG. 11**

**FIG. 12**

**FIG. 13**

**FIG. 14**

**FIG. 15**

**FIG. 16**

**FIG. 17**

**FIG. 18**

**FIG. 19**

**FIG. 20**

**FIG. 21**

**FIG. 22**

**FIG. 23**

**FIG. 24**

**FIG. 25**

**FIG. 26**

**FIG. 27**

**FIG. 28**

FIG. 29

TYR

FIG. 30

Rab27a

FIG. 31

Myo5a

FIG. 32

FIG. 33

FIG. 34

FIG. 35

**FIG. 36**

**FIG. 37**

**FIG. 38**

FIG. 39

FIG. 40

FIG. 41

**FIG. 42**

**FIG. 43**

**FIG. 44**

**FIG. 45**

**FIG. 46**

**FIG. 47**

**FIG. 48**

**FIG. 49**

**FIG. 50**

FIG. 51

FIG. 52

FIG. 53

*Fibronectin*

**FIG. 54**

**FIG. 55**

**FIG. 56**

**FIG. 57**

**FIG. 58**

**FIG. 59**

**FIG. 60**

**FIG. 61**

**FIG. 62**

**Fig. 63**

**FIG. 64**

**FIG. 65**

**FIG. 66**

HA

**FIG. 67**

**FIG. 68**

**FIG. 69**

**FIG. 70**

Collagen Ⅲ

**FIG. 71**

**FIG. 72**

Collagen IV

**FIG. 73**

**FIG. 74**

**Collagen X VII**

**FIG. 75**

*LC3-II*

**FIG. 76**

**FIG. 77**

**FIG. 78**

Europäisches
Patentamt
European
Patent Office
Office européen
des brevets

# EUROPEAN SEARCH REPORT

**Application Number**

EP 24 21 4894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MA XUE ET AL: "Bifidobacterium infantis strain YLGB-1496 possesses excellent antioxidant and skin barrier-enhancing efficacy in vitro", EXPERIMENTAL DERMATOLOGY, vol. 31, no. 7, 6 May 2022 (2022-05-06), pages 1089-1094, XP093270126, COPENHAGEN; DK ISSN: 0906-6705, DOI: 10.1111/exd.14583 Retrieved from the Internet: URL:https://onlinelibrary.wiley.com/doi/full-xml/10.1111/exd.14583> * abstract; page 1090 col. 1 lines 4-5; page 1092, col. 1 first full par.; Figs. 2, 3 * | 1-15 | INV. C12N1/20 A61Q19/00 ADD. C12R1/01 |
| X | CN 116 716 206 A (WEIKANG PROBIOTICS SUZHOU CO LTD) 8 September 2023 (2023-09-08) * examples 1, 7, 8; claims 1-10 * | 1-15 | |
| X | CN 113 897 300 A (UNIV JIANGNAN) 7 January 2022 (2022-01-07) * Claims 1-10 * | 1-15 | **TECHNICAL FIELDS SEARCHED (IPC)** C12N A61K A61Q |
| A | DEVON W. KAVANAUGH ET AL: "Exposure of Bifidobacterium longum subsp. infantis to Milk Oligosaccharides Increases Adhesion to Epithelial Cells and Induces a Substantial Transcriptional Response", PLOS ONE, vol. 8, no. 6, 21 June 2013 (2013-06-21), page e67224, XP055474541, DOI: 10.1371/journal.pone.0067224 * abstract; Discussion * | 1-15 | C12R |

The present search report has been drawn up for all claims

| Place of search | Date of completion of the search | Examiner |
|---|---|---|
| Munich | 15 April 2025 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 24 21 4894

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2025

| Patent document cited in search report | Publication date | Patent family member(s) | Publication date |
|---|---|---|---|
| CN 116716206 A | 08-09-2023 | NONE | |
| CN 113897300 A | 07-01-2022 | NONE | |

EPO FORM P0459

For more details about this annex : see Official Journal of the European Patent Office, No. 12/82